(19) Europäisches Patentamt
European Patent Office
Office européen des brevets

(11) EP 1 042 355 B1

(12) **EUROPÄISCHE PATENTSCHRIFT**

(45) Veröffentlichungstag und Bekanntmachung des Hinweises auf die Patenterteilung:
**12.02.2003 Patentblatt 2003/07**

(21) Anmeldenummer: **98966410.7**

(22) Anmeldetag: **23.12.1998**

(51) Int Cl.[7]: **C07J 41/00**, A61K 31/565, C07J 43/00, C07J 31/00, A61K 31/58

(86) Internationale Anmeldenummer:
**PCT/EP98/08470**

(87) Internationale Veröffentlichungsnummer:
**WO 99/033855 (08.07.1999 Gazette 1999/27)**

(54) **11BETA-HALOGEN-7ALPHA-SUBSTITUIERTE-ESTRATRIENE, VERFAHREN ZUR HERSTELLUNG PHARMAZEUTISCHER PRÄPARATE, DIE DIESE 11BETA-HALOGEN-7ALPHA-SUBSTITUIERTE-ESTRATRIENE ENTHALTEN, SOWIE DEREN VERWENDUNG ZUR HERSTELLUNG VON ARZNEIMITTELN**

11BETA-HALOGEN-7ALPHA-SUBSTITUTED ESTRATRIENES, METHOD FOR PRODUCING PHARMACEUTICAL PREPARATIONS CONTAINING SAID 11BETA-HALOGEN-7ALPHA-SUBSTITUTED ESTRATRIENES AND USE OF THE SAME FOR PRODUCING MEDICAMENTS

ESTRATRIENES 11BETA-HALOGENE-7ALPHA-SUBSTITUES, PROCEDE DE PREPARATION DE PREPARATIONS PHARMACEUTIQUES, QUI CONTIENNENT LESDITS ESTRATRIENES 11BETA-HALOGENE-7ALPHA-SUBSTITUES ET LEUR UTILISATION POUR PREPARER DES MEDICAMENTS

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **23.12.1997 DE 19758390**
**10.02.1998 DE 19806357**

(43) Veröffentlichungstag der Anmeldung:
**11.10.2000 Patentblatt 2000/41**

(73) Patentinhaber: **Schering Aktiengesellschaft
13353 Berlin (DE)**

(72) Erfinder:
- **BOHLMANN, Rolf**
  **D-14055 Berlin (DE)**
- **HEINRICH, Nikolaus**
  **D-12159 Berlin (DE)**
- **HOFMEISTER, Helmut**
  **D-13465 Berlin (DE)**
- **KROLL, Jorg**
  **D-12157 Berlin (DE)**
- **KÜNZER, Hermann**
  **D-13347 Berlin (DE)**
- **SAUER, Gerhard**
  **D-13465 Berlin (DE)**
- **ZORN, Ludwig**
  **D-13509 Berlin (DE)**
- **FRITZEMEIER, Karl-Heinrich**
  **D-13503 Berlin (DE)**
- **LESSL, Monika**
  **D-13467 Berlin (DE)**
- **LICHTNER, Rosemarie**
  **D-10823 Berlin (DE)**
- **NISHINO, Yukishige**
  **D-14089 Berlin (DE)**
- **PARCZYK, Karsten**
  **D-12207 Berlin (DE)**
- **SCHNEIDER, Martin**
  **D-13469 Berlin (DE)**

(56) Entgegenhaltungen:
**EP-A- 0 138 504**
**EP-A- 0 367 576**
**WO-A-98/07740**
**US-A- 3 076 829**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- **ANSTEAD G M ET AL: "The estradiol pharmacophore: Ligand structure-estrogen receptor binding affinity relationships and a model for the receptor binding site" STEROIDS: STRUCTURE, FUNCTION, AND REGULATION, Bd. 62, Nr. 3, März 1997, Seite 268-303 XP004057108**

## Beschreibung

Die vorliegende Erfindung betrifft 11β-Halogen-7α-substituierte-Estratriene der allgemeinen Formel I

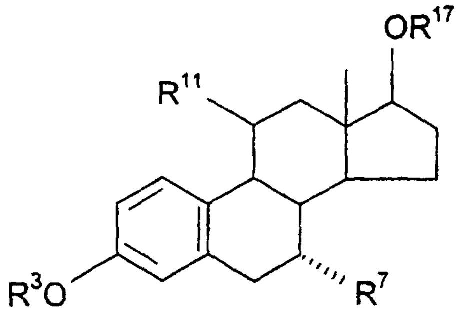

(I)

worin

$R^3$ ein Wasserstoffatom, einen Kohlenwasserstoffrest mit bis zu 8 Kohlenstoffatomen oder einen Rest der Teilformel $R^{3'}$—C(O)—, worin $R^{3'}$ ein Wasserstoffatom oder ein Kohlenwasserstoffrest mit bis zu 8 Kohlenstoffatomen oder ein Phenylrest ist, bedeutet,
$R^7$ einen Rest der Formel -A-B-Z-$R^{20}$ bedeutet,
worin

A fiir eine direkte Bindung oder einen Benzylidenrest, wobei die Methylengruppe an das 7-Kohlenstoffatom des Steroids gebunden ist, oder einen Phenylenrest,
B fiir eine gerad- oder verzweigtkettige Alkylen-, Alkenylen- oder Alkinylengruppe mit 3 bis 14 Kohlenstofatomen,

stehen, und

Z für -$NR^{21}$- und $R^{21}$ für eine $C_1$-$C_3$-Alkylgruppe stehen,
wobei dann $R^{20}$

ein Wasserstoffatom,
eine gerad- oder verzweigtkettige Alkyl-, Alkenyl- oder Alkinylgruppe mit bis zu 10 Kohlenstofatomen, oder eine der Gruppierungen -D-$C_nF_{2n+1}$, wobei D eine gerad- oder verzweigtkettige Alkylen-, Alkenylen- oder Alkinylengruppe mit bis zu 8 Kohlenstofatomen und n eine ganze Zahl von 1 bis 8 ist,
-L-CH=CF-$C_pF_{2p+1}$, wobei L eine gerad- oder verzweigtkettige Alkylen-, Alkenylen- oder Alkinylengruppe mit 2 bis 7 Kohlenstofatomen und p eine ganze Zahl von 2 bis 7 ist,
-D-O-$(CH_2)_q$-Aryl, wobei D die bereits angegebenen Bedeutung hat, q 0, 1, 2 oder 3 ist und Aryl für einen gegebenenfalls ein- oder zweifach substituierten Phenyl-, 1- oder 2-Naphthyl- oder einen Heteroarylrest steht,
-D-O-$(CH_2)_r$-$C_nF_{2n+1}$, wobei D und n die bereits angegebenen Bedeutungen haben und r für eine ganze Zahl von 1 bis 5 steht, bedeutet, oder
$R^{20}$ und $R^{21}$ mit dem Stickstoffatom, an das sie gebunden sind, einen gesättigten oder ungesättigten Heterocyclus mit 5 oder 6 Kettengliedern, der gegebenenfalls ein oder zwei weitere Heteroatome, ausgewählt aus Stickstoff, Sauerstoff und Schwefel, enthält und gegebenenfalls substituiert ist,
bilden, oder

Z für -$SO_x$- und x fiir 0, 1 oder 2 stehen,
wobei $R^{20}$ dann

eine gerad- oder verzweigtkettige Alkyl-, Alkenyl- oder Alkinylgruppe mit bis zu 10 Kohlenstofatomen, oder eine der Gruppierungen

$-D-C_nF_{2n+1}$, wobei D eine gerad- oder verzweigtkettige Alkylen-, Alkenylen- oder Alkinylengruppe mit bis zu 8 Kohlenstofatomen und n eine ganze Zahl von 1 bis 8 ist,

$-L-CH=CF-C_pF_{2p+1}$, wobei L eine gerad- oder verzweigtkettige Alkylen-, Alkenylen- oder Alkinylengruppe mit 2 bis 7 Kohlenstofatomen und p eine ganze Zahl von 2 bis 7 ist,

$-D-O-(CH_2)_q$-Aryl, wobei D die bereits angegebenen Bedeutung hat, q 0, 1, 2 oder 3 ist und Aryl fiir einen gegebenenfalls ein- oder zweifach substituierten Phenyl-, 1- oder 2-Naphthyl- oder einen Heteroarylrest steht,

$-D-O-(CH_2)_r-C_nF_{2n+1}$, wobei D und n die bereits angegebenen Bedeutungen haben und r für eine ganze Zahl von 1 bis 5 steht, bedeutet, oder

Z für $-NR^{31}-$ steht,

wobei dann $R^{20}$ ein gerad- oder verzweigtkettiger, gegebenenfalls teilweise fluorierter Alkyl-, Alkenyl- oder Alkinylrest mit bis zu 14 Kohlenstoffatomen, der durch ein bis drei Heteroatome -O- und -S- und Gruppierungen $-NR^{32}-$, worin $R^{32}$ ein Wasserstoffatom oder ein $C_1$-$C_3$-Alkylrest ist, unterbrochen sein kann, ein gegebenenfalls ein- oder zweifach substituierter Aryl- oder Heteroarylrest, ein gegebenenfalls ein- oder zweifach substituierter $C_3$-$C_{10}$-Cycloalkylrest, ein gegebenenfalls ein- oder zweifach substituierter $C_4$-$C_{15}$-Cycloalkylalkylrest, ein gegebenenfalls ein- oder zweifach substituierter $C_7$-$C_{20}$-Aralkylrest, ein gegebenenfalls ein- oder zweifach substituierter Heteroaryl-$C_1$-$C_6$-alkylrest oder ein gegebenenfalls substituerter Aminoalkylrest, ist, und

$R^{31}$ ein Rest der Formel $-C(O)R^{33}$ oder $-CH_2-R^{33}$ ist,

wobei dann $R^{33}$ ein gerad- oder verzweigtkettiger, gegebenenfalls teilweise fluorierter Alkyl-, Alkenyl- oder Alkinylrest mit bis zu 14 Kohlenstoffatomen, der durch ein bis drei Heteroatome -O- und -S- und Gruppierungen $-NR^{32}-$, worin $R^{32}$ ein Wasserstoffatom oder ein $C_1$-$C_3$-Alkylrest ist, unterbrochen sein kann, ein gegebenenfalls ein- oder zweifach substituierter Aryl- oder Heteroarylrest, ein gegebenenfalls ein- oder zweifach substituierter $C_3$-$C_{10}$-Cycloalkylrest, ein gegebenenfalls ein- oder zweifach substituierter $C_4$-$C_{15}$-Cycloalkylalkylrest, ein gegebenenfalls ein- oder zweifach substituierter $C_7$-$C_{20}$-Aralkylrest, ein gegebenenfalls ein- oder zweifach substituierter Heteroaryl-$C_1$-$C_6$-alkylrest, ein gegebenenfalls substituerter Aminoalkylrest oder ein Biphenylenrest ist,

ausgenommen der Verbindungen

11β-Fluor-7α-{5-[N-methyl-N-3-(4,4,5,5,5-pentafluorpentylthio)-propylamino]-pentyl}estra-1,3,5(10)-trien-3,17β-diol

11β-Fluor-7α-{5-[2-(4,4,5,5,5-pentafluorpentylthiomethyl)-pyrrolidin-1-yl]-pentyl} -estra-1,3,5(10)-trien-3,17β-diol

11β-Fluor-7α-{6-[N-methyl-N-3-(4,4,5,5,5-pentafluorpentylthio)-propylamino]-hexyl}-estra-1,3,5(10)-trien-3,17β-diol

11β-Fluor-7α-{5-[(2S)-2-(4-trifluormethylphenylthiomethyl)-pyrrolidin-1-yl]-pentyl}-estra-1,3,5(10)-trien-3,17β-diol

11β-Fluor-7α-{5-[(2S)-2-(4,4,5,5,5-pentafluorpentylthiomethyl)-pyrrolidin-1-yl]-pentyl}estra-1,3,5(10)-trien-3,17β-diol

11β-Fluor-7α-{5-[(2S)-2-(4,4,5,5,5-pentafluorpentansulfinylmethyl)-pyrrolidin-1-yl]-pentyl}estra-1,3,5(10)-trien-3,17β-diol

11β-Fluor-7α- {5-[(2S)-2-(4,4,5,5,5-pentafluorpentansulfonylmethyl)-pyrrolidin-1-yl]-pentyl}-estra-1,3,5(10)-trien-3,17β-diol

$R^{11}$ ein Fluor- oder Chloratom,

$R^{17}$ ein Wasserstoffatom oder einen Rest der Teilformel $R^{17'}$—C(O)—, worin $R^{17'}$ ein Wasserstoffatom oder ein Kohlenwasserstoffrest mit bis zu 8 Kohlenstoffatomen ist.

Die disclaimten Verbindungen sind bereits in der WO 98/07740 beschrieben. Eine Variant der Erfindung sieht vor, daß die Seitenkette $R^7$ nicht die in der WO 98/07740 dort für SK angegebenen Bedeutungen haben kann.

$R^7$ kann die in der EP 138 504 B1 dort für die 7α-Seitenkette des Steroids beschriebenen Bedeutungen haben.

Die erfindungsgemäßen 7α-substituierte-Estratriene weisen als $R^3$ vorzugsweise ein Wasserstoffatom auf. Die Hydroxygruppe kann aber auch mit einem gerad- oder verzweigtkettigen, gesättigten oder ungesättigten Kohlenwasserstoffrest mit bis zu 8 Kohlenstoffatomen, wie z.B. einer Methyl-, Ethyl-, Propyl-, Isopropyl-, Butyl-, Isobutyl-, tert. -Butyl-, Pentyl,- Isopentyl-, Neopentyl-, Heptyl-, Hexyl- oder Octylrest verethert oder mit einem Acylrest $R^{3'}$—C(O)—, worin $R^{3'}$ ein Wasserstoffatom oder ein Kohlenwasserstoffrest mit bis zu 8 Kohlenstoffatomen oder ein Phenylrest ist, verestert sein.

Für den Substituenten $R^{11}$ ist eín Fluoratom bevorzugt.

Für den Substituenten $R^{17}$ kann ein Wasserstoffatom oder einen Rest der Teilformel $R^{17'}$— C(O)— stehen, worin $R^{17'}$ ein Wasserstoffatom oder ein Kohlenwasserstoffrest mit bis zu 8 Kohlenstoffatomen ist. Ein Wasserstoffatom

ist für $R^{17}$ bevorzugt. Der Kohlenwasserstoffrest kann beispielsweise die Bedeutung eines Methyl-, Ethyl-, Propyl-, Isopropyl-, Butyl-, Isobutyl-, tert.-Butyl-, Pentyl,- Isopentyl-, Neopentyl-, Heptyl-, Hexyl- oder Octylrestes haben.

In den erfindungsgemäßen Verbindungen der allgemeinen Formel I steht A in fiir eine direkte Bindung, einen Phenylen- oder Benzylidenrest, wobei in letztem Falle die Methylengruppe an das Kohlenstoffatom 7 des Steroidgerüstes gebunden ist.

Beim einem Arylrest handelt es sich im Sinne der vorliegenden Erfindung um einen Phenyl-, 1- oder 2-Naphthylrest; der Phenylrest ist bevorzugt. Wenn nicht ausdrücklich erwähnt, schließt Aryl immer auch einen Heteroarylrest mit ein. Beispiele fiir einen Heteroarylrest sind der 2-, 3- oder 4-Pyridinyl-, der 2- oder 3-Furyl-, der 2- oder 3-Thienyl-, der 2- oder 3-Pyrrolyl, der 2-, 4- oder 5-Imidazolyl-, der Pyrazinyl-, der 2-, 4- oder 5-Pyrimidinyl- oder 3-oder 4-Pyridazinylrest.

Aralkylgruppen in $R^{20}$ und $R^{31}$ können im Ring bis 14 C-Atome, bevorzugt 6 bis 10 C-Atome, und in der Alkylkette 1 bis 8, bevorzugt 1 bis 4 C-Atome, enthalten. Als Aralkylreste kommen beispielsweise in Betracht Benzyl, Phenylethyl, Phenylpropyl, Naphthylmethyl, Naphthylethyl.
Als Heteroarylalkylrest seien beispielsweise Furylmethyl, Thienylethyl, Pyridylpropyl genannt.
Der Aralkyl- bzw. Heteroarylalkylrest kann substituiert sein.

Wenn $R^{20}$ und $R^{21}$ mit dem Stickstoffatom, an das sie gebunden sind, einen gesättigten oder ungesättigten Heterocyclus mit 5 oder 6 Kettengliedern, der gegebenenfalls ein oder zwei weitere Heteroatome, ausgewählt aus Stickstoff, Sauerstoff und Schwefel, enthält, ist dies insbesondere ein Pyrrolidin-, Piperidin-, Morpholin- oder Piperazinring.

Als Substituenten fiir den Aryl-, Heteroaryl-, Aralkyl- und Heteroarylalkylrest seien beispielsweise ein Trifluormethyl-, Pentafluorethyl-, Trifluormethylthio-, Methoxy-, Ethoxy-, Nitro-, Cyano-, Halogen- (Fluor, Chlor, Brom, Iod), Hydroxy-, Amino-, Mono($C_{1-8}$-alkyl)-oder Di($C_{1-8}$-alkyl)amino, wobei beide Alkylgruppen identisch oder verschieden sind, Di(aralkyl)amino, wobei beide Aralkylgruppen identisch oder verschieden sind (Aralkyl siehe oben bei $R^{20}$ und $R^{31}$) oder der 1-Methoxyacetylaminorest genannt.

Das Schwefelatom in der Seitenkette kann als einfache Schwefelbrücke (Sulfid), als Sulfon oder Sulfoxid vorliegen.

Für die Seitenkette $R^7$ gemäß vorliegender Erfindung können beispielsweise folgende Reste stehen (A bedeutet eine direkte Bindung):

ein Rest der Formel $-(CH_2)_s-Z-R^{20}$,
wobei
s eine ganze Zahl von 3 bis 8 ist,
Z für $-NR^{21}$ und $R^{21}$ für eine $C_1$-$C_3$-Alkylgruppe steht,
worin $R^{20}$
ein Wasserstoffatom,
eine $C_1$-$C_9$-Alkylgruppe, oder
eine der Gruppierungen
$-(CH_2)_m-C_nF_{2n+1}$, wobei m und n unabhängig voneinander jeweils eine ganze Zahl von 1 bis 8 ist,
$-(CH_2)_o-CH=CF-C_pF_{2p+1}$, wobei o 1, 2 oder 3 und p eine ganze Zahl von 2 bis 7 ist,
$-(CH_2)_m-O-(CH_2)_q$-Aryl, wobei m die bereits angegebenen Bedeutung hat, q 0, 1,2 oder 3 ist und Aryl für einen gegebenenfalls ein- oder zweifach substituierten Phenyl- oder Heteroarylrest steht,
$-(CH_2)_m-O-(CH_2)_r-C_nF_{2n+1}$, wobei m und n die bereits angegebenen Bedeutungen haben und r für eine ganze Zahl von 1 bis 5 steht,
bedeutet;

ein Rest der Formel $-(CH_2)_s-Z-R^{20}$,
wobei
s eine ganze Zahl von 3 bis 8 ist,
Z für $-NR^{21}$ und $R^{21}$ für eine $C_1$-$C_3$-Alkylgruppe steht,
worin $R^{20}$ und $R^{21}$
mit dem Stickstoffatom, an das sie gebunden sind, einen gesättigten oder ungesättigten Heterocyclus mit 5 oder 6 Kettengliedern bilden, der gegebenenfalls ein oder zwei weitere Heteroatome, ausgewählt aus Stickstoff, Sauerstoff und Schwefel, enthält und gegebenenfalls substituiert ist,
bedeutet;

ein Rest der Formel $-(CH_2)_s-Z-R^{20}$,
wobei
s eine ganze Zahl von 3 bis 8 ist,

Z fiir $-SO_x-$ und x fiir 0, 1 oder 2 steht,
wobei $R^{20}$
$-(CH_2)_m-O-(CH_2)_r-C_nF_{2n+1}$, wobei m und n die bereits angegebenen Bedeutungen haben und r fiir eine ganze Zahl von 1 bis 5 steht,
bedeutet.

Als spezifische Seitenketten seien genannt

$-(CH_2)_5N(CH_3)(CH_2)_3C_2F_5$
$-(CH_2)_5N(CH_3)(CH_2)_6C_2F_5$
$-(CH_2)_5N(CH_3)(CH_2)_7C_2F_5$
$-(CH_2)_5N(CH_3)(CH_2)_8C_2F_5$
$-(CH_2)_6N(CH_3)(CH_2)_6C_2F_5$
$-(CH_2)_6N(CH_3)(CH_2)_7C_2F_5$
$-(CH_2)_6N(CH_3)(CH_2)_8C_2F_5$
$-(CH_2)_5N(CH_3)(CH_2)_2C_4F_9$
$-(CH_2)_5N(CH_3)(CH_2)_3C_6F_{13}$
$-(CH_2)_5N(CH_3)(CH_2)_3C_8F_{17}$
$-(CH_2)_5N(CH_3)(CH_2)_6C_4F_9$
$-(CH_2)_5N(CH_3)(CH_2)_6C_6F_{13}$
$-(CH_2)_5N(CH_3)(CH_2)_6C_8F_{17}$
$-(CH_2)_5N(CH_3)H$
$-(CH_2)_5N(CH_3)(CH_2)_9H$
$-(CH_2)_5N(CH_3)CH_2CH=CF-C_2F_5$
$-(CH_2)_5N(CH_3)CH_2CH=CF-C_3F_7$
$-(CH_2)_5N(CH_3)CH_2CH=CF-C_5F_{11}$
$-(CH_2)_5N(CH_3)CH_2CH=CF-C_7F_{15}$
$-(CH_2)_5$-1-Pyrrolidinyl
$-(CH_2)_5N(CH_3)(CH_2)_3$OPhenyl
$-(CH_2)_5N(CH_3)(CH_2)_3$OBenzyl
$-(CH_2)_5N(CH_3)(CH_2)_3O(CH_2)_3C_2F_5$
$-(CH_2)_9S(CH_2)_3C_2F_5$
$-(CH_2)_9SO(CH_2)_3C_2F_5$
$-(CH_2)_9SO_2(CH_2)_3C_2F_5$
$-(CH_2)_5N(CH_3)(CH_2)_3CH(CH_3)_2$
$-(CH_2)_5N(CH_3)(CH_2)_3$-Pyridyl
$-(CH_2)_5N(CH_3)(CH_2)_3$-Phenyl
$-(CH_2)_5N(CH_3)(CH_2)_2$-p-Tolyl
$-(CH_2)_5N(CH_3)(CH_2)_2$-p-Ethoxyphenyl
$-(CH_2)_5N(CH_3)(CH_2)_3$-p-Tolyl
$-(CH_2)_5N(CH_3)(CH_2)_3$-p-Chlorphenyl
$-(CH_2)_5N(CH_3)(CH_2)_3-O-CH_2$-Phenyl
$-(CH_2)_5N(CH_3)(CH_2)_2$-O-p-Br-Phenyl
$-(CH_2)_5N(CH_3)(CH_2)_2-O-p-CF_3$-Phenyl

Spezifische Verbindungen der allgemeinen Formel I sind in den Beispielen beschrieben.

Die vorliegende Erfindung betrifft außer diesen Verbindungen der allgemeinen Formel I, wenn in R7 ein Stickstoffatom enthalten ist, auch deren physiologisch verträgliche Additionssalze mit organischen und anorganischen Säuren, diese Verbindungen der allgemeinen Formel I inclusive der Additionssalze enthaltende pharmazeutische Präparate sowie deren Verwendung zur Herstellung von Arzneimitteln.

Zur Bildung von Säureadditionssalzen sind anorganische und organische Säuren geeignet, wie sie dem Fachmann zur Bildung physiologisch verträglicher Salze bekannt sind. Als Additionssalze mit Säuren sind insbesondere die Hydrochloride, Hydrobromide, Acetate, Citrate, Oxalate, Tartrate und die Methansulfonate zu nennen.

Die Verbindungen der allgemeinen Formel I stellen Verbindungen mit starker antiestrogener Wirksamkeit dar.

Bei den erfindungsgemäßen Verbindungen handelt es sich zum einen Teil um reine Antiestrogene oder zum anderen Teil um sogenannte Partialantagonisten, d. h. um Antiestrogene mit estrogener Partialwirkung wie das Tamoxifen oder das Raloxifen. Im Gegensatz zum Tamoxifen tritt bei den Partialantagonisten der allgemeinen Formel I deren agonistische, estrogene Wirkung gewebeselektiv auf. Insbesondere tritt die agonistische Wirkung am Knochen,

im Herz-Kreislaufsystem und im ZNS (Zentrales Nervensystem) auf. Insbesondere tritt am Uterus keine oder nur geringe agonistische Wirkung tritt auf.

Verbindungen mit antiestrogenen Eigenschaften, d.h. Stoffe mit Hemmwirkungen gegenüber Estrogenen, sind bereits zahlreich beschrieben worden.

Als den vorliegenden Verbindungen der allgemeinen Formel I strukturell am nächsten kommende Verbindungen sind die aus der EP 0 138 504 B1 hervorgehenden Steroid-Derivate anzusehen. Das 7α-[9-(4,4,5,5,5-Pentafluorpentylsulfinyl)-n-nonyl]-estra-1,3,5(10)-trien-3,17β-diol (EP-A 0 138 504, Seite 58, vorletzte Verbindung) befindet sich gegenwärtig in klinischer Entwicklung für hormonabhängige Tumoren (Brustkrebs) und stellt die derzeit beste bekannte Verbindung, d.h. diejeniege mit der stärksten antiestrogenen Wirksamkeit, dieser Steroid-Derivate dar.

Pharmazeutische Zusammensetzungen, die Sexualsteroid-Inhibitoren enthalten, welche ein steroidales Grundgerüst, das eine 7α-Seitenkette bei gleichzeitiger Anwesenheit mindestens eines weiteren Substituenten in Position 14, 15 oder 16 aufweist, sind Gegenstand der EP-A 0 367 576.

Eine Vielzahl verschiedenartigster Verbindungen - u. a. solche steroidalen Ursprungs als auch solche mit 2-Phenylindol-Grundgerüst - die als Antiestrogen wirken und/oder die Estrogenbiosynthese unterdrücken, werden in der WO 93/10741 offenbart.

Weitere steroidale Antiestrogene, die einen 11β-Phenylrest tragen, sind in den EP-AS 0 384 842 und 0 629 635 beschrieben.

Bei den erfindungsgemäßen Verbindungen handelt es sich um Antiestrogene mit meist stärkerer antiestrogener Wirkung als das schon erwähnte 7α-[9-(4,4,5,5,5-Pentafluorpentylsulfinyl)-n-nonyl]-estra-1,3,5(10)-trien-3,17β-diol und/oder um Verbindungen, die sich von 7α-[9-(4,4,5,5,5-Pentafluorpentylsulfinyl)-n-nonyl]-estra-1,3,5(10)-trien-3,17β-diol durch ihre estrogene Partialwirkung unterscheiden.

Die Verbindungen der allgemeinen Formel I gemäß vorliegender Anmeldung zeichnen sich im Vergleich zu den bereits bekannten Steroid-Derivaten gemäß der EP-A 0 138 504 und EP-A 0 367 576 durch das zusätzliche 11β-Fluoratom aus.

Diese Strukturmodifikation ist für die interessanten Eigenschaften der erfindungsgemäßen Verbindungen von entscheidender Bedeutung.

Die antiestrogene Wirkung der erfindungsgemäßen Verbindungen wurde in Transaktivierungsassays bestimmt [Demirpence E., Duchesne M.-J., Badia E., Gagne D. und Pons M.: MVLN Cells: A Bioluminescent MCF-7-Derived Cell Line to study the Modulation of Estrogenic Activity; J. Steroid. Molec. Biol. Vol. 46, No. 3, 355 - 364 (1993) sowie Berry M., Metzger D. Chambon P.: Role of the two activating domains of the estrogen receptor in the cell-type and promoter-context dependent agonistic activity of the anti-estrogen 4-hydroxytamoxifen; The EMBO Journal Vol. 9, 2811 - 2818 (1990)].

Die MVLN-Zellen sind stabil mit dem Reportergen Vit-TK-LUC transfiziert. Es wurde die antiestrogene Wirkstärke in Gegenwart von 0,1 nM Estradiol bestimmt.

Die $IC_{50}$-Werte für die neuen Verbindungen liegen im nanomolaren Bereich. In der MVLN-Zellinie ergeben sich für die Verbindungen der Beispiele 3 und 8 folgende $IC_{50}$-Werte (Testdurchführung gemäß der oben angegebenen Literaturstellen):

| | IC50 [nM] | IC50 [nM] |
|---|---|---|
| Verbindung | MVLN | HeLa |
| Beispiel 3 | 6,3 | 0,2 |
| Beispiel 8 | 37 | 8 |

Der Uteruswachstumstest bei der infantilen Ratte, p.o. (Test auf antiestrogene Wirkung invivo) belegt ebenfalls die antiestrogene Wirksamkeit der erfindungsgemäßen Verbindungen. Der Test wurde wie nachstehend beschrieben durchgeführt:

**Uteruswachstumstest bei der infantilen Ratte (antiestrogene Wirkung)**

Prinzip der Methode

Bei Nagern reagiert der Uterus auf die Applikation von estrogenen mit einer Gewichtszunahme (sowohl Proliferation als auch Wassereinlagerung). Dieses Wachstum ist durch gleichzeitige Gabe antiestrogen-wirkender Verbindungen dosisabhängig zu hemmen.

**Versuchsdurchführung**

Tiere:

Infantile weibliche Ratten im Gewicht von 35-45 g bei Versuchsbeginn, pro Dosis 5-6 Tiere.

Formulierung und Applikation der Substanzen:

Für die p.o. Applikation werden die Substanzen in 1 Teil Ethanol (E) gelöst und mit 9 Teilen Erdnußöl (EÖ) aufgefüllt.

Versuchsansatz

Die gerade von den Müttern abgesetzten jungen Ratten werden zur Eingewöhnung einen Tag vor Behandlungsbeginn geliefert und sofort mit Futter - auch in dem Tierkäfig - versorgt.
Die Behandlung erfolgt dann täglich einmal über 3 Tage in Kombination mit 0,5 µg Estradiolbenzoat (EB). EB wird immer subcutan (s.c.) appliziert, während die Testsubstanz p.o. (peroral) verabreicht wird. 24 Stunden nach der letzten Applikation werden die Tiere gewogen, getötet und die Uteri entnommen. Von den präparierten Uteri werden die Feuchtgewichte (ohne Inhalt) ermittelt.

Kontrollen

negative Kontrolle: Vehikel (E/EÖ), 0,2 ml/Tier/Tag
positive Kontrolle: 0,5 µg EB/0,1 ml/Tier/Tag

Auswertung

Von den relativen Organgewichten (mg/100 g Körpergewicht) werden für jede Gruppe die Mittelwerte mit Standardabweichung (X+SD), sowie die Signifikanz der Unterschiede zur Kontrollgruppe (EB) im Dunnett-Test ($p<0.05$) ermittelt. Die Berechnung der Hemmung (in %) gegenüber der EB-Kontrolle erfolgt mit einem Programm. Die relativen Wirksamkeiten der Prüfsubstanzen werden durch eine Kovarianz- und Regressionsanalyse ermittelt.

| **Antiuterotrophe Wirkung an der Ratte** | |
|---|---|
| Verbindung aus Beispiel | Antiuterotrophe Wirkung bei 0,3 mg [% Hemmung] |
| 3 | 68 |
| 8 | 0 |

Als reine Antiestrogene im Sinne vorliegender Erfindung sind solche Verbindungen der allgemeinen Formel I anzusehen, die im nachstehend beschriebenen in-vitro Test auf estrogene Wirkung keine oder bestenfalls nur geringfügige agonistische Wirkung, d.h. eine agonistische Wirkung bis zu etwa 20 % der Wirkung von Estradiol, zeigen.

**Estrogene Partialwirkung**

Der Übergang zwischen den reinen Antiestrogenen und den Partialagonisten, den gewebeselektiven Estrogenen, ist fließend. Verbindungen, die eine geringfügige agonistische Wirkung aufweisen, können ebenso in den nachfolgend für die reinen Antiestrogene genannten Indikationen verwendet werden

Die estrogene Partialwirkung erfindungsgemäßer Verbindungen wurde durch Transaktivierungsassays bestimmt. HeLa Zellen wurden mit humanem Estrogenrezeptor-Expressionsvektor (HEGO) und einem Reportergen rPR-TK-CAT transfiziert. Dieses Reportergen enthält das "Estrogen Responsive Element" des Kaninchen Progesteronrezeptorgens (+698/+729-Region) vor einem TK-CAT Gen (Savouret J.F., Bailly A., Misrahi M., Rauch C., Redeuilh G., Chauchereau A., Milgrom E., Characterization of the hormone responsive element involved in the regulation of the progesterone receptor gene. EMBO J. 10, 1875 - 1883 (1991).
Es wurde die estrogene Wirkstärke bei einer Konzentration von 1µM bestimmt.

| Estrogene Partialwirkung | |
|---|---|
| Verbindung aus Beispiel | Aktivierung des rPR-TK-Promotors [% Estradiol]* |
| 3 | -25 |
| 8 | 48 |
| 18 | 29 |
| 12 | 34 |
| 22 | 53 |

*Ein negativer Wert bedeutet Suppression der Reportergen-Aktivität unterhalb die Werte der Kontrollen

Die erfindungsgemäßen Verbindungen, insbesondere wenn es reine Antiestrogene sind, eignen sich zur Therapie von estrogen-abhängigen Erkrankungen, zum Beispiel Mammacarcinom (second-line Therapie des Tamoxifen-resistenten Mammacarcinoms; zur adjuvanten Behandlung des Mammacarcinoms anstelle von Tamoxifen), Endometriumcarcinom, Prostatacarcinom, Prostatahyperplasie, anovulatorische Infertilität und Melanom.
Die reinen Antiestrogene der allgemeinen Formel I können außerdem als Komponente in den in der EP 346 014 B 1 beschriebenen Produkten verwendet werden, die ein Estrogen und ein reines Antiestrogen enthalten, und zwar zur gleichzeitigen, sequentiellen oder getrennten Verwendung für die selektive Estrogentherapie peri- oder postmenopausaler Frauen.
Die Verbindungen der allgemeinen Formel I, insbesondere wenn es sich um reine Antiestrogene handelt, können gemeinsam mit Antigestagenen (kompetitiven Progesteronantagonisten) zur Behandlung hormonabhängiger Tumoren verwendet werden (EP 310 542 A).
Weitere Indikationen, in denen die Verbindungen der allgemeinen Formel zum Einsatz kommen können, ist der männliche Haarausfall, eine diffuse Alopecie, eine durch eine Chemotherapie hervorgerufene Alopecie sowie Hirsutismus (Hye-Sun Oh und Robert C. Smart, Proc. Natl. Acad. Sci. USA, 93 (1996) 12525 - 12530).
Außerdem können die Verbindungen der allgemeinen Formel I zur Herstellung von Medikamenten zur Behandlung der Endometriose und von Endometrialkarzinomen verwendet werden.
Ferner kann man die Verbindungen der allgemeinen Formel I zur Herstellung pharmazeutischer Zusammensetzungen für die männliche und weibliche Fertilitätskontrolle einsetzen (männliche Fertilitätskontrolle: DE-A 195 10 862.0).

Diejenigen Verbindungen der allgemeinen Formel I mit gewebeselektiver estrogener Partialwirkung können in erster Linie zur Prophylaxe und Therapie der Osteoporose und zur Herstellung von Präparaten fiir die Substitutionstherapie in der Prae-, Peri- und Postmenopause (HRT = Hormone Replacement Therapy; Hormonersatz-Therapie) Verwendung finden (Black, L.J., Sato,M.,Rowley, E.R.,Magee, D.E., Bekele, A., Williams, D.C., Cullinan, G.J., Bendele, R., Kauffman, R.F., Bensch, W.R., Frolik, C.A., Termine, J.D. and Bryant, H.U.: Raloxifene [LY 139481 HCI] prevents bone loss and reduces serum cholesterol without causing uterine hypertrophy in ovariectomized rats; J. Clin. Invest. 93: 63 - 69,1994).

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere zur Behandlung von estrogenabhängigen Krankheiten und Tumoren und von Arzneimitteln für die Hormonsubstitutions-Therapie (HRT).

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 u. ff.; H.v. Czetsch-Lindenwald, Hilfsstoffe für Pharmazie und angrenzende Gebiete; Pharm. Ind. Heft 2, 1961, Seite 72 u. ff.; Dr. H.P. Fiedler, Lexikon der Hilfsstoffe für Pharmazie, Kosmetik und angrenzende Gebiete Cantor KG. Aulendorf in Württemberg 1971.

Die Verbindungen können oral oder parenteral, beispielsweise intraperitoneal, intramuskulär, subkutan oder perkutan, verabreicht werden. Die Verbindungen können auch in das Gewebe implantiert werden. Die zu verabreichende Menge der Verbindungen schwankt innerhalb eines weiten Bereichs und kann jede wirksame Menge abdecken.

In Abhängigkeit des zu behandelnden Zustandes und der Art der Verabreichung kann die Menge der verabreichten Verbindung 0,1-25 mg/kg Körpergewicht, vorzugsweise 0,5-5 mg/kg Körpergewicht, je Tag betragen. Beim Menschen entspricht dies einer täglichen Dosis von 5 bis 1250 mg.
Die bevorzugte tägliche Dosierung beim Menschen ist 50 bis 200 mg. Dies gilt insbesondere für die Tumortherapie.

Zur oralen Verabreichung kommen Kapseln, Pillen, Tabletten, Dragèes usw. infrage. die Dosierungseinheiten können neben dem Wirkstoff einen pharmazeutisch verträglichen Träger, wie zum Beispiel Stärke, Zucker, Sorbit, Gelantine, Gleitmittel, Kieselsäure, Talkum usw., enthalten. Die einzelnen Dosierungseinheiten für die orale Applikation können beispielsweise 5 bis 500 mg des Wirkstoffs enthalten.

Um eine bessere Bioverfügbarkeit des Wirkstoffes zu erreichen, können die Verbindungen auch als Cyclodextrinclathrate formuliert werden. Hierzu werden die Verbindungen mit α-, β-oder γ-Cyclodextrin oder Derivaten von diesen umgesetzt (PCT/EP95/02656).

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.

Die Verbindungen der allgemeinen Formel I können auch in Form einer Lösung formuliert werden, die für die orale Verabreichung bestimmt ist und die neben der aktiven Verbindung der allgemeinen Formel I enthält

a) ein pharmazeutisch verträgliches Öl und/oder
b) eine pharmazeutisch verträgliche lipophile oberflächenaktive Substanz und/oder
c) eine pharmazeutisch verträgliche hydrophile oberfächenaktive Substanz und/oder
d) ein pharmazeutisch verträgliches wassermischbares Lösungsmittel.

Hierzu wird außerdem auf die WO 97/21440 verwiesen.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel I beladenen Intravaginal- (z.B. Vaginalringe) oder Intrauterinsystemen (z.B. Pessare, Spiralen) eignen sich verschiedene Polymere wie zum Beispiel Silikonpolymere, Ethylenvinylacetat, Polyethylen oder Polypropylen.

Die erfindungsgemäßen Verbindungen können wie nachstehend beschrieben hergestellt werden. Die nachfolgenden Beispiele dienen der näheren Erläuterung der Erfindung.
Durch analoge Vorgehensweise unter Verwendung analoger Reagenzien zu den in den Beispielen enthaltenen Angaben lassen sich weitere Verbindungen der allgemeinen Formel I erhalten.

Als Verfahren zum Aufbau der Seitenkette $R^7$ in den erfindungsgemäßen Verbindungen sind insbesondere auch die in der EP 0 138 504 B1 beschriebenen Methoden der Seitenketteneinführung und des Seitenkettenaufbaus geeignet, wobei dann als Startverbindung anstelle von $\Delta^6$-Nortestosteron, dessen 17-Hydroxyfunktion acyliert ist, die 11β-Fluorverbindung 11β-Flour- $\Delta^6$-androstendion zu verwenden ist. Die Reduktion der 17-Ketogruppe findet dann auf einer späteren Stufe statt.

Die Verbindungen der allgemeinen Formel I, worin $R^{31}$ ein Rest der Formel
$-C(O)R^{33}$ ist, lassen sich durch vollständige Reduktion der Ketogruppe des Carbonsäureamids mit Lithiumaluminiumhydrid oder ähnlichen Reduktionsmitteln nach dem Fachmann geläufigen Methoden in die Verbindungen überführen, worin $R^{31}$ dann $-CH_2-R^{33}$ ist.

Eine Thiobrücke in der Seitenkette kann mit Natriumperiodat zum Sulfoxid oxidiert werden (Beispiel 24 n)); mit einer Persäure als Oxidationsmittel, z.B. *m*-Chlorperbenzoesäure, werden aus den Sulfiden die Sulfone erhalten.

Die Verseifung von Estergruppierungen sowieVeresterung und Veretherung freier Hydroxygruppen erfolgt jeweils nach etablierten Verfahren der organischen Chemie. Durch Beachtung der unterschiedlichen Reaktivität der veresterten und freien 3- und 17-Hydroxygruppe lassen sich die 3,17-Diester selektiv in 3-Position spalten und die 3-Hydroxy-17-acyloxy-Verbindung läßt sich dann gezielt in 3-Position weiter funktionalisieren; genauso gut ist es möglich, die 3,17-Dihydroxyverbindung selektiv nur in 3-Position zu verestern oder zu verethern und dann gezielt in 17-Position einen anderen als bereits in 3-Stellung sich befindenden Rest einzuführen.

Die Säureadditionssalze der Verbindungen der allgemeinen Formel I lassen sich ebenfalls nach gängigen Verfahren aus den Verbindungen der allgemeinen Formel I herstellen.

Die nachfolgenden Beispiele dienen der näheren Erläuterung der Erfindung:

**Beispiele**

Beispiel 1

**11β-Fluor-7α-{5-[methyl-(8,8,9,9,9-pentafluor-nonyl)-amino}-pentyl}-estra-1,3,5(10)-trien-3,17β-diol**

**a) 11β-Fluor-estr-4-en-3,17-dion**

Zu 5,0 g 11α-Hydroxy-estr-4-en-3,17-dion in 100 ml Toluol und 7,3 ml 1,8-Diazabicyclo[5,4,0]undec-7-en werden bei 0°C 4,6 ml Perfluorbutan-1-sulfonsäurefluorid getropft. Nach 30 min verdünnt man die Lösung mit Essigester, wäscht mit gesättigter Natriumchlordlösung, trocknet und engt im Vakuum ein. Nach Chromatographieren des Rohproduktes an Kieselgel mit einem Hexan-Essigester -Gradienten werden 3,8 g 11β-Fluorestr-4-en-3,17-dion vom Schmelzpunkt 173-174°C erhalten.

**b) 11β-Fluor-3-methoxy-estra-3,5-dien-17-on**

7,8 g 11β-Fluor-estr-4-en-3,17-dion werden in 40 ml 2,2-Dimethoxypropan mit 780 mg Pyridinium-toluol-4-sulfonat 5 h bei 80°C gerührt. Anschließend setzt man 1,5 ml Triethylamin zu, verdünnt mit Essigester und wäscht mit gesättigter Natriumchlorilösung. Nach Kristallisieren aus Methanol werden 5,3 g 11β-Fluor-3-methoxy-estra-3,5-dien-17-on vom Schmelzpunkt 173°C erhalten.

**c) 11β-Fluor-estra-4,6-dien-3,17-dion**

Zu 5,0 g 11β-Fluor-3-methoxy-estra-3,5-dien-17-on in 50 ml DMF gibt man bei 0°C nacheinander 5 ml einer 10 proz. Natriumacetatlöung und portionsweise 2,5 g 1,3-Dibrom-5,5-dimethylhydantoin. Nach 30 min setzt man 2,3 g Natriumsulfit und anschließend 2,5 g Lithiumbromid und 2,0 g Lithiumcarbonat zu.und rührt 2 h bei 100°C. Das Reaktionsgemisch wird in Eis-Wasser eingerührt. Man saugt das ausgefallene Produkt ab, löst in Essigester, wäscht mit Wasser, trocknet und engt im Vakuum ein. Nach Umkristallisieren aus Essigester werden 3,6g 11β-Fluor-estra-4,6-dien-3,17-dion vom Schmelzpunkt 198°C erhalten.

**d) 11β-Fluor-7α-(5-*tert.*-butyl-dimethylsilyloxypentyl)-estr-4-en-3,17-dion**

7,9 g Magnesium in 40 ml THF werden unter Stickstoff mit einer Lösung von 95,3 g 1-Brom-5-tert.-butyldimethylsilyloxypentan [Tetrahedron Letters 1982, 4147-4150] in 260 ml THF zum Grignardreagenz umgestzt. Bei -30°C gibt man 32 g Kupfer(I)-iodid und anschließend tropfenweise 29 g 11β-Fluor-estra-4,6-dien-3,17-dion in 290 ml THF zu. Nach beendeter Reaktion versetzt man mit 20,4 ml Eisessig und rührt das Reaktionsgemisch in Eis-Wasser ein. Das ausgefallene Produkt wird abgesaugt, in Essigester gelöst, mit Wasser neutral gewaschen und getrocknet. Nach Chromatographieren des Rohproduktes an Kieselgel mit einem Hexan-Essigester-Gradienten werden 23,9 g 11β-Fluor-7α-(5-*tert.*-butyldimethylsilyloxypentyl)-estr-4-en-3,17-dion als Schaum erhalten.

**e) 11 β-Fluor-7α-(5-hydroxypentyl)-estr-4-en-3,17-dion**

Eine Lösung von 23,1 g 11β-Fluor-7α-(5-*tert.*-butyl-dimethylsilyloxypentyl)-estr-4-en-3,17-dion in 115 ml THF und 64 ml Wasser werden mit 128 ml Eisessig 2,5 h bei 50°C gerührt. Das Reaktionsgemisch wird im Vakuum eingeengt, in Essigester aufgenommen, mit Wasser gewaschen und getrocknet. Man erhält 20,4 g 11β-Fluor-7α-(5-hydroxypentyl)-estr-4-en-3,17-dion als Schaum.

**f) 7α-(5-Brompentyl)-11β-fluor-estr-4-en-3,17-dion**

Eine Lösung von 33 g 11β-Fluor-7α-(5-hydroxypentyl)-estr-4-en-3,17-dion in 330 ml Dichlormethan wird bei -5 °C mit 28,9 g Triphenylphosphin und 36,7 g Tetrabromkohlenstoff versetzt und 0,5 Stunden gerührt. Anschließend setzt man Dichlormethan zu und wäscht mit Wasser, gesättigter Natriumhydrogencarbonat- und Kochsalzlösung. Die organische Phase wird über Natriumsulfat getrocknet und i.Vak. eingeengt. Dann wird das Rohprodukt an Kieselgel mit einem Hexan-Essigester-Gradienten chromatographiert. Es werden 28,5 g 7α-(5-Brompentyl)-11β-fluor-estr-4-en-3,17-dion vom Schmelzpunkt 75-76 °C erhalten.

**g) 7α-(5-Brompentyl)-11β-fluor-3-hydroxy-estra-1,3,5(10)-trien-17-on**

Zu 27,8 g 7α-(5-Brompentyl)-11β-fluor-estr-4-en-3,17-dion in 190 ml Acetonitril werden bei 80°C 17,0 g Kupfer (II)-bromid gegeben. Nach 8 Stunden wird das Reaktionsgemisch in Wasser eingerührt, dreimal mit Essigester extrahiert, zweimal mit Ammoniumchlorid, mit Natriumhydrogencarbonat und Kochsalz gewaschen, getrocknet und im Vakuum eingeengt. Nach Chromatographieren des Rohproduktes an Kieselgel mit einem Hexan-Essigester-Gradienten werden 20,4 g 7α-(5-Brompentyl)-11β-fluor-3-hydroxy-estra-1,3,5(10)-trien-17-on als farblose Kristalle vom Schmelzpunkt 178 °C.

**h) 7α-(5-Brompentyl)-11β-fluor-estra-1,3,5(10)-trien-3,17β-diol**

Eine Lösung von 16,2 g 7α-(5-Brompentyl)-11β-fluor-3-hydroxy-estra-1,3,5(10)-trien-17-on in 162 ml Tetrahydrofuran sowie 90 ml Ethanol und 36 ml Wasser wird bei 0 °C mit 4,7 g Natriumborhydrid portionsweise versetzt und 2 Stunden bei 0 °C gerührt. Dann wird auf Wasser gegeben, viermal mit Essigester extrahiert, mit Wasser und Kochsalzlösung gewaschen, über Natriumsulfat getrocknet und i.Vak. eingeengt. Man erhält 17,1 g Rohprodukt. Nach Chromatographie an Kieselgel mit Hexan / Essigester erhält man 15,6 g reines 7α-(5-Brompentyl)-11β-fluor-estra-1,3,5(10)-trien-3,17β-diol.

**i) 11β-Fluor-7α-[5-(methyl-amino)-pestyl]-estra-1,3,5(10)-trien-3,17β-diol**

Eine Lösung von 2 g 7α-(5-Brompentyl)-11β-fluor-estra-1,3,5(10)-trien-3,17β-diol in 20 ml Dimethylformamid wird mit 8 ml einer 40%igen wässrigen Methylaminlösung 3,5 Stunden bei 80 °C gerührt. Dann wird auf Wasser gegeben, dreimal mit Essigester extrahiert, mit Wasser und Kochsalzlösung gewaschen, über Natriumsulfat getrocknet und i.Vak. eingeengt. Man erhält 1,77 g 11β-Fluor-7α-[5-(methyl-amino)-pentyl]-estra-1,3,5(10)-trien-3,17β-diol.

**j) 11β-Fluor-7α-{5-[methyl-(8,8,9,9,9-pentafluor-nonyl)-amino]-pentyl}-estra-1,3,5(10)-trien-3,17β-diol**

Eine Lösung von 1,77 g 11β-Fluor-7α-[5-(methyl-amino)-pentyl]-estra-1,3,5(10)-trien-3,17βdiol in 18 ml Dimethylformamid wird mit 1,4 g 8,8,9,9,9-Pentafluor-nonyltosylat 1 Stunde bei 80 C Badtemperatur gerührt. Dann wird auf Wasser gegeben, dreimal mit Essigester extrahiert, mit Wasser und Kochsalzlösung gewaschen, über Natriumsulfat getrocknet, i.Vak. eingeengt und an Kieselgel mit Dichlormethan / Methanol chromatographiert. Man erhält 11β -Fluor-7α-{5-[methyl-(8,8,9,9,9-pentafluor-nonyl)-amino]-pentyl}-estra-1,3,5(10)-trien-3,17β -diol als Kristalle vom Schmelzpunkt 110 C.

Herstellung der Ausgangsverbindungen:

**8,8,9,9,9-Pentafluor-nonyltosylat**

**a) 4-Benzyloxy-butan-1-ol**

42 g Natriumhydrid (60%ig) werden bei Raumtemperatur portionsweise in 900 ml abs. DMF eingetragen. Zu der auf -20 °C gekühlten Suspension werden 88,6 ml 1,4-Butandiol in 450 ml abs. DMF so zugetropft, daß die Innentemperatur -15 °C nicht übersteigt. Nach beendeter Zugabe wird zügig eine Lösung aus 121 ml Benzylbromid in 870 ml abs. DMF zugetropft und anschließend das Reaktionsgemisch für 30 Minuten bei Raumtemperatur gerührt. Die Reaktion wird durch vorsichtige Zugabe von 315 ml Wasser beendet. Zur Aufarbeitung wird das Reaktionsgemisch in 1,5 1 Wasser eingerührt und 3 mal mit jeweils 11 Ether extrahiert.

Die etherischen Phasen werden vereinigt, mit Wasser gewaschen, über Magnesiumsulfat getrocknet und i. Vak. eingeengt. Chromatographie an Kieselgel mit einem Hexan-Essigester-Gradienten liefert 105 g 4-Benzyloxy-butan-1-ol als Öl.

**b) 4-Benzyloxy-1-brom-butan**

Zu der auf -15 °C gekühlten Lösung aus 105 g 4-Benzyloxy-butan-1-ol und 191 g Triphenylphosphin in 11 Methylenchlorid wird portionsweise 239 g Tetrabrommethan gegeben und nach beendeter Zugabe 1h bei 0 °C gerührt. Nachdem das Reaktionsgemisch i. Vak. eingeengt wurde, erfolgt die Aufreinigung durch Chromatographie an Kieselgel mit einem Hexan-Essigester-Gradienten. Man erhält 133 g 4-Benzyloxy-1-brom-butan als Öl.

**c) 1-Benzyloxy-8,8,9,9,9-pentafluor-nonan**

Zu einer Suspension von 2,23 g Magnesiumspänen in 58 ml abs. THF werden zunächst bei Raumtemperatur 10% einer Lösung aus 20g 4-Benzyloxy-1-brom-butan in 20 ml abs. THF gegeben. Nach dem Anspringen der Reaktion, welches durch Zugabe von Iod erreicht werden kann, wird die restliche Lösung so zugetropft, daß die Innentemperatur bei 40 °C gehalten wird. Nach beendeter Zugabe wird noch 1 Stunde bei Raumtemperatur gerührt bevor vom überschüssigem Magnesium abdekantiert wird und die Lösung in einen Tropftrichter überführt wird. Nun wird diese Lösung gleichzeitig mit einer Lösung aus 21 g 1,1,1,2,2-Pentafluor-5-iod-pentan in 97 ml abs THF zu einer Lösung aus 555 mg Kupfer(II)chlorid und 350 mg Lithiumchlorid in 58 ml abs. THF bei 0 °C getropft. Es wird noch 1 Stunde bei Raumtemperatur gerührt, dann das Reaktionsgemisch in gesättigte Ammoniumchlorid-Lösung eingerührt, 3 mal mit Ether extrahiert, die organische Phase über Magnesiumsulfat getrocknet und i. Vak. eingeengt. Präparative Säulenchromatographie an Kieselgel mit einem Hexan-Essigester-Gradienten liefert 22 g weitgehend aufgereinigtes 1-Benzyloxy-8,8,9,9,9-pentafluor-nonan als Öl.

**d) 8,8,9,9,9-Pentafluor-nonan-1-ol**

16 g 1-Benzyloxy-8,8,9,9,9-pentafluor-nonan werden in 700 ml abs. Methylenchlorid gelöst, mit 18,4 ml N, N-Dimethylanilin bei 0 °C versetzt und 5 Minuten gerührt. Dann fügt man portionsweise 26,4 g Aluminiumtrichlorid zu und erwärmt das Reaktionsgemisch fiir 45 Minuten auf 50 °C. Zur Aufarbeitung läßt man den Ansatz auf Raumtemperatur kommen, rührt ihn in 2N Salzsäure ein, extrahiert 3 mal mit Methylenchlorid, trocknet über Magnesiumsulfat und engt i. Vak. ein. Das Rohprodukt wird an Kieselgel mit einem Hexan-Essigester-Gradienten chromatographiert. Man erhält 8,6 g 8,8,9,9,9-Pentafluor-nonan-1-ol als Öl.

**e) 8,8,9,9,9-Pentafluor-nonyltosylat**

In 26 ml abs. Pyridin löst man 3,0 g 8,8,9,9,9-Pentafluor-nonan-1-ol, gibt bei 0 °C 3,1 g p-Toluolsulfonylchlorid hinzu und rührt für 1,5 Stunden in der Kälte. Anschließend wird das Reaktionsgemisch in Wasser gegeben, 3 mal mit Ether extrahiert, über Magnesiumsulfat getrocknet und i. Vak. eingeengt. Präparative Säulenchromatographie an Kieselgel mit einem Hexan-Essigester-Gradienten führt zu 4,1 g 8,8,9,9,9-Pentafluor-nonyltosylat als klarem Öl.

**9,9,10,10,10-Pentafluor-decyltosylat**

**a) 5-Benzyloxy-pentan-1-ol**

31,5 g Natriumhydrid (60%ig) werden bei Raumtemperatur portionsweise in 900 ml abs. DMF eingetragen. Zu der auf -20 °C gekühlten Suspension werden 104,8 ml 1,5-Pentandiol in 450 ml abs. DMF so zugetropft, daß die Innentemperatur -15 °C nicht übersteigt. Nach beendeter Zugabe wird zügig eine Lösung aus 121 ml Benzylbromid in 870 ml abs. DMF zugetropft und anschließend das Reaktionsgemisch für 30 Minuten bei Raumtemperatur gerührt. Die Reaktion wird durch vorsichtige Zugabe von 315 ml Wasser beendet. Zur Aufarbeitung wird das Reaktionsgemisch in 1,5 1 Wasser eingerührt und 3 mal mit jeweils 11 Ether extrahiert. Die etherischen Phasen werden vereinigt, mit Wasser gewaschen, über Magnesiumsulfat getrocknet und i. Vak. eingeengt. Chromatographie an Kieselgel mit einem Hexan-Essigester-Gradienten liefert 85 g 5-Benzyloxy-pentan-1-ol als Öl.

**b) 5-Benzyloxy-1-brom-pentan**

Zu der auf-15 °C gekühlten Lösung aus 85 g 5-Benzyloxy-pentan-1-ol und 143 g Triphenylphosphin in 720 ml Methylenchlorid wird portionsweise 179 g Tetrabrommethan gegeben und nach beendeter Zugabe 3h bei 0 °C gerührt. Nachdem das Reaktionsgemisch i. Vak. eingeengt wurde, erfolgt die Aufreinigung durch Chromatographie an Kieselgel mit einem Hexan-Methylenchlorid-Gradienten. Man erhält 71 g 5-Benzyloxy-1-brom-pentan als Öl.

**c) 1-Benzyloxy-9,9,10,10,10-pentafluor-decan**

Zu einer Suspension von 2,23 g Magnesiumspänen in 58 ml abs. THF werden zunächst bei Raumtemperatur 10% einer Lösung aus 21,1g 5-Benzyloxy-1-brom-pentan in 20 ml abs. THF gegeben. Nach dem Anspringen der Reaktion, welches durch Zugabe von Iod erreicht werden kann, wird die restliche Lösung so zugetropft, daß die Innentemperatur bei 40 °C gehalten wird. Nach beendeter Zugabe wird noch 1 Stunde bei Raumtemperatur gerührt bevor vom überschüssigem Magnesium abdekantiert wird und die Lösung in einen Tropftrichter überführt wird. Nun wird diese Lösung gleichzeitig mit einer Lösung aus 21 g 1,1,1,2,2-Pentafluor-5-iod-pentan in 97 ml abs THF zu einer

Lösung aus 555 mg Kupfer(II)chlorid und 350 mg Lithiumchlorid in 58 ml abs. THF bei 0 °C getropft. Es wird noch 1 Stunde bei Raumtemperatur gerührt, dann das Reaktionsgemisch in gesättigte Ammoniumchlorid-Lösung eingerührt, 3 mal mit Ether extrahiert, die organische Phase über Magnesiumsulfat getrocknet und i. Vak. eingeengt. Man erhält 26,8 g Rohprodukt, das ohne weitere Aufreinigung in der nächsten Stufe eingesetzt wird.

**d) 9,9,10,10,10-Pentafluor-decan-1-ol**

26 g 1-Benzyloxy-9,9,10,10,10-pentafluor-decan werden in 1000 ml abs. Methylenchlorid gelöst, mit 28,9 ml N,N-Dimethylanilin bei 0 °C versetzt und 5 Minuten gerührt. Dann fügt man portionsweise 41,1 g Aluminiumtrichlorid zu und erwärmt das Reaktionsgemisch für 45 Minuten auf 50 °C. Zur Aufarbeitung läßt man den Ansatz auf Raumtemperatur kommen, rührt ihn in 2N Salzsäure ein, extrahiert 3 mal mit Methylenchlorid, wäscht die organische Phase mit Kochzallösung, trocknet über Magnesiumsulfat und engt i. Vak. ein. Das Rohprodukt wird an Kieselgel mit einem Hexan-Essigester-Gradienten chromatographiert. Man erhält 7,8 g 9,9,10,10,10-Pentafluor-decan-1-ol als Öl.

e) 9,9,10,10,10-Pentafluor-decyltosylat

In 8 ml abs. Pyridin löst man 1,0 g 9,9,10,10,10-Pentafluor-decan-l-ol, gibt bei 0 °C 985 mg p-Toluolsulfonylchlorid hinzu und rührt fiir 2 Stunden in der Kälte. Anschließend wird das Reaktionsgemisch in Wasser gegeben, 3 mal mit Ether extrahiert, über Magnesiumsulfat getrocknet und i. Vak. eingeengt. Präparative Säulenchromatographie an Kieselgel mit einem Hexan-Essigester-Gradienten führt zu 1,5 g 9,9,10,10,10-Pentafluor-decyltosylat als Öl.

**N-Methyl-[3-(4,4,5,5,5-pentafluor-pentylthio)-propyl]-amin**

**a) 3-Iodpropyl-4,4,5,5,5-pentafluor-pentylsulfid**

Eine Lösung von 22,8 g 3-Chlorpropyl-4,4,5,5,5-pentafluor-pentylsulfid in 500 ml Ethylmethylketon wird mit 40 g Natriumiodid 5 Stunden bei 100 C Badtemperatur unter Stickstoff gerührt. Dann wird i. Vak. zur Trockne eingeengt, auf Wasser gegeben, dreimal mit Essigester extrahiert, neutralgewaschen, und über Natriumsulfat getrocknet und i. Vak. eingeengt. Man erhält 30,6 g 3-Iodpropyl-4,4,5,5,5-pentafluor-pentylsulfid.

**b) N-Methyl-[3-(4,4,5,5,5-pentafluor-pentylthio)-propyl]-amin**

In eine Lösung von 30,6 g 3-Iodpropyl-4,4,5,5,5-pentafluor-pentylsulfid in 200 ml abs. Tetrahydro-furan werden bei -78 °C Badtemperatur 45 g Methylamin kondensiert und 1,5 Stunden bei Raumtem-peratur sowie 4 Stunden bei 60 C im Druckreaktor gerührt. Zur Öffnung des Reaktors läßt man über Nacht auf Raumtemperatur und dann auf -78 C abkühlen. Dann läßt man auf Raumtemperatur kommen, überschüssiges Methylamin abdampfen, verdünnt mit Essigester, wäscht neutral, trocknet über Natriumsulfat, engt i.Vak. ein und chromatographiert an Kieselgel mit Dichlormethan / Methanol. Man erhält 15,7 g N-Methyl-[3-(4,4,5,5,5-pentafluor-pentylthio)-propyl]-amin als Öl.

**N-Methyl-[3-(4,4,5,5,5-pentafluor-pentansulfonyl)-propyl]-amin**

**a) 3-Chlorpropyl-4,4,5,5,5-pentafluor-pentansulfon**

Eine Lösung von 23 g 3-Chlorpropyl-4,4,5,5,5-pentafluor-pentylsulfid in 230 ml Chloroform wird bei 0 °C mit 41,8 g 70 %iger *m*-Chlorperbenzoesäure portionsweise versetzt und 1,5 Stunden bei Raumtemperatur gerührt. Dann wird mit Dichlormethan verdünnt, mit Natriumhydrogensulfit-, Natrium-hydrogencarbonat- und Kochsalzlösung gewaschen, über Natriumsulfat getrocknet und i.Vak. einge-engt. Man erhält 23,8 g reines 3-Chlorpropyl-4,4,5,5,5-pentafluor-pentansulfon als Kristalle vom Schmelzpunkt 74-76 C.

**b) 3-Iodpropyl-4,4,5,5,5-pentafluor-pentansulfon**

Eine Lösung von 23,5 g 3-Chlorpropyl-4,4,5,5,5-pentafluor-pentansulfon in 500 ml Ethylmetylketon wird mit 40 g Natriumiodid 5 Stunden bei 100 °C Badtemperatur unter Stickstoff gerührt. Dann wird i. Vak. zur Trockne eingeengt, auf Wasser gegeben, dreimal mit Essigester extrahiert, neutral gewaschen, und über Natriumsulfat getrocknet und i. Vak. eingeengt. Man erhält 30,6 g 3-Iodpropyl-4,4,5,5,5-pentafluor-pentansulfon als Kristalle vom Schmelzpunkt 88-89°C,

**c) N-Methyl-[3-(4,4,5,5,5-pentafluor-pentansulfonyl)-propyl]-amin**

Eine Lösung von 23,5 g 3-Iodpropyl-4,4,5,5,5-pentafluor-pentansulfon in 200 ml abs. Tetrahydrofuran werden bei -78°C Badtemperatur 44 g Methylamin kondesiert und 1,5 Stunden bei Raumtemperatur sowie 4 Stunden bei 60 °C im Druckreaktor gerührt. Zur Öffnung des Reaktors läßt man über Nacht auf Raumtemperatur und dann auf -78°C abkühlen. Dann läßt man auf Raumtemperatur kommen, überschüssiges Methylamin abdampfen, verdünnt mit Essigester, wäscht neutral, trocknet über Natriumsulfat, engt i.Vak. ein und chromatographiert an Kieselgel mit Dichlormethan / Methanol. Man erhält 14,8 g N-Methyl-[3-(4,4,5,5,5-pentafluor-pentansulfonyl)-propyl]-amin als Kristalle vom Schmelzpunkt 55-57°C.

**1-Brom-5-*tert*.-butyldimethylsilyloxypentan**

**a) 5-Brom-1-pentanol**

Zu einer Lösung von 50 g 5-Brompentylacetat in 1,6 1 Methanol werden 50 ml konzentrierte Schwefelsäure getropft und die Mischung 30 Stunden bei Raumtemperatur gerührt. Das Methanol wird im Vakuum abgezogen, der Rückstand in Diethylether aufgenommen, mit gesättigter Kochsalzlösung neutral gewaschen, über Natriumsulfat getrocknet und eingeengt. Es werden 28 g 5-Brom-1-pentanol als Rohprodukt erhalten.

**b) 1-Brom-5-*tert*.-butyldimethylsilyloxypentan**

Eine Lösung von 28 g des rohen 5-Brom-1-pentanols in 144 ml Tetrahydrofuran wird mit 24 g Imidazol versetzt. Anschließend wird eine Lösung von 30,3 g *tert*.-Butyldimethylchlorsilan in 46 ml Tetrahydrofuran zugetropft und 4 Stunden bei Raumtemperatur gerührt. Die Reaktionsmischung wird in Wasser gegossen, mit Diethylether ausgeschüttelt, die organische Phase 4 mal mit Wasser gewaschen, über Natriumsulfat getrocknet und eingeengt. Das Rohprodukt wird an Kieselgel mit Hexan /Diethylether chromatographiert. Es werden 42 g der Titelverbindung als farblose Flüssigkeit erhalten.

**Beispiel 2**

**11β-Fluor-7α-{5-[methyl-nonyl-amino]-pentyl}-estra-1,3,5(10)-trien-3,17β-diol**

**a) 7α-(5-Chlorpentyl)-11β-fluor-estr-4-en-3,17-dion**

Zu einer Suspension von 7,2 g Magnesiumspänen in 100 ml THF werden unter Stickstoff zunächst 20% einer Lösung aus 39 ml 1-Brom-5-chlorpentan in 300 ml THF zugegeben. Nach dem Anspringen der Reaktion, welches durch Zugabe von Iod und Dibrommethan erreicht werden kann, wird die restliche Lösung so zugetropft, daß die Innentemperatur 35 °C nicht überschreitet. In einem zweiten Kolben werden zu einer Suspension von 28,1 g Kupfer (I)iodid in 130 ml THF bei 0 °C 51,2 g Lithiumbromid zugegeben wobei die Innentemperatur auf 40 °C steigt. Ohne Kühlung werden nun 49,4 ml 1,3-Dimethyl-3,4,5,6-tetrahydro-(IH)-pyrimidin-2-on hinzugefügt und 15 Minuten bei 40 °C gerührt. Man erhält eine klare Lösung, die zu der auf -50 °C gekühlten Grignard-Lösung getropft wird. Anschließend wird 15 Minuten bei -30 °C nachgerührt und bei -70°C tropfenweise mit einer Lösung von 25 g 11β-Fluor-estra-4,6-dien-3,17-dion in 260 ml THF, 26 ml 1,3-Dimethyl-3,4,5,6-tetrahydro-(1H)-pyrimidin-2-on und 59 ml Trimethylchlorsilan so versetzt, daß die Innentemperatur -65 °C nicht überschreitet. Es wird 30 Minuten in der Kälte gerührt, dann 34,7 ml Eisessig zugetropft, das Kühlbad entfernt und 1 Stunde bei Raumtemperatur nachgerührt. Zur Aufarbeitung wird das Reaktionsgemisch auf 1,51 Wasser gegeben, mit der gleichen Menge Essigester verdünnt, der Niederschlag über Celite abgetrennt, mit Essigester nachgewaschen, die wässrige Phase 3 mal mit Essigester extrahiert, mit Natriumhydrogencarbonat- und Kochsalzlösung gewaschen, über Magnesiumsulfat getrocknet und i. Vak. eingeengt. Nach Chromatographieren des Rohproduktes an Kieselgel mit einem Hexan-Essigester-Gradienten werden 22,1 g 7α-(5-Chlorpentyl)-11β-fluor-estr-4-en-3,17-dion erhalten.

**b) 7α-(5-Chlorpentyl)-11β-fluor-3-hydroxy-estra-1,3,5(10)-trien-17-on**

Zu 22,1 g 7α-(5-Chlorpentyl)-11β-fluor-estr-4-en-3,17-dion in 160 ml wasserfreiem Acetonitril werden bei 80 °C 25,4 g Kupfer(II)bromid und 4,9 g Lithiumbromid in 95 ml wasserfreiem Acetonitril gegeben. Nach 20 Minuten wird das Reaktionsgemisch auf 0 °C gekühlt und 200 ml gesättigte Natriumhydrogencarbonat-Lösung zugetropft. Anschließend wird die Lösung in 750 ml Wasser eingerührt, mit 2N Salzsäure auf pH=6 gebracht, 3 mal mit Essigester extrahiert, mit Kochsalzlösung gewaschen, über Magnesiumsulfat getrocknet und i. Vak. eingeengt. Chromatographie des Rohproduktes an Kieselgel mit einem Hexan-Essigester-Gradienten liefert 14,7 g 7α-(5-Chlorpentyl)-11β-fluor-3-hydroxy-estra-1,3,5(10)-trien-17-on.

**c) 11 β-Fluor-3-hydroxy-7α-(5-iodpentyl)-estra-1,3,5(10)-trien-17-on**

5,0 g 7α-(5-Chlorpentyl)-11β-fluor-3-hydroxy-estra-1,3,5(10)-trien-17-on werden in 80 ml Ethylmethylketon gelöst, mit 5,7 g Natriumjodid versetzt und über Nacht bei 90 °C Badtemperatur gerührt. Zur Aufarbeitung wird das Reaktionsgemisch auf Raumtemperatur abgekühlt, in Wasser eingerührt, 3 mal mit Essigester extrahiert, mit Kochsalzlösung gewaschen, über Magnesiumsulfat getrocknet und i. Vak. eingeengt. Man erhält 6,8 g 11β-Fluor-3-hydroxy-7α-(5-iodpentyl)-estra-1,3,5(10)-trien-17-on als Rohprodukt, welches ohne weitere Aufreinigung in der nächsten Stufe eingesetzt wird.

**c) 11β-Floor-3-hydroxy-7α-[5-(methyl-amino)-pentyl]-estra-1,3,5(10)-trien-17-on**

In eine Lösung von 6,8 g 11β-Fluor-3-hydroxy-7α-(5-iodpentyl)-estra-1,3,5(10)-trien-17-on in 35 ml wasserfreiem Tetrahydrofuran werden bei -78 °C 5,1 g Methylamin kondensiert und über Nacht bei Raumtemperatur im Druckreaktor gerührt. Nachdem der Druckreaktor bei -20 °C geöffnet wurde, läßt man auf Raumtemperatur kommen, um überschüssiges Methylamin abdampfen zu lassen. Anschließend wir die Raktionslösung in gesättigte Natriumhydrogencarbonat-Lösung gegeben, 3 mal mit Essigester extrahiert, über Magnesiumsulfat getrocknet und i. Vak. eingeengt. Es werden 6,7 g 11β-Fluor-3-hydroxy-7α-[5-(methyl-amino)-pentyl]-estra-1,3,5(10)-trien-17-on als Rohprodukt erhalten.

**d) 11 β-Fluor-3-hydroxy-7α-{5-[methyl-nonyl-amino]-pentyl}-estra-1,3,5(10)-trien-17-on**

526 mg 11β-Fluor-3-hydroxy-7α-[5-(methyl-amino)-pentyl]-estra-1,3,5(10)-trien-17-on und 127 mg Iodnonan werden in 5 ml wasserfreiem DMF gelöst und 4h bei 100 °C Badtemperatur gerührt. Zur Aufarbeitung wird der Ansatz in halbgesättigte Natriumhydrogencarbonat-Lösung gegeben, 3 mal mit Methylenchlorid extrahiert, über Magnesiumsulfat getrocknet und i. Vak. eingeengt. Präparative Säulenchromatographie liefert 85 mg 11 β-Fluor-3-hydroxy-7α-{5-[methyl-nonyl-amino]-pentyl}-estra-1,3,5(10)-tries-17-on als Schaum.

**e) 11β-Fluor-7α-{5-[methyl-nonyl-amino]-pentyl}-estra-1,3,5(10)-trien-3,17β-diol**

85 mg 11 β-Fluor-3-hydroxy-7α-{5-[methyl-nonyl-amino]-pentyl}-estra-1,3,5(10)-trien-17-on werden in 3 ml Methanol gelöst und mit 25 mg Natriumborhydrid versetzt. Nach 30 Minuten Rühren bei Raumtemperatur wird das Lösungsmittel größtenteils im Vakuum abgezogen, der Rückstand mit Kochsalzlösung versetzt, 3 mal mit Methylenchlorid extrahiert, über Magnesiumsulfat getrocknet und i. Vak. eingeengt. Präparative Dünnschichtchromatographie mit Methylenchlorid/Methanol=9/1 + 0,5% Triethylamin als Laufmittel führt zu 33 mg 11β-Fluor-7α-{5-[methyl-nonyl-amino]-pentyl}-estra-1,3,5(10)-trien-3,17β-diol als Schaum.

**Beispiel 3**

**11β-Fluor-7α-{5-[methyl-(9,9,10,10,10-pentafluor-decyl)-amino]-pentyl}-estra-1,3,5(10)-trien-3,17β-diol**

**a) 11β-Fluor-3-hydroxy-7α-{5-[methyl-(9,9,10,10,10-pentafluor-decyl)-amino]-pentyl}estra-1,3,5(10)-trien-17-on**

Eine Lösung von 260 mg 11β-Fluor-3-hydroxy-7α-[5-(methyl-amino)-pentyl]-estra-1,3,5(10)-trien-17-on in 3 ml Dimethylformamid wird mit 180 mg 9,9,10,10,10-Pentafluordecyltosylat 1 Stunde bei 100°C Badtemperatur gerührt. Dann wird auf halbgesättigte Natriumhydrogencarbonat-Lösung gegeben, dreimal mit Methylenchlorid extrahiert, über Magnesiumsulfat getrocknet, i.Vak. eingeengt und an Kieselgel mit Dichlormethan / Methanol / Triethylamin chromatographiert. Man erhält 92 mg 11β-Fluor-3-hydroxy-7α-{5-[methyl-(9,9,10,10,10-pentafluor-decyl)-amino]-pentyl}-estra-1,3,5(10)-trien-17-on als Schaum, $[\alpha]_D$ = +48 ° (c=1,0 in Chloroform).

**b) 11β-Fluor-7α-{5-[methyl-(9,9,10,10,10-pentafluor-decyl)-amino]-pentyl}-estra-1,3,5(10)-trien-3,17β-diol**

92 mg 11β-Fluor-3-hydroxy-7α-{ 5-[methyl-(9,9,10,10,10-pentafluor-decyl)-amino]-pentyl}estra-1,3,5(10)-trien-17-on werden in 2 ml Methanol gelöst und mit 23 mg Natriumborhydrid versetzt. Nach 1 Stunde Rühren bei Raumtemperatur wird das Lösungsmittel größtenteils im Vakuum abgezogen, der Rückstand mit Kochsalzlösung versetzt,

3 mal mit Methylenchlorid extrahiert, über Magnesiumsulfat getrocknet und i. Vak. eingeengt. Präparative Dünnschichtchromatographie mit Methylenchlorid / Methanol=9/1 als Laufmittel führt zu 42 mg 11β-Fluor-7α-{5-[methyl-(9,9,10,10,10-pentafluor-decyl)-amino]-pentyl} -estra-1,3,5(10)-trien-3,17β-diol als Schaum, $[\alpha]_D$ = +44 ° (c=1,0 in Chloroform).

**Beispiel 4**

**11β-Fluor-7α-{6-[methyl-(8,8,9,9,9-pentafluor-nonyl)-amino]-hexyl}-estra-1,3,5(10)-trien-3,17β-diol**

**a) 7α-(6-Chlorhexyl)-11β-fluor-estr-4-en-3,17-dion**

Zu einer Suspension von 6,8 g Magnesiumspänen in 100 ml THF werden unter Stickstoff zunächst 30 ml einer Lösung aus 41 ml 1-Brom-6-chlor-hexan in 270 ml THF zugegeben. Nach dem Anspringen der Reaktion wird die restliche Lösung so zugetropft, daß die Innentemperatur 35 °C nicht überschreitet. In einem zweiten Kolben werden zu einer Suspension von 26,4 g Kupfer(I)iodid in 120 ml THF bei 0 °C 48,1 g Lithiumbromid zugegeben wobei die Innentemperatur auf 40 °C steigt. Ohne Kühlung werden nun 46,4 ml 1,3-Dimethyl-3,4,5,6-tetrahydro-(1H)-pyrimidin-2-on hinzugefügt und 30 Minuten bei 40 °C gerührt. Man erhält eine klare Lösung, die zu der auf -40 °C gekühlten Grignard-Lösung getropft wird. Anschließend wird 30 Minuten bei -30 °C nachgerührt und bei -50°C tropfenweise mit einer Lösung von 23,5 g 11 β-Fluor-estra-4,6-dien-3,17-dion in 230 ml THF, 24,6 ml 1,3-Dimethyl-3,4,5,6-tetrahydro-(1H)-pyrimidin-2-on und 55 ml Trimethylchlorsilan so versetzt, daß die Innentemperatur -40 °C nicht überschreitet. Es wird 1h in der Kälte gerührt, dann 32 ml Eisessig zugetropft, das Kühlbad entfernt und 1 Stunde bei Raumtemperatur nachgerührt. Zur Aufarbeitung wird das Reaktionsgemisch auf 1,51 Wasser gegeben, mit der gleichen Menge Essigester verdünnt, der Niederschlag über Celite abgetrennt, mit Essigester nachgewaschen, die wässrige Phase 3 mal mit Essigester extrahiert, mit Natriumhydrogencarbonat- und Kochsalzlösung gewaschen, über Magnesiumsulfat getrocknet und i. Vak. eingeengt. Nach Chromatographieren des Rohproduktes an Kieselgel mit einem Hexan-Essigester-Gradienten werden 25,2g 7α-(6-Chlorhexyl)-11β-fluor-estr-4-en-3,17-dion erhalten.

**b) 7α-(6-Chlorhexyl)-11β-fluor-3-hydroxy-estra-1,3,5(10)-trien-17-on**

Zu 25,2 g 7α-(6-Chlorhexyl)-11β-fluor-estr-4-en-3,17-dion in 175 ml wasserfreiem Acetonitril werden bei 80 °C 28,1 g Kupfer(II)bromid und 5,4 g Lithiumbromid in 105 ml wasserfreiem Acetonitril gegeben. Nach 15 Minuten wird das Reaktionsgemisch auf 0 °C gekühlt und 250 ml gesättigte Natriumhydrogencarbonat-Lösung zugetropft. Anschließend wird die Lösung in 1 Liter Wasser eingerührt, mit 2N Salzsäure auf pH=6 gebracht, 3 mal mit Essigester extrahiert, mit Kochsalzlösung gewaschen, über Magnesiumsulfat getrocknet und i. Vak. eingeengt. Chromatographie des Rohproduktes an Kieselgel mit einem Hexan-Essigester-Gradienten liefert 5,7 g 7α-(6-Chlorhexyl)-11β-fluor-3-hydroxy-estra-1,3,5(10)-trien-17-on als Schaum.

**c) 11β-Fluor-3-hydroxy-7α-(6-iodhexyl)-estra-1,3,5(10)-trien-17-on**

2,7 g 7α-(6-Chlorhexyl)-11β-fluor-3-hydroxy-estra-1,3,5(10)-trien-17-on werden in 40 ml Ethylmethylketon gelöst, mit 3,0 g Natriumjodid versetzt und über Nacht bei 90 °C Badtemperatur gerührt. Zur Aufarbeitung wird das Reaktionsgemisch auf Raumtemperatur abgekühlt, in Wasser eingerührt, 3 mal mit Essigester extrahiert, mit Kochsalzlösung gewaschen, über Magnesiumsulfat getrocknet und i. Vak. eingeengt. Man erhält 3.4 g 11β-Fluor-3-hydroxy-7α-(6-iodhexyl)-estra-1,3,5(10)-trien-17-on als Rohprodukt, welches ohne weitere Aufreinigung in der nächsten Stufe eingesetzt wird.

**d) 11β-Fluor-3-hydroxy-7α-[6-(methyl-amino)-hexyl]-estra-1,3,5(10)-trien-17-on**

In eine Lösung von 960 mg 11β-Fluor-3-hydroxy-7α-(6-iodhexyl)-estra-1,3,5(10)-trien-17-on in 9 ml wasserfreiem Tetrahydrofuran werden bei -78 °C 718 mg Methylamin kondensiert und über Nacht bei Raumtemperatur im Druckreaktor gerührt. Nachdem der Druckreaktor bei -20 °C geöffnet wurde, läßt man auf Raumtemperatur kommen, um überschüssiges Methylamin abdampfen zu lassen. Anschließend wir die Raktionslösung in gesättigte Natriumhydrogencarbonat-Lösung gegeben, 3 mal mit Essigester extrahiert, über Natriumsulfat getrocknet und i. Vak. eingeengt. Es werden 763 mg 11β-Fluor-3-hydroxy-7α-[5-(methyl-amino)-hexyl]-estra-1,3,5(10)-trien-17-on als Rohprodukt erhalten.

**e) 11β-Fluor-3-hydroxy-7α-{6-[methyl-(8,8,9,9,9-pentafluor-nonyl)-amino]-hexyl}-estra-1,3,5(10)-trien-17-on**

Eine Lösung von 381 mg 11β-Fluor-3-hydroxy-7α-[6-(methyl-amino)-hexyl]-estra-1,3,5(10)-trien-17-on in 5 ml Dimethylformamid wird mit 200 mg 8,8,9,9,9-Pentafluomonyltosylat 2 Stunden bei 100°C Badtemperatur gerührt. Dann wird auf halbgesättigte Natriumhydrogencarbonatlösung gegeben, dreimal mit Methylenchlorid extrahiert, über Magnesiumsulfat getrocknet, i.Vak. eingeengt und an Kieselgel mit Dichlormethan /Methanol chromatographiert. Man erhält 90 mg 11β-Fluor-3-hydroxy-7α-{6-[methyl-(8,8,9,9,9-pentafluor-nonyl)-amino]-hexyl}-estra-1,3,5(10)-trien-17-on als als Schaum.

**f) 11β-Fluor-7α-{6-[methyl-(8,8,9,9,9-pentafluor-nonyl)-amino]-hexyl}-estra-1,3,5(10)-trien-3,17β-diol**

89 mg 11β-Fluor-3-hydroxy-7α- {6-[methyl-(8,8,9,9,9-pentafluor-nonyl)-amino]-hexyl}estra-1,3,5(10)-trien-17-on werden in 2 ml Methanol gelöst und mit 22 mg Natriumborhydrid versetzt. Nach 1 Stunde Rühren bei Raumtemperatur wird das Lösungsmittel größtenteils im Vakuum abgezogen, der Rückstand mit Kochsalzlösung versetzt, 3 mal mit Methylenchlorid extrahiert, über Magnesiumsulfat getrocknet und i. Vak. eingeengt. Präparative Dünnschichtchromatographie mit Methylenchlorid / Methanol=9/1 als Laufmittel führt zu 53 mg 11β-Fluor-7α-{5-[methyl-(8,8,9,9,9-pentafluor-nonyl)-amino]-hexyl}-estra-1,3,5(10)-trien-3,17β-diol als Schaum, $[\alpha]_D$ = +32 ° (c=1,0 in Chloroform).

**Beispiel 5**

**11 β-Fluor-7α-{6-[methyl-(9,9,10,10,10-pentafluor-decyl)-amino]-hexyl}-estra-1,3,5(10)-trien-3,17β-diol**

**a) 11β-Fluor-3-hydroxy-7α-{6-[methyl-(9,9,10,10,10-pentafluor-decyl)-amino]-hexyl}estra-1,3,5(10)-trien-17-on**

Eine Lösung von 381 mg 11β-Fluor-3-hydroxy-7α-[6-(methyl-amino)-hexyl]-estra-1,3,5(10)-trien-17-on in 5 ml Dimethylformamid wird mit 180 mg 9,9,10,10,10-Pentafluor-decyltosylat 2 Stunden bei 100°C Badtemperatur gerührt. Dann wird auf halbgesättigte Natriumhydrogencarbonat-Lösung gegeben, dreimal mit Methylenchlorid extrahiert, über Magnesiumsulfat getrocknet, i.Vak. eingeengt und an Kieselgel mit Dichlormethan /Methanol / Triethylamin chromatographiert. Man erhält 121 mg 11β-Fluor-3-hydroxy-7α-{6-[methyl-(9,9,10,10,10-pentafluor-decyl)-amino]-hexyl}-estra-1,3,5(10)-trien-17-on als Schaum, $[\alpha]_D$ = +59 ° (c=1,0 in Chloroform).

**b) 11β-Fluor-7α-{6-[methyl-(9,9,10,10,10-pentanuor-decyl)-amino]-hexyl}-estra-1,3,5(10)-trien-3,17β-diol**

120 mg 11β-Fluor-3-hydroxy-7α-{6-[methyl-(9,9,10,10,10-pentafluor-decyl)-amino]-hexyl}estra-1,3,5(10)-trien-17-on werden in 2,5 ml Methanol gelöst und mit 29 mg Natriumborhydrid portionsweise versetzt. Nach 30 Minuten Rühren bei Raumtemperatur wird das Lösungsmittel größtenteils im Vakuum abgezogen, der Rückstand mit Wasser versetzt, 3 mal mit Methylenchlorid extrahiert, über Magnesiumsulfat getrocknet und i. Vak. eingeengt. Man erhält 106 mg 11β-Fluor-7α-{5-[methyl-(9,9,10,10,10-pentafluor-decyl}-amino]-hexyl}estra-1,3,5(10)-trien-3,17-diol als Schaum, $[\alpha]_D$ = +38 ° (c=0,5 in Chloroform).

**Beispiel 6**

**11β-Fluor-7α-(5-(methyl-amino)-pentyl)-estra-1,3,5(10)-brien-3,17β-diol 11β-Fluor-7α-(5-(methyl-amino)-pentyl)-estra-1,3,5(10)-trien-3,17β-diol**

880 mg 11β-Fluor-3-hydroxy-7α-(5-(methyl-amino)-pentyl)-estra-1,3,5(10)-trien-17-on werden in 15 ml Methanol gelöst, portionsweise mit 252 mg Natriumborhydrid versetzt und 15 min bei Raumtemperatur gerührt. Zur Aufarbeitung wird die Raektionslösung in gesättigte Kochsalzlösung gegeben, mehrmals mit Methylenchlorid extrahiert, die organischen Phasen mit Wasser gewaschen, über Magnesiumsulfat getrocknet und i. Vak. eingeengt. Man erhält 540 mg 11β-Fluor-7α-(5-(methyl-amino)-pentyl)-estra-1,3,5(10)-trien-3,17β-diol als Schaum, $[\alpha]_D$ = +63 ° (c=0,5 in Chloroform).

**Beispiel 7**

**11β-Fluor-7α-(5-pyrrolidin-1-yl-pentyl)-estra-1,3,5(10)-trien-3,17β-diol**

**a) 11β-Fluor-3-hydroxy-7α-(5-pyrrolidin-1-yl-penty)-estra-1,3,5(10)-trien-17-on**

Eine Lösung von 1,0 g 11β-Fluor-3-hydroxy-7α-(5-iodpentyl)-estra-1,3,5(10)-trien-17-on in 10 ml Dimethylformamid wird mit 0,24 ml Pyrrolidin 2 Stunden bei 100 °C Badtemperatur gerührt. Dann wird auf gesättigte Natriumhydrogencarbonat-Lösung gegeben, dreimal mit Methylenchlorid ex-trahiert, über Magnesiumsulfat getrocknet, i.Vak. eingeengt und an Kieselgel mit Dichlormethan / Methanol / Triethylamin chromatographiert. Man erhält 367 mg 11β-Fluor-3-hydroxy-7α-(5-pyrrolidin-1-yl-pentyl)-estra-1,3,5(10)-trien-17-on als Schaum.

**b) 11β-Fluor-7α-(5-pyrrolidin-1-yl-pentyl)-estra-1,3,5(10)-trien-3,17β-diol**

324 mg 11β-Fluor-3-hydroxy-7α-(5-pyrrolidin-1-yl-pentyl)-estra-1,3,5(10)-trien-17-on werden in 10 ml Methanol gelöst und mit 63 mg Natriumborhydrid versetzt. Nach 1,5 Stunden Rühren bei Raum-temperatur wird der Ansatz in halbgesättigte Kochsalzlösung gegeben, 3 mal mit Methylenchlorid extrahiert, über Magnesiumsulfat getrocknet und i. Vak. eingeengt. Nach Chromatographieren des Rohproduktes an Kieselgel mit einem Methylenchlorid-Methanol-Gradienten werden 192 mg 11β-Fluor-7α-(5-pyrrolidin-1-yl-pentyl)-estra-1,3,5(10)-trien-3,17ß-diol vom Schmp. 95-118 °C erhalten.

**Beispiel 8**

**11β-Fluor-7α-{5-[methyl-(4,4,5,5,5-pentafluor-pentyl)-amino]-pentyl}-estra-1,3,5(10)-trien-3,17β-diol**

**a) 11β-Fluor-3-hydroxy-7α-{5-[methyl-(4,4,5,5,5-pentafluor-pentyl)-amino]-pentyl}estra-1,3,5(10)-trien-17-on**

500 mg 11β-Fluor-3-hydroxy-7α-(5-(methyl-amino)-pentyl)-estra-1,3,5(10)-trien-17-on werden in 15 ml Ethylmethylketon gelöst, mit 1,1 g Kaliumcarbonat und 1,5 ml 1,1,1,2,2-Pentafluor-5-iod-pentan versetzt und 2,5 Stunden unter Rückfluß erhitzt. Zur Aufarbeitung wird das Reaktionsgemisch auf Raumtemperatur abgekühlt, in gesättigte Kochsalzlösung eingerührt, mit Essigester extrahiert, über Magnesiumsulfat getrocknet und i. Vak. eingeengt. Präparative Säulenchromatographie an Kieselgel mit einem Methylenchlorid-Methanol-Gradienten führt zu 397 mg 11β-Fluor-3-hydroxy-7α-{5-[methyl-(4,4,5,5,5-pentafluorpentyl)-amino]-pentyl} -estra-1,3,5(10)-trien-17-on.

**b) 11β-Fluor-7α-{5-[methyl-(4,4,5,5,5-pentafluor-pentyl)-amino]-pentyl}-estra-1,3,5(10)-trien-3,17β-diol**

380 mg 11β-Fluor-3-hydroxy-7α-{5-[methyl-(4,4,5,5,5-pentafluor-pentyl)-amino]-pentyl}estra-1,3,5(10)-trien-17-on werden in 25 ml Methanol gelöst und mit 77 mg Natriumborhydrid versetzt. Nach 30 Minuten Rühren bei Raumtemperatur wird der Ansatz in gesättigte Kochsalzlösung gegeben, mit Methylenchlorid extrahiert, über Magnesiumsulfat getrocknet und i. Vak. eingeengt. Nach Chromatographieren des Rohproduktes an Kieselgel mit einem Methylenchlorid-Methanol-Gradienten werden 317 mg 11β-Fluor-7α-{5-[methyl-(4,4,5,5,5-pentafluor-pentyl)-amino]-pentyl}-estra-1,3,5(10)-trien-3,17β-diol als Schaum erhalten, $[\alpha]_D$ = +49 ° (c=0,5 in Methanol).

**Beispiel 9**

**11β-Fluor-7α-{5-[methyl-(4,4,5,5,6,6,7,7,8,8,9,9,9-trdecafluor-nonyl)-amino]-pentyl}-estra-1,3,5(10)-trien-3,17β-diol**

Eine Lösung von 466 mg 7α-(5-Brompentyl)-11β-fluor-estra-1,3,5(10)-trien-3,17-diol in 10 ml 1-Methyl-2-pyrrolidinon wird mit 1,17 g Methyl-(4,4,5,5,6,6,7,7,8,8,9,9,9-tridecaftuornonyl)-amin 3 Stunden bei 80°C Badtemperatur gerührt. Zur Aufarbeitung wird der Ansatz auf gesättigte Natriumchloridlösung gegeben, mit Essigester extrahiert, über Natriumsulfat getrocknet, i. Vak. eingeengt und an Kieselgel mit Dichlormethan / Methanol chromatographiert. Man erhält 625 mg 11β-Fluor-7α-{5-[methyl-(4,4,5,5,6,6,7,7,8,8,9,9,9-tridecafluor-nonyl)-amino]-pentyl}-estra-1,3,5(10)-trien-3,17β-diol als Schaum, $[\alpha]_D$ = +35 ° (c=0,5 in $CHCl_3$).

**Beispiel 10**

**11 β-Fluor-7α-{5-[(4,4,5,5,6,6,7,7,8,8,9,9,10,10,11,11,11-heptadecafluor-undecyl)-methyl-amino]-pentyl}-estra-1,3,5(10)-trien-3,17β-diol**

Eine Lösung von 466 mg 7α-(5-Brompentyl)-11β-fluor-estra-1,3,5(10)-trien-3,17-diol in 10 ml 1-Methyl-2-pyrrolidinon wird mit 1,47 g (4,4,5,5,6,6,7,7,8,8,9,9,10,10,11,11,11-Heptadecafluor-undecyl)-methyl-amin 3 Stunden bei 80°C Badtemperatur gerührt. Zur Aufarbeitung wird der Ansatz auf gesättigte Natriumchloridlösung gegeben, mit Ether extrahiert, über Natriumsulfat getrocknet, i. Vak. eingeengt und an Kieselgel mit Essigester /Methanol chromatographiert. Man erhält 524 mg 11β-Fluor-7α-{5-[(4,4,5,5,6,6,7,7,8,8,9,9,10,10,11,11,11-heptadecafluor-undecyl)-methyl-amino]-pentyl}estra-1,3,5(10)-trien-3,17β-diol als Schaum, $[\alpha]_D$ = +24 ° (c=0,5 in $CHCl_3$).

**Beispiel 11**

**11β-Fluor-7α-{5-[methyl-(3,3,4,4,5,5,6,6,6-nonafluor-hexyl)-amino]-pentyl}-estra-1,3,5(10)-trien-3,17β-diol**

**a) 11β-Fluor-3-hydroxy-7α-{5-[methyl-(3,3,4,4,5,5,6,6,6-nonafluor-hexyl)-amino]-pentyl}-estra-1,3,5(10)-trien-17-on**

276 mg 11β-Fluor-3-hydroxy-7α-(5-(methyl-amino)-pentyl)-estra-1,3,5(10)-trien-17-on werden in 3 ml N-Methylpyrrolidon gelöst, mit 0,6 ml 1,1,1,2,2,3,3,4,4-Nonafluor-5-iodhexan versetzt und 1,5 Stunden bei 80 °C Badtemperatur erhitzt. Zur Aufarbeitung wird das Reaktionsgemisch auf Raumtemperatur abgekühlt, in gesättigte Kochsalzlösung eingerührt, mit Essigester extrahiert, die organischen Phasen mit Wasser gewaschen, über Magnesiumsulfat getrocknet und i. Vak. eingeengt. Präparative Säulenchromatographie an Kieselgel mit einem Methylenchlorid-Methanol-Gradienten führt zu 176 mg 11β-Fluor-3-hydroxy-7α-{5-[methyl-(3,3,4,4,5,5,6,6,6-nonafluor-hexyl)-amino]-pentyl}-estra-1,3,5(10)-trien-17-on.

**b) 11β-Fluor-7α-{5-[methyl-(3,3,4,4,5,5,6,6,6-nonafluor-hexyl)-amino]-pentyl}-estra-1,3,5(10)-trien-3,17β-diol**

160 mg 11β-Fluor-3-hydroxy-7α-{5-[methyl-(3,3,4,4,5,5,6,6,6-nonafluor-hexyl)-amino]-pentyl}-estra-1,3,5(10)-trien-17-on werden in 5 ml Methanol gelöst und mit 28 mg Natriumborhydrid versetzt. Nach 30 Minuten Rühren bei Raumtemperatur wird der Ansatz in gesättigte Kochsalzlösung gegeben, mit Methylenchlorid extrahiert, über Magnesiumsulfat getrocknet und i. Vak. eingeengt. Nach Chromatographieren des Rohproduktes an Kieselgel mit einem Methylenchlorid-MTBE- und einem Essigester-Aceton-Gradienten werden 105 mg 11β-Fluor-7α-{5-[methyl-(3,3,4,4,5,5,6,6,6-nonafluor-hexyl)-amino]-pentyl}-estra-1,3,5(10)-trien-3,17β-diol als Schaum erhalten, $[\alpha]_D$ = +43 ° (c=0,5 in $CHCl_3$).

**Beispiel 12**

**11β-Fluor-7α-{5-[methyl-(7,7,8,8,8-pentafluor-octyl)-amino]-pentyl}-estra-1,3,5(10)-trien-3,17β-diol**

Eine Lösung von 466 mg 7α-(5-Brompentyl)-11β-fluor-estra-1,3,5(10)-trien-3,17-diol in 10 ml 1-Methyl-2-pyrrolidinon wird mit 702 mg Methyl-(7,7,8,8,8-Pentafluor-octyl)-amin 3 Stunden bei 80°C Badtemperatur gerührt. Zur Aufarbeitung wird der Ansatz auf gesättigte Natriumchloridlösung gegeben, mit Essigester extrahiert, über Natriumsulfat getrocknet, i. Vak. eingeengt und an Kieselgel mit Dichlormethan / Methanol chromatographiert. Man erhält 595 mg 11β-Fluor-7α-{5-[methyl-(7,7,8,8,8-pentafluor-octyl)-amino]-pentyl}-estra-1,3,5(10)-trien-3,17β-diol als Schaum, $[\alpha]_D$ = +39 ° (c=0,5 in $CHCl_3$).

**Beispiel 13**

**11β-Fluor-7α-{6-[methyl-(7,7,8,8,8-pentafluor-octyl)-amino]-hexyl}-estra-1,3,5(10)-trien-3,17β-diol**

**a) 7α-(6-Chlorhexyl)-11β-fluor-estra-1,3,5(10)-trien-3,17-diol**

611 mg 7α-(6-Chlorhexyl)-11β-fluor-3-hydroxy-estra-1,3,5(10)-trien-17-on werden in 10 ml Methanol gelöst und mit 166 mg Natriumborhydrid vorsichtig versetzt. Nach 15 Minuten Rühren bei Raumtemperatur wird der Ansatz in gesättigte Kochsalzlösung gegeben, mit Methylenchlorid extrahiert, über Magnesiumsulfat getrocknet und i. Vak. eingeengt. Man erhält 599 mg 7α-(6-Chlorhexyl)-11β-fluor-estra-1,3,5(10)-trien-3,17-diol als Rohprodukt, welches

ohne weitere Aufreinigung in der nächsten Stufe eingesetzt wird.

**b) 11 β-Fluor-7α-(6-iodhexyl)-estra-1,3,5(10)-trien-3,17-diol**

599 mg 7α-(6-Chlorhexyl)-11β-fluor-estra-1,3,5(10)-trien-3,17-diol werden in 10 ml Ethylmethylketon gelöst, mit 675 mg Natriumjodid versetzt und über Nacht bei 90 °C Badtemperatur gerührt. Zur Aufarbeitung wird das Reaktionsgemisch auf Raumtemperatur abgekühlt, in Essigester aufgenommen, 1 mal mit 10%iger Natriumthiosulfatlösung extrahiert, mit Kochsalzlösung gewaschen, über Magnesiumsulfat getrocknet und i. Vak. eingeengt. Man erhält 691 mg 11β-Fluor-7α-(6-iodhexyl)-estra-1,3,5(10)-trien-3,17-diol als Rohprodukt, welches ohne weitere Aufreinigung in der nächsten Stufe eingesetzt wird.

**c) 11β-Fluor-7α-{6-[methyl-(7,7,8,8,8-pentafluor-octyl)-amino]-hexyl}-estra-1,3,5(10)-trien-3,17β-diol**

Eine Lösung von 690 mg 11β-Fluor-7α-(6-iodhexyl)-estra-1,3,5(10)-trien-3,17-diol in 15 ml 1-Methyl-2-pyrrolidinon wird mit 810 mg Methyl-(7,7,8,8,8-Pentafluor-octyl)-amin 3 Stunden bei 80°C Badtemperatur gerührt. Zur Aufarbeitung wird der Ansatz auf gesättigte Natriumchloridlösung gegeben, mit Ether extrahiert, über Natriumsulfat getrocknet, i. Vak. eingeengt und an Kieselgel mit Dichlormethan / Methanol / Ammoniak (25%ig) chromatographiert. Man erhält 576 mg 11β-Fluor-7α-{6-[methyl-(7,7,8,8,8-pentafluor-octyl)-amino]-hexyl}-estra-1,3,5(10)-trien-3,17β-diol als Schaum, $[\alpha]_D$ = +42 ° (c=0,5 in $CHCl_3$).

**Beispiel 14**

**11β-Fluor-7α-{5-[methyl-(7,7,8,8,9,9,10,10,10-nonafluor-decyl)-amino]-pentyl}-estra-1,3,5(10)-trien-3,17β-diol**

Eine Lösung von 466 mg 7α-(5-Brompentyl)-11β-fluor-estra-1,3,5(10)-trien-3,17-diol in 10 ml 1-Methyl-2-pyrrolidinon wird mit 1,0 g Methyl-(7,7,8,8,9,9,10,10,10-nonafluor-decyl)-amin 3 Stunden bei 80°C Badtemperatur gerührt. Zur Aufarbeitung wird der Ansatz auf gesättigte Natriumchlorid-lösung gegeben, mit Ether extrahiert, über Natriumsulfat getrocknet, i. Vak, eingeengt und an Kieselgel mit Essigester / Methanol chromatographiert. Man erhält 546 mg 11β-Fluor-7α-{5-[methyl-(7,7,8,8,9,9,10,10,10-nonafluor-decyl)-amino]-pentyl}-estra-1,3,5(10)-trien-3,17ß-diol als Schaum, $[\alpha]_D$ = +39 ° (c=0,5 in $CHCl_3$).

**Beispiel 15**

**11β-Fluor-7α-{5-[methyl-(7,7,8,8,9,9,10,10,11,11,12,12,12-tridecafluor-dodecyl)-amino]-pentyl}-estra-1,3,5(10)-trien-3,17β-diol**

Eine Lösung von 466 mg 7α-(5-Brompentyl)-11β-fluor-estra-1,3,5(10)-trien-3,17-diol in 10 ml 1-Methyl-2-pyrrolidinon wird mit 1,3 g Methyl-(7,7,8,8,9,9,10,10,11,11,12,12,12-tridecafluor-dodecyl)-amin 3 Stunden bei 80°C Badtemperatur gerührt. Zur Aufarbeitung wird der Ansatz auf gesättigte Natriumchloridlösung gegeben, mit Ether extrahiert, über Natriumsulfat getrocknet, i. Vak. eingeengt und an Kieselgel mit Essigester / Methanol chromatographiert. Man erhält 628 mg 11β-Fluor-7α-{5-[methyl-(7,7,8,8,9,9,10,10,11,11,12,12,12-tridecafluor-dodecyl)-amino]-pentyl}-estra-1,3,5(10)-trien-3,17β-diol als Schaum, $[\alpha]_D$ = +24 ° (c=0,5 in $CHCl_3$).

**Beispiel 16**

**11 β-Fluor-7α-{5-[(7,7,8,8,9,9,10,10,11,11,12,12,13,13,14,14,14-heptadecafluortetradecyl)-methyl-amino]-pentyl}-estra-1,3,5(10)-trien-3,17β-diol**

Eine Lösung von 431 mg 7α-(5-Brompentyl)-11β-fluor-estra-1,3,5(10)-trien-3,17-diol in 10 ml 1-Methyl-2-pyrrolidinon wird mit 1,6 g (7,7,8,8,9,9,10,10,11,11,12,12,13,13,14,14,14-Heptadecafluor-tetradecyl)-methyl-amin 3 Stunden bei 80°C Badtemperatur gerührt. Zur Aufarbeitung wird der Ansatz auf gesättigte Natriumchloridlösung gegeben, mit Ether extrahiert, über Natriumsulfat getrocknet, i. Vak. eingeengt und an Kieselgel mit Essigester /Methanol chromatographiert. Man erhält 318 mg 11β-Fluor-7α-{5-[(7,7,8,8,9,9,10,10,11,11,12,12,13,13,14,14,14-heptadecylfluor-tetradecyl)-methyl-amino]-pentyl}-estra-1,3,5(10)-trien-3,17β-diol als Schaum, $[\alpha]_D$ = +23 ° (c=0,5 in $CHCl_3$).

**Beispiel 17**

**11β-Fluor-7α-{5-[(3,4,4,5,5,5-hexafluor-pent-2-enyl)-methyl-amino]-pentyl}-estra-1,3,5(10)-trien-3,17β-diol**

**a) 11β-Fluor-7α-{5-[(3,4,4,5,5,5-hexafluor-pent-2-enyl)-methyl-amino]-pentyl}-3-hydroxy-estra-1,3,5(10)-trien-17-on**

880 mg 11β-Fluor-3-hydroxy-7α-(5-iodpentyl)-estra-1,3,5(10)-trien-17-on und 1,26 g (3,4,4,5,5,5-Hexafluor-pent-2-enyl)-methyl-amin werden in 20 ml N-Methylpyrrolidon gelöst und 3 Stunden bei 80 °C Badtemperatur gerührt. Nachdem die Reaktionslösung auf Raumtemperatur abgekühlt ist, wird der Ansatz auf gesättigte Kochsalzlösung gegeben, mit Diethylether extrahiert, getrocknet und i. Vak. eingeengt. Man erhält 1,86 g 11β-Fluor-7α-{5-[(3,4,4,5,5,5-hexafluor-pent-2-enyl)-methyl-amino]-pentyl}-3-hydroxy-estra-1,3,5(10)-trien-17-on als Rohprodukt, welches ohne Aufreinigung in der nächsten Stufe eingesetzt wird.

**b) 11β-Fluor-7α-{5-[(3,4,4,5,5,5-hexafluor-pent-2-enyl)-methyl-amino]-pentyl}-estra-1,3,5(10)-trien-3,17β-diol**

1,86 g 11β-Fluor-7α-{5-[(3,4,4,5,5,5-hexafluor-pent-2-enyl)-methyl-amino]-pentyl}-3-hydroxy-estra-1,3,5(10)-trien-17-on werden in 15 ml Methanol gelöst und mit 222 mg Natriumborhydrid vorsichtig versetzt. Nach 15 Minuten Rühren bei Raumtemperatur wird der Ansatz in gesättigte Kochsalzlösung gegeben, mit Methylenchlorid extrahiert, über Magnesiumsulfat getrocknet und i. Vak. eingeengt. Nach Chromatographieren des Rohproduktes an Kieselgel mit einem Hexan-Essigester- und einem Essigester-Aceton-Gradienten werden 241 mg 11β-Fluor-7α-{5-[(3,4,4,5,5,5-hexafluor-pent-2-enyl)-methylamino]-pentyl}-estra-1,3,5(10)-trien-3,17β-diol vom Schmelzpunkt 122 °C erhalten, $[\alpha]_D$ = +47 ° (c=0,5 in $CHCl_3$)

**Beispiel 18**

**11β-Fluor-7α-{5-[methyl-(3,4,4,5,5,6,6,6-octafluor-hex-2-enyl)-amino]-pentyl}-estra-1,3,5(10)-trien-3,17β-diol**

**a) 11 β-Fluor-3-hydroxy-7α-{5-[methyl-(3,4,4,5,5,6,6,6-octafluor-hex-2-enyl)-amino]-pentyl}-estra-1,3,5(10)-trien-17-on**

880 mg 11β-Fluor-3-hydroxy-7α-(5-iodpentyl)-estra-1,3,5(10)-trien-17-on und 2,21 g Methyl-(3,4,4,5,5,6,6,6-octafluor-hex-2-enyl)-amin werden in 20 ml N-Methylpyrrolidon gelöst und 1 Stunde bei 80 °C Badtemperatur gerührt. Nachdem die Reaktionslösung auf Raumtemperatur abgekühlt ist, wird der Ansatz auf gesättigte Kochsalzlösung gegeben, mit Diethylether extrahiert, getrocknet und i. Vak. eingeengt. Man erhält 1,69 g 11β-Fluor-3-hydroxy-7α-{ 5-[methyl-(3,4,4,5,5,6,6,6-octafluor-hex-2-enyl)-amino]-pentyl}-estra-1,3,5(10)-trien-17-on als Rohprodukt, welches ohne Aufreinigung in der nächsten Stufe eingesetzt wird.

**b) 11β-Fluor-7α-{5-[methyl-(3,4,4,5,5,6,6,6-octafluor-hex-2-enyl)-amino]-pentyl}-estra-1,3,5(10)-trien-3,17β-diol**

1,7 g 11β-Fluor-3-hydroxy-7α- {5-[methyl-(3,4,4,5,5,6,6,6-octafluor-hex-2-enyl)-amino]-pentyl}-estra-1,3,5(10)-trien-17-on werden in 15 ml Methanol gelöst und mit 222 mg Natriumborhydrid vorsichtig versetzt. Nach 15 Minuten Rühren bei Raumtemperatur wird der Ansatz in gesättigte Kochsalzlösung gegeben, mit Methylenchlorid extrahiert, über Magnesiumsulfat getrocknet und i. Vak. eingeengt. Nach Chromatographieren des Rohproduktes an Kieselgel mit Essigester als Eluenten werden 84 mg 11β-Fluor-7α-{5-[methyl-{3,4,4,5,5,6,6,6-octafluor-hex-2-enyl)-amino]-pentyl)-estra-1,3,5(10)-trien-3,17β-diol vom Schmelzpunkt 130 °C erhalten.

**Beispiel 19**

**7α-{5-[(3,4,4,5,5,6,6,7,7,8,8,8-Dodecafluor-oct-2-enyl)-methyl-amino]-pentyl}-11β-fluorestra-1,3,5(10)-trien-3,17β-diol**

**a) 7α-{5-[(3,4,4,5,5,6,6,7,7,8,8,8-Dodecafluor-oct-2-enyl)-methyl-amino)-pentyl}-11β-fluor-3-hydroxy-estra-1,3,5(10)-trien-17-on**

880 mg 11β-Fluor-3-hydroxy-7α-(5-iodpentyl)-estra-1,3,5(10)-trien-17-on und 2,23 g (3,4,4,5,5,6,6,7,7,8,8,8-Dodecafluor-oct-2-enyl)-methyl-amin werden in 20 ml N-Methylpyrrolidon gelöst und 1 Stunde bei 80 °C Badtemperatur gerührt. Nachdem die Reaktionslösung auf Raumtemperatur abgekühlt ist, wird der Ansatz

auf gesättigte Kochsalzlösung gegeben, mit Diethylether extrahiert, getrocknet und i. Vak. eingeengt. Man erhält 2,47 g 7α-{5-[(3,4,4,5,5,6,6,7,7,8,8,8-Dodecafluor-oct-2-enyl)-methyl-amino]-pentyl}-11β-fluor-3-hydroxy-estra-1,3,5(10)-trien-17-on als Rohprodukt, welches ohne Aufreinigung in der nächsten Stufe eingestzt wird.

**b) 7α-{5-[(3,4,4,5,5,6,6,7,7,8,8,8-Dodecafluor-oct-2-enyl)-methyl-amino]-pentyl}-11β-fluor-estra-1,3,5(10)-trien-3,17β-diol**

2,4 g 7α-{5-[(3,4,4,5,5,6,6,7,7,8,8,8-Dodecafluor-oct-2-enyl}-methyl-ammo]-pentyl}-11β-fluor-3-hydroxy-estra-1,3,5(10)-trien-17-on werden in 15 ml Methanol gelöst und mit 222 mg Natriumborhydrid vorsichtig versetzt. Nach 15 Minuten Rühren bei Raumtemperatur wird der Ansatz in gesättigte Kochsalzlösung gegeben, mit Methylenchlorid extrahiert, über Magnesiumsulfat getrocknet und i. Vak. eingeengt. Nach Chromatographieren des Rohproduktes an Kieselgel mit Essigester als Eluenten werden 319 mg 7α-{5-[(3,4,4,5,5,6,6,7,7,8,8,8-Dodecafluor-oct-2-enyl)-methyl-amino]-pentyl}-11β-fluor-estra-1,3,5(10)-trien-3,17β-diol als Schaum erhalten, $[\alpha]_D$ = +37 ° (c=0,5 in $CHCl_3$).

**Beispiel 20**

**11β-Fluor-7α-{5-[(3,4,4,5,5,6,6,7,7,8,8,9,9,10,10,10-hexadecafluor-dec-2-enyl)-methylamino]-pentyl}-estra-1,3,5(10)-trien-3,17β-diol**

**a) 11β-Fluor-7α-{5-[(3,4,4,5,5,6,6,7,7,8,8,9,9,10,10,10-hexadecafluor-dec-2-enyl)-methylamino]-pentyl}-3-hydroxy-estra-1,3,5(10)-trien-17-on**

880 mg 11β-Fluor-3-hydroxy-7α-(5-iodpentyl)-estra-1,3,5(10)-trien-17-on und 1,83 g (3,4,4,5,5,6,6,7,7,8,8,9,9,10,10,10-Hexadecafluor-dec-2-enyl)-methyl-amin werden in 20 ml N-Methylpyrrolidon gelöst und 1 Stunde bei 80 °C Badtemperatur gerührt. Nachdem die Reaktionslösung auf Raumtemperatur abgekühlt ist, wird der Ansatz auf gesättigte Kochsalzlösung gegeben, mit Diethylether extrahiert, getrocknet und i. Vak. eingeengt. Nach Chromatographieren des Rohproduktes an Kieselgel mit Essigester als Eluenten werden 1,2 g 11β-Fluor-7α-{5-[(3,4,4,5,5,6,6,7,7,8,8,9,9,10,10,10-hexadecafluor-dec-2-enyl)-methylamino]-pentyl}-3-hydroxy-estra-1,3,5(10)-trien-17-on erhalten.

**b) 11β-Fluor-7α-{5-[(3,4,4,5,5,6,6,7,7,8,8,9,9,10,10,10-hexadecafluor-dec-2-enyl)-methylamino]-pentyl}-estra-1,3,5(10)-trien-3,17β-diol**

1,1 g 11β-Fluor-7α-{5-[(3,4,4,5,5,6,6,7,7,8,8,9,9,10,10,10-hexadecafluor-dec-2-enyl)-methylamino]-pentyl}-3-hydroxy-estra-1,3,5(10)-trien-17-on werden in 10 ml Methanol gelöst und mit 148 mg Natriumborhydrid vorsichtig versetzt. Nach 15 Minuten Rühren bei Raumtemperatur wird der Ansatz in gesättigte Kochsalzlösung gegeben, mit Methylenchlorid extrahiert, über Magnesiumsulfat getrocknet und i. Vak. eingeengt. Nach Chromatographieren des Rohproduktes an Kieselgel mit einem Hexan-Essigester- und einem Essigester-Aceton-Gradienten werden 127 mg 11β-Fluor-7α-{5-[(3,4,4,5,5,6,6,7,7,8,8,9,9,10,10,10-hexadecafluor-dec-2-enyl)-methyl-amino]-pentyl}-estra-1,3,5 (10)-trien-3,17β-diol als Schaum erhalten, $[\alpha]_D$ = +35 ° (c=0,5 in $CHCl_3$)

**Herstellung der Ausgangsverbindungen:**

**(3,4,4,5,5,5-Hexafluor-pent-2-enyl)-methyl-amin**

In eine Lösung von 2,6 ml 1,1,1,2,2,3,3 Heptafluor-5-iod-pentan in 20 ml wasserfreiem Tetrahydrofuran werden bei -40 °C 3,55 g Methylamin kondensiert und über Nacht bei Raumtemperatur im Druckreaktor gerührt. Nachdem der Druckreaktor bei -30 °C geöffnet wurde, läßt man auf Raumtemperatur kommen, um überschüssiges Methylamin abdampfen zu lassen. Anschließend wir die Raktionslösung mit Diethylether versetzt, mit gesättigter Kochsalzlösung gewaschen, über Magnesiumsulfat getrocknet und i. Vak. eingeengt. Es werden 1,26 g (3,4,4,5,5,5-Hexafluor-pent-2-enyl)-methyl-amin als Rohprodukt erhalten.

**Methyl-(3,4,4,5,5,6,6,6-octafluor-hex-2-enyl)-amin**

In eine Lösung von 2,96 ml 1,1,1,2,2,3,3,4,4 Nonafluor-6-iod-hexan in 20 ml wasserfreiem Tetrahydrofuran werden bei -40 °C 2,36 g Methylamin kondensiert und über Nacht bei Raumtemperatur im Druckreaktor gerührt. Nachdem der Druckreaktor bei -30 °C geöffnet wurde, läßt man auf Raumtemperatur kommen, um überschüssiges Methylamin abdampfen zu lassen. Anschließend wir die Raktionslösung mit Diethylether versetzt, mit gesättigter Kochsalzlösung gewaschen, über Magnesiumsulfat getrocknet und i. Vak. eingeengt. Es werden 2,21 g Methyl-(3,4,4,5,5,6,6,6-octafluor-hex-2-enyl)-amin als Rohprodukt erhalten.

**(3,4,4,5,5,6,6,7,7,8,8,8-Dodecafluor-oct-2-enyl)-methyl-amin**

In eine Lösung von 2,82 ml 1,1,1,2,2,3,3,4,4,5,5,6,6 Tridecafluor-8-iod-octan in 20 ml wasserfreiem Tetrahydrofuran werden bei -40 °C 2,44 g Methylamin kondensiert und über Nacht bei Raumtemperatur im Druckreaktor gerührt. Nachdem der Druckreaktor bei -30 °C geöffnet wurde, läßt man auf Raumtemperatur kommen, um überschüssiges Methylamin abdampfen zu lassen. Anschließend wir die Raktionslösung mit Diethylether versetzt, mit gesättigter Kochsalzlösung gewaschen, über Magnesiumsulfat getrocknet und i. Vak. eingeengt. Es werden 2,23 g (3,4,4,5,5,6,6,7,7,8,8,8-Dodecafluor-oct-2-enyl)-methyl-amin als Rohprodukt erhalten.

**(3,4,4,5,5,6,6,7,7,8,8,9,9,10,10,10-Hexadecafluor-dec-2-enyl)-methyl-amin**

In eine Lösung von 2,86 g 1,1,1,2,2,3,3,4,4,5,5,6,6,7,7,8,8 Heptadecafluor-10-iod-decan in 20 ml wasserfreiem Tetrahydrofuran werden bei -40 °C 3,84 g Methylamin kondensiert und über Nacht bei Raumtemperatur im Druckreaktor gerührt. Nachdem der Druckreaktor bei -30 °C geöffnet wurde, läßt man auf Raumtemperatur kommen, um überschüssiges Methylamin abdampfen zu lassen. Anschließend wir die Raktionslösung mit Diethylether versetzt, mit gesättigter Kochsalzlösung gewaschen, über Magnesiumsulfat getrocknet und i. Vak. eingeengt. Es werden 1,83 g (3,4,4,5,5,6,6,7,7,8,8,9,9,10,10,10-Hexadecafluor-dec-2-enyl)-methyl-amin als Rohprodukt erhalten.

**Methyl-(4,4,5,5,6,6,7,7,8,8,9,9,9-tridecafluor-nonyl)-amin**

**a) 4,4,5,5,6,6,7,7,8,8,9,9,9-Tridecafluor-nonyltosylat**

2,33 ml 4,4,5,5,6,6,7,7,8,8,9,9,9-Tridecafluor-nonan-l-ol werden in 20 ml abs. Pyridin gelöst, bei 0°C mit 2,48 g p-Toluolsulfonylchlorid versetzt und 3h in der Kälte gerührt.. Dann wird das Reaktionsgemisch in Wasser eingerührt, mit Dichlormethan extrahiert, über Magnesiumsulfat getrocknet und i. Vak, eingeengt. Säulenchromatographische Reinigung an Kieselgel mit einem Hexan-Essigester-Gradienten liefert 3,86 g 4,4,5,5,6,6,7,7,8,8,9,9,9-Tridecafluor-nonyltosylat als klares Öl.

**b) Methyl-(4,4,5,5,6,6,7,7,8,8,9,9,9-tridecafluor-nonyl)-amin**

In eine Lösung von 3,86 g 4,4,5,5,6,6,7,7,8,8,9,9,9-Tridecafluor-nonyltosylat in 10 ml abs. Tetrahydrofuran werden bei -20°C 4,44 g Methylamin kondensiert und über Nacht im Druckgefäß bei Raumtemperatur gerührt. Nachdem das Druckgefäß bei -20°C geöffnet wurde, läßt man auf Raumtemperatur kommen, um überschüssiges Methylamin abdampfen zu lassen. Die Reaktionslösung wird in Dichlormethan aufgenommen, mit Wasser gewaschen, über Magnesiumsulfat getrocknet und i. Vak. eingeengt. Es werden 2,38 g Methyl-(4,4,5,5,6,6,7,7,8,8,9,9,9-tridecafluornonyl)-amin als Rohprodukt erhalten.

**(4,4,5,5,6,6,7,7,8,8,9,9,10,10,11,11,11-Heptadecafluor-undecyl)-methyl-amin**

**a) 4,4,5,5,6,6,7,7,8,8,9,9,10,10,11,11,11-Heptadecafluor-undecyltosylat**

4,78 g 4,4,5,5,6,6,7,7,8,8,9,9,10,10,11,11,11-Heptadecafluor-undecan-1-ol werden in 20 ml abs. Pyridin gelöst, bei 0°C mit 2,48 g p-Toluolsulfonylchlorid versetzt und 3h in der Kälte gerührt.. Dann wird das Reaktionsgemisch in Wasser eingerührt, mit Dichlormethan extrahiert, über Magnesiumsulfat getrocknet und i. Vak. eingeengt. Säulenchromatographische Reinigung an Kieselgel mit einem Hexan-Essigester-Gradienten liefert 4,3 g 4,4,5,5,6,6,7,7,8,8,9,9,10,10,11,11,11-Heptadecafluor-undecyltosylat als klares Öl.

**b) (4,4,5,5,6,6,7,7,8,8,9,9,10,10,11,11,11-Heptadecafluor-undecyl)-methyl-amin**

In eine Lösung von 4,3 g 4,4,5,5,6,6,7,7,8,8,9,9,10,10,11,11,11-Heptadecafluor-undecyltosylat in 10 ml abs. Tetrahydrofuran werden bei -20°C 3,95 g Methylamin kondensiert und über Nacht im Druckgefäß bei Raumtemperatur gerührt. Nachdem das Druckgefäß bei -20°C geöffnet wurde, läßt man auf Raumtemperatur kommen, um überschüssiges Methylamin abdampfen zu lassen. Die Reaktionslösung wird in Dichlormethan aufgenommen, mit Wasser gewaschen, über Magnesiumsulfat getrocknet und i. Vak. eingeengt. Es werden 3,1 g (4,4,5,5,6,6,7,7,8,8,9,9,10,10,11,11,11-Heptadecafluorundecyl)-methyl-amin als Rohprodukt erhalten.

**Methyl-(7,7,8,8,8-pentafluor-octyl)-amin**

**a) 7,7,8,8,8-Pentafluor-octyltosylat**

1,6 ml 7,7,8,8,8-Pentafluor-octan-1-ol werden in 20 ml abs. Pyridin gelöst, bei 0°C mit 2,48 g p-Toluolsulfonylchlorid versetzt und 3h in der Kälte gerührt.. Dann wird das Reaktionsgemisch in Wasser eingerührt, mit Dichlormethan extrahiert, über Magnesiumsulfat getrocknet und i. Vak. eingeengt. Säulenchromatographische Reinigung an Kieselgel mit einem Hexan-Essigester-Gradienten liefert 2,92 g 7,7,8,8,8-Pentafluor-octyltosylat als klares Öl.

**b) Methyl-(7,7,8,8,8-pentafluor-octyl)-amin**

In eine Lösung von 2,9 g 7,7,8,8,8-Pentafluor-octyltosylat in 10 ml abs. Tetrahydrofuran werden bei -20°C 4,0 g Methylamin kondensiert und über Nacht im Druckgefäß bei Raumtemperatur gerührt. Nachdem das Druckgefäß bei -20°C geöffnet wurde, läßt man auf Raumtemperatur kommen, um überschüssiges Methylamin abdampfen zu lassen. Die Reaktionslösung wird in Dichlormethan aufgenommen, mit Wasser gewaschen, über Magnesiumsulfat getrocknet und i. Vak. eingeengt. Es werden 1,58 g Methyl-(7,7,8,8,8-pentafluor-octyl)-amin als Rohprodukt erhalten.

**Methyl-(7,7,8,8,9,9,10,10,10-nonafluor-decyl)-amin**

**a) 7,7,8,8,9,9,10,10,10-Nonafluor-decyltosylat**

2,27 ml 7,7,8,8,9,9,10,10,10-Nonafluor-decan-1-ol werden in 20 ml abs. Pyridin gelöst, bei 0°C mit 2,48 g p-Toluolsulfonylchlorid versetzt und 3h in der Kälte gerührt.. Dann wird das Reaktionsgemisch in Wasser eingerührt, mit Dichlormethan extrahiert, über Magnesiumsulfat getrocknet und i. Vak. eingeengt. Säulenchromatographische Reinigung an Kieselgel mit einem Hexan-Essigester-Gradienten liefert 3,89 g 7,7,8,8,9,9,10,10,10-Nonafluor-decyltosylat als klares Öl.

**b) Methyl-(7,7,8,8,9,9,10,10,10-nonafluor-decyl)-amin**

In eine Lösung von 3,89 g 7,7,8,8,9,9,10,10,10-Nonafluor-decyltosylat in 10 ml abs. Tetrahydrofuran werden bei -20°C 4,12 g Methylamin kondensiert und über Nacht im Druckgefäß bei Raumtemperatur gerührt. Nachdem das Druckgefäß bei -20°C geöffnet wurde, läßt man auf Raumtemperatur kommen, um überschüssiges Methylamin abdampfen zu lassen. Die Reaktionslösung wird in Dichlormethan aufgenommen, mit Wasser gewaschen, über Magnesiumsulfat getrocknet und i. Vak. eingeengt. Es werden 2,63 g Methyl-(7,7,8,8,9,9,10,10,10-nonafluor-decyl)-amin als Rohprodukt erhalten.

**Methyl-(7,7,8,8,9,9,10,10,11,11,12,12,12-tridecafluor-dodecyl)-amin**

**a) 7,7,8,8,9,9,10,10,11,11,12,12,12-Tridecafluor-dodecyltosylat**

2,76 ml 7,7,8,8,9,9,10,10,11,11,12,12,12-Tridecafluor-dodecan-l-ol werden in 20 ml abs. Pyridin gelöst, bei 0°C mit 2,48 g p-Toluolsulfonylchlorid versetzt und 3h in der Kälte gerührt.. Dann wird das Reak-tionsgemisch in Wasser eingerührt, mit Dichlormethan extrahiert, über Magnesiumsulfat getrocknet und i. Vak. eingeengt. Säulenchromatographische Reinigung an Kieselgel mit einem Hexan-Essig-ester-Gradienten liefert 4,33 g 7,7,8,8,9,9,10,10,11,11,12,12,12-Tridecafluor-dodecyltosylat als klares Öl.

**b) Methyl-(7,7,8,8,9,9,10,10,11,11,12,12,12-tridecafluor-dodecyl)-amin**

In eine Lösung von 4,33 g 7,7,8,8,9,9,10,10,11,11,12,12,12-Tridecafluor-dodecyltosylat in 10 ml abs. Tetrahydrofuran werden bei -20°C 3,23 g Methylamin kondensiert und über Nacht im Druckgefaß bei Raumtemperatur gerührt. Nachdem das Druckgefäß bei -20°C geöffnet wurde, läßt man auf Raumtemperatur kommen, um überschüssiges Methylamin abdampfen zu lassen. Die Reaktionslösung wird in Dichlormethan aufgenommen, mit Wasser gewaschen, über Magnesiumsulfat getrocknet und i. Vak. eingeengt. Es werden 3,2 g Methyl-(7,7,8,8,9,9,10,10,11,11,12,12,12-tridecafluor-dodecyl)-amin als Rohprodukt erhalten.

**(7,7,8,8,9,9,10,10,11,11,12,12,13,13,14,14,14-Heptadecafluor-tetradecyl)-methyl-amin**

**a) 7,7,8,8,9,9,10,10,11,11,12,12,13,13,14,14,14-Heptadecafluor-tetradecyltosylat**

5,2 g 7,7,8,8,9,9,10,10,11,11,12,12,13,13,14,14,14-Heptadecafluor-tetradecan-1-ol werden in 20 ml abs. Pyridin gelöst, bei 0°C mit 2,48 g p-Toluolsulfonylchlorid versetzt und 3h bei Raumtemperatur gerührt.. Dann wird das Reaktionsgemisch in Wasser eingerührt, mit Dichlormethan extrahiert, über Magnesiumsulfat getrocknet und i. Vak. eingeengt. Säulenchromatographische Reinigung an Kieselgel mit einem Hexan-Essigester-Gradienten liefert 5,72 g 7,7,8,8,9,9,10,10,11,11,12,12,13,13,14,14,14-Heptadecafluor-tetradecyltosylat als klares Öl.

**b) (7,7,8,8,9,9,10,10,11,11,12,12,13,13,14,14,14-Heptadecafluor-tetradecyl)-methyl-amin**

In eine Lösung von 5,72 g 7,7,8,8,9,9,10,10,11,11,12,12,13,13,14,14,14-Heptadecafluortetradecyl-tosylat in 10 ml abs. Tetrahydrofuran werden bei -20°C 3,78 g Methylamin kondensiert und über Nacht im Druckgefäß bei Raumtemperatur gerührt. Nachdem das Druckgefäß bei -20°C geöffnet wurde, läßt man auf Raumtemperatur kommen, um überschüssiges Methylamin abdampfen zu lassen. Die Reaktionslösung wird in Dichlormethan aufgenommen, mit Wasser gewaschen, über Magnesium-sulfat getrocknet und i. Vak. eingeengt. Es werden 4,29 g (7,7,8,8,9,9,10,10,11,11,12,12,13,13, 14,14,14-Heptadecafluor-tetradecyl)-methyl-amin als Rohprodukt erhalten.

Beispiel 21

**11β-Fluor-7α-{5-[methyl-(3-phenoxy-propyl)-amino]-pentyl}-estra-1,3,5(10)-trien-3,17β-diol**

**a) 7α-(5-Chlorpentyl)-11β-fluor-estra-1,3,5(10)-trien-3,17β-diol**

5,0 g 7α-(5-Chlorpentyl)-11β-fluor-3-hydroxy-estra-1,3,5(10)-trien-17-on werden in 100 ml Methanol gelöst und portionsweise bei 0°C mit 0,96 g Natriumborhydrid versetzt. Nach 30 min Rühren bei 0°C wird das Reaktionsgemisch in halbgesättigte Natriumchloridlösung gegeben, 3 mal mit Essigester extrahiert und 1 mal mit gesättigter Kochsalzlösung gewaschen. Nach dem Trocknen über Magnesiumsulfat wird i. Vak. eingeengt. Präparative Säulenchromatographie an Kieselgel mit einem Hexan-Essigester-Gradienten liefert 4,29 g 7α-(5-Chlorpentyl)-11β-fluor-estra-1,3,5(10)-trien-3,17β-diol.

**b) 11β-Fluor-7α-(5-iodpentyl)-estra-1,3,5(10)-trien-3,17β-diol**

4,29 g 7α-(5-Chlorpentyl)-11β-fluor-estra-1,3,5(10)-trien-3,17β-diol werden in 60 ml Ethylmethylketon gelöst, mit 4,91 g Natriumjodid versetzt und über Nacht bei 90°C gerührt. Zur Aufarbeitung wird das Reaktionsgemisch auf Raumtemperatur abgekühlt, in Wasser eingerührt, 3 mal mit Essigester extrahiert, mit Thiosulfatlösung gewaschen, über Magnesium-sulfat getrocknet und i. Vak. eingeengt. Man erhält 5,16 g 11β-Fluor-7α-(5-iodpentyl)-estra-1,3,5(10)-trien-3,17β-diol als Rohprodukt, welches ohne weitere Aufreinigung in der nächsten Stufe eingesetzt wird.

**c) 11β-Fluor-7α-{5-[methyl-(3-phenoxy-propyl)-amino]-pentyl}-estra-1,3,5(10)-trien-3,17β-diol**

Eine Lösung von 486 mg 11β-Fluor-7α-(5-iodpentyl)-estra-1,3,5(10)-trien-3,17β-diol in 10 ml 1-Methyl-2-pyrrolidinon wird mit 600 mg Methyl-(3-phenoxy-propyl)-amin 3h bei 80°C Badtemperatur gerührt. Anschließend wird der Ansatz auf gesättigte Natriumchloridlösung gegeben, mit Ether extrahiert, über Natiumsulfat getrocknet, i. Vak. eingeengt und an Kieselgel mit Methylenchlorid / Methanol / Ammoniak chromatographiert. Man erhält 443 mg 11β-Fluor-7α-{5-[methyl-(3-phenoxy-propyl)-amino]-pentyl}-estra-1,3,5(10)-trien-3,17β-diol als Schaum [a]D= +47° (c=0,5 in $CHCl_3$).

Beispiel 22

**7α-{5-[(3-Benzyloxy-propyl)-methyl-amino]-pentyl}-11β-fluor-estra-1,3,5(10)-trien-3,17β-diol**

Eine Lösung von 486 mg 11β-Fluor-7α-(5-iodpentyl)-estra-1,3,5(10)-trien-3,17β-diol in 10 ml 1-Methyl-2-pyrrolidinon wird mit 540 mg (3-Benzyloxy-propyl)-methyl-amin 3h bei 80°C Badtemperatur gerührt. Anschließend wird der Ansatz auf gesättigte Natriumchloridlösung gegeben, mit Ether extrahiert, über Natiumsulfat getrocknet, i. Vak. eingeengt und an Kieselgel mit Methylenchlorid / Methanol / Ammoniak chromatographiert. Man erhält 237 mg 7α-{5-[(3-Benzyloxy-propyl)-methyl-amino]-pentyl}-11β-fluor-estra-1,3,5(10)-trien-3,17β-diol als Schaum $[a]_D$= +48°

(c=0,5 in $CHCl_3$).

**Herstellung der Ausgangsverbindungen**

**Methyl-(3-phenoxy-propyl)-amin**

In eine Lösung von 1,6 ml 3-Phenoxy-propylbromid in 15 ml wasserfreiem Tetrahydrofuran werden bei - 78°C 3,57 g Methylamin kondensiert und über Nacht bei Raumtemperatur im Druckreaktor gerührt. Nachdem der Druckreaktor bei -20°C geöffnet wurde, läßt man auf Raumtemperatur kommen, um überschüssiges Methylamin abdampfen zu lassen. Anschließend wird die Reaktionslösung in gesättigte Natriumchloridlösung gegeben, mit Ether extrahiert, über Magnesiumsulfat getrocknet und i. Vak. eingeengt. Es werden 2,01 g Methyl-(3-phenoxy-propyl)-amin als Rohprodukt erhalten, welches ohne weitere Aufreinigung in der nächsten Stufe eingesetzt wird.

**(3-Benzyloxy-propyl)-methyl-amin**

In eine Lösung von 1,8 ml (3-Brom-propoxymethyl)-benzen in 15 ml wasserfreiem Tetrahydrofuran werden bei - 78°C 3.57 g Methylamin kondensiert und über Nacht bei Raum-temperatur im Druckreaktor gerührt. Nachdem der Druckreaktor bei -20°C geöffnet wurde, läßt man auf Raumtemperatur kommen, um überschüssiges Methylamin abdampfen zu lassen. Anschließend wird die Reaktionslösung in gesättigte Natriumchloridlösung gegeben, mit Ether extrahiert, über Magnesiumsulfat getrocknet und i. Vak. eingeengt. Es werden 1,91 g (3-Benzyloxy-propyl)-methylamin als Rohprodukt erhalten, welches ohne weitere Aufreinigung in der nächsten Stufe eingesetzt wird.

Beispiel 23

**11β-Fluor-7α-{5-[N-methyl-N-3-(4,4,5,5,5-pentafluorpentyloxy)-proopylamino]-pentyl}estra-1,3,5(10)-trien-3,17β-diol**

**a) 11β-Fluor-7α-(5-brompentyl)-3-(tetrahydropyran-2-yloxy)-estra-1,3,5(10)-trien-17-on**

8.4 g 11β-Fluor-7α-(5-brompentyl)-3-hydroxy-estra-1,3,5(10)-trien-17-on in 90 ml Tetrahydrofuran läßt man mit 9.0 ml 3,4-Dihydropyran und 805 mg p-Toluolsulfonsäure Hydrat bei Raumtemperatur reagieren. Nach 7 h gibt man 1 ml Triethylamin zu, verdünnt mit Essigester, wäscht mit gesättigter Natriumchlorid-Lösung neutral und trocknet über Natriumsulfat. Die Chromatographie an Kieselgel mit einem Hexan-Essigester-Gradienten ergibt 7.6 g 11β-Fluor-7α-(5-brompentyl)-3-(tetrahydropyran-2-yloxy)-estra-1,3,5(10)-trien-17-on als Schaum.

**b) 11-β-Fluor-7α-{5-[(3-tert-butyl-dimethylsilyloxypropyl)-methylamino]-pentyl}-3-(tetrahydropyran-2-yloxy)-estra-1,3,5(10)-trien-17-on**

6.0 g 11β-Fluor-7α-(5-brompentyl)-3-(tetrahydropyran-2-yloxy)-estra-1,3,5(10)-trien-17-on in 130 ml Dimethylformamid werden bei 100 °C mit 5.7 g 1-N-Methylamino-3-tert-butyldimethylsilyloxypropan (hergestellt aus 1-Brom-3-hydroxypropan durch Umsetzung mit tert-Butyl-dimethylsilylchlorid zum 1-Brom-3-tert-butyl-dimethylsilyloxypropan und folgender Reaktion mit Methylamin) umgesetzt. Nach 7 Stunden verdünnt man mit Essigester, wäscht mit gesättigter Natriumchlorid-Lösung und engt im Vak. ein. Nach Chromatographieren des Rohproduktes an Kieselgel mit einem Methylenchlorid-Methanol-Gradienten werden 4.5 g 11-β-Fluor-7α-{5-[(3-tert-butyl-dimethylsilyloxypropyl)-methylamino]-pentyl}-3-(tetrahydropyran-2-yloxy)-estra-1,3,5(10)-trien-17-on als Öl erhalten.

**c) 11β-Fluor-7α-{5-[(3-hydroxypropyl)-methylamino]-pentyl}-estra-1,3,5(10)-trien-17-on**

2.3 g 11-β-Fluor-7α-{5-[(3-tert-butyl-dimethylsilyloxypropyl}-methylamino]-pentyl}-3-(tetrahydropyran-2-yloxy)-estra-1,3,5(10)-trien-17-on in 23 ml Tetrahydrofuran werden bei Raumtemperatur mit 6 ml Tetrabutylammoniumfluorid-Lösung (1M in Tetrahydrofuran) umgesetzt. Nach 2 Stunden verdünnt man mit Essigester, wäscht mit gesättigter Natriumchlorid-Lösung, trocknet und engt i. Vak. ein. Die Chromatographie des Rohproduktes an Kieselgel mit einem Methylenchlorid-Methanol- Gradienten ergibt 1.5 g 11 β-Fluor-7α-{5-[(3-hydroxypropyl)-methylamino]-pentyl)-extra-1,3,5(10)-trien-17-on als Schaum.

**d) 11β-Fluor-3-(tetrahydropyran-2-yloxy)-7α-{5-[N-methyl-N-3-(4,4,5,5,5-pentafluorpentyl-oxy)-propylamino]-pentyl}-estra-1,3,5(10)-trien-17-on**

Zu 710 mg 11β-Fluor-7α-{5-[(3-hydroxypropyl)-methylamino]-pentyl}-estra-1,3,5(10)-trien-17-on in 10 ml Toluol werden bei Raumtemperatur 1,8 g Pentafluorpentyliodid, 10 ml 40 proz. Natronlauge und 465 mg Tetrabutylammoniumhydrogensulfat gegeben. Nach 6 Tagen gibt man das Reaktionsgemisch in Eis / Wasser, extrahiert mit Essigester, wäscht mit gesättigter Natriumchlorid-Lösung, trocknet und engt im.Vak. ein. Es werden nach Chromatographieren des Rohproduktes an Kieselgel mit einem Methylenchlorid-Methanol-Gradienten 123 mg 11β-Fluor-3-(tetrahydropyran-2-yloxy)-7α-{5-[N-methyl-N-3-(4,4,5,5,5-pentafluorpentyloxy)-propylamino]-pentyl}-estra-1,3,5(10)-trien-17-on als Öl erhalten.

**e) 11β-Fluor-3-(tetrahydropyran-2-yloxy)-7α-{5-[N-methyl-N-3-(4,4,5,5,5-pentafluorpentyl-oxy)-propylamino]-pentyl}-estra-1,3,5(10)-trien-17β-ol**

120 mg 11β-Fluor-3-(tetrahydropyran-2-yloxy)-7α-{5-[N-methyl-N-3-(4,4,5,5,5-pentafluorpentyloxy)-propylamino]-pentyl}-estra-1,3,5(10)-trien-17-on werden bei Raumtemperatur in einem Gemisch aus 1 ml Tetrahydrofuran, 0.6 ml Ethanol und 0.3 ml Wasser mit 20 mg Natriumborhydrid reduziert. Nach 1 h verdünnt man mit Essigester, wäscht mit Wasser neutral, engt im Vak. ein und trocknet. Es werden 76 mg 11β-Fluor-3-(tetrahydropyran-2-yloxy)-7α-{5-[N-methyl-N-3-(4,4,5,5,5-pentafluorpentyloxy)-propylamino]-pentyl}-estra-1,3,5(10)-trien-17β-ol als Rohprodukt erhalten.

**f) 11β-Fluor-7α-{5-[N-methyl-N-3-(4,4,5,5,5-pentafluorpentyloxy)-propylamino]-pentyl}-estra-1,3,5(10)-trien-3,17β-diol**

75 mg 11β-Fluor-3-(tetrahydropyran-2-yloxy)-7α-{5-[N-methyl-N-3-(4,4,5,5,5-pentafluorpentyloxy)-propylamino]-pentyl}-estra-1,3,5(10)-trien-17β-ol werden in 1 ml Methanol und 0.1 ml Wasser mit 50 mg Oxalsäure bei Raumtemperatur umgesetzt. Nach 2 Stunden verdünnt man mit Methylenchlorid, wäscht mit gesättigter Natriumchlorid-Lösung neutral, trocknet und engt im Vak. ein. Das Rohprodukt ergibt nach Chromatographieren an Kieselgel mit einem Methylenchlorid-Methanol-Gradienten 25 mg 11β-Fluor-7α-{5-[Nmethyl-N-3-(4,4,5,5,5-pentafluorpentyloxy)-propylamino]-pentyl}-estra-1,3,5(10)-trien-3,17β -diol als Schaum.

Beispiel 24

**11β-Fluor-7α-[9-(4,4,5,5,5-pentafluorpentylsulfinyl)-nonyl]-estra-1,3,5(10)-trien-3,17βdiol**

**a) 3,11α-Diacetoxy-estra-3,5-dien-17-on**

Zu 100 g 11α-Hydroxy-estr-4-en-3,17-dion in 1 Liter Essigsäureanhydrid gibt man bei Raumtemperatur 10 g p-Toluolsulfonsäure. Nach 5 h rührt man die Lösung in pyridinhaltiges Eis / Wasser ein, saugt das ausgefallene Produkt ab und wäscht mit Wasser neutral. Das erhaltene, wasserfeuchte Rohprodukt versetzt man mit 200 ml Methanol, rührt 30 min bei -30 °C, saugt das Kristallisat ab, wäscht mit kaltem Methanol und trocknet im Vak. bei 50 °C. Es werden 115 g 3,1 1α-Diacetoxy-estra-3,5-dien-17-on erhalten.

**b) 11α-Acetoxy-estra-4,6-dien-3,17-dion**

Zu 57 g 3,11α-Diacetoxy-estra-3,5-dien-17-on in 470 ml N-Methyl-2-pyrrolidinon gibt man unter Eiskühlung 48 ml einer 10 proz. wäßrigen Natriumacetat-Lösung und portionsweise 22,3 g 1,3-Dibrom-5,5-dimethylhydantoin. Nach 30 min setzt man 16,7 g Natriumsulfit zu, rührt 15 min bei 0 °C, versetzt anschließend mit 20 g Lithiumbromid und 16 g Lithimcarbonat und rührt bei 100 °C. Nach 2 Stunden gibt man das Reaktionsgemisch in Eis / Wasser. Das ausgefallene Produkt wird abgesaugt, in Methylenchlorid gelöst, mit Wasser neutral gewaschen und getrocknet. Das Rohprodukt wird aus 200 ml Essigester umkristallisiert. Es werden 30 g 11α-Acetoxy-estra-4,6-dien-3,17-dion vom Schmelzpunkt 246-248 °C erhalten.

**c) 11α-Acetoxy-7α-(9-tert-butyldimethylsilyloxynonyl)-estr-4-en-3,17-dion**

Die Grignard-Lösung aus 2,2 g Magnesiumspäne und 34,2 g 1-Brom-9-tert-butyl-dimethylsilyloxynonan ( hergestellt aus 9-Brom-1-nonanol und tert-Butyl-dimethylsilylchlorid) in 140 ml Tetrahydrofuran versetzt man bei -30 °C mit 8,8 g Kupfer(I)-iodid. Nach 30 min tropft man bei gleicher Temperatur die Lösung von 9 g 11α-Acetoxy-estra-4,6-dien-3,17-dion in einem Gemisch aus 64 ml Tetrahydrofuran und 10,6 ml 1,3-Dimethyl-2-oxohexahydropyrimidin

(DMPU) bei -30 °C zu, rührt 2 Stunden und versetzt dann mit 6 ml Eisessig. Das Reaktionsgemisch wird nach 15 Minuten mit Essigester verdünnt, mit gesättigter Ammoniumchlorid-Lösung und Wasser neutral gewaschen und getrocknet. Nach Chromatographieren des Rohproduktes an Kieselgel mit einem Hexan-Essigester-Gradienten erhält man 6 g 11α-Acetoxy-7α-(9-tert-butyldimethylsilyloxynonyl)-estr-4-en-3,17-dion vom Schmelzpunkt 120-122 °C.

**d) 11α-Acetoxy-7α-(9-hydroxynonyl)-estr-4-en-3,17-dion**

19.9 g 11α-Acetoxy-7α-(9-tert-butyldimethylsilyloxynonyl)-estr-4-en-3,17-dion in 90 ml Methanol werden bei Raumtemperatur mit 20 ml 8 proz. Schwefelsäure versetzt. Nach 2.5 Stunden verdünnt man mit Essigester, wäscht mit gesättigter Natriumhydrogencarbonat-Lösung und Wasser neutral und trocknet. Die Chromatographie des Rohproduktes an Kieselgel mit einem Methylenchlorid-Aceton-Gradienten ergibt 15.8 g 11α-Acetoxy-7α-(9-hydroxynonyl)-estr-4-en-3,17-dion als Schaum.

**e) 11α-Acetoxy-7α-(9-chlornonyl)-estr-4-en-3,17-dion**

15.8 g 11α-Acetoxy-7α-(9-hydroxynonyl)-estr-4-en-3,17-dion werden in 65 ml Acetonitril mit 184 ml Tetrachlormethan und 17.9 g Triphenylphosphin bei Raumtemperatur umgesetzt. Nach 5 Stunden verdünnt man mit Methylenchlorid, wäscht mit gesättigter Natriumhydrogencarbonat-Lösung und Wasser neutral und trocknet. Das Rohprodukt wird an Kieselgel mit einem Hexan-Essigester-Gradienten chromatographiert. Ausbeute: 7,8 g 11α-Acetoxy-7α-(9-chlornonyl)-estr-4-en-3,17-dion vom Schmelzpunkt 113-115 °C.

**f) 7α-(9-Chlornonyl)-11α-hydroxy-estr-4-en-3,17-dion**

6.0 g 11α-Acetoxy-7α-(9-chlornonyl)-estr-4-en-3,17-dion werden bei 60 °C in einem Gemisch aus 12 ml Tetrahydrofuran und 12 ml Methanol mit 17 ml einer 1N Kalilauge verseift. Nach 3 Stunden wird mit Essigester verdünnt, mit gesättigter Natriumchlorid-Lösung und Wasser neutal gewaschen und getrocknet. Die Chromatographie des Rohproduktes an Kieselgel mit einem Methylenchlorid-Aceton-Gradienten ergibt 3.2 g 7α-(9-Chlornonyl)-11α -hydroxy-estr-4-en-3,17-dion als Öl.

**g) 7α-(9-Chlornonyl)-11β-fluor-estr-4-en-3,17-dion**

Zu 6.7 g 7α-(9-Chlomonyl)-11α-hydroxy-estr-4-en-3,17-dion in 35 ml Essigester werden bei 0 °C 3.3 ml 1,8 -Diazabicyclo[5,4,0]undec-7-en und tropfenweise 4 ml Perfluorbutan-1-sulfonsäurefluorid gegeben. Nach 2 Stunden verdünnt man mit Essigester, wäscht mehrmals mit 2N Salzsäure und neutralisiert mit gesättigter Natriumhydrogencarbonat-Lösung und Wasser. Das Rohprodukt wird an Kieselgel mit einem Hexan-Essigester-Gradienten chromatographiert. Ausbeute: 6.2 g 7α-(9-Chlornonyl)-11β-fluor-estr-4-en-3,17-dion als Öl.

**h) 7α-(9-Chlornonyl)-11β-fluor-3-hydroxy-estra-1,3,5(10)-trien-17-dion**

Zu 5.6 g 7α-(9-Chlomonyl)-11β-fluor-estr-4-en-3,17-dion in 55 ml Acetonitril wird bei 80 °C eine Lösung von 5.5 g Kupfer(II)-bromid und 1.1 g Lithiumbromid in 55 ml Acetonitril getropft. Nach 2 Stunden verdünnt man mit Essigester, neutralisiert mit Natriumhydrogen-carbonat-Lösung, wäscht mit Wasser, trocknet und engt im Vak. ein. Die Chromatographie an Kieselgel mit einem Hexan-Ether-Gradienten ergibt 1.1 g 7α-(9-Chlornonyl)-11β-fluor-3-hydroxy-estra-1,3,5(10)-trien-17-dion als Schaum.

**i) 7α-(9-Chlornonyl)-11β-flu or-3-(tetrahydropyran-2-yloxy)-estra-1,3,5(10)-trien-17-dion**

3.0 g 7α-(9-Chlornonyl)-11β-fluor-3-hydroxy-estra-1,3,5(10)-trien-17-dion in 30 ml Tetrahydrofuran werden bei Raumtemperatur mit 2.9 ml 3,4-Dihydropyran und 150 mg p-Toluolsulfonsäure Hydrat umgesetzt. Nach 3 Stunden verdünnt man mit Essigester, neutralisiert mit gesättigter Natriumhydrogencarbonat-Lösung und Wasser, trocknet und engt im Vak. ein. Das Rohprodukt ergibt nach Chromatographieren an Kieselgel mit einem Hexan-Essigester-Gradienten 2.8 g 7α-(9-Chlornonyl)-11β-fluor-3-(tetrahydropyran-2-yloxy)-estra-1,3,5(10)-trien-17-dion als Öl.

**j) 11β-Fluor-7α-(9-iodnonyl)-3-(tetrahydropyran-2-yloxy)-estra-1,3,5(10)-trien-17-dion**

2.6 g 7α-(9-Chlornonyl)-11β-fluor-3-(tetrahydropyran-2-yloxy)-estra-1,3,5(10)-trien-17-dion in 20 ml Ethylmethylketon werden bei 80 °C mit 2.9 g Natriumiodid gerührt. Nach 20 Stunden verdünnt man mit Ether, wäscht mit Wasser, trocknet und engt im Vak. ein. Das Rohprodukt wird an Kieselgel mit einem Hexan-Ether-Gradienten chromatographiert. Ausbeute: 1.2 g 11β-Fluor-7α-(9-iodnonyl)-3-(tetrahydropyran-2-yloxy)-estra-1,3,5(10)-trien-17-dion als Öl.

**k) 11β-Fluor-[9-(4,4,5,5,5-pentafluorpentylthio)-nonyl]-3-(tetrahydropyran-2-yloxy)-estra-1,3,5(10)-trien-17-on**

Zu 980 mg S-(4,4,5,5,5-Pentafluorpentyl)thioacetat in 10 ml Methanol werden unter Eiskühlung 0.83 ml 30 proz.methanolische Natriummethylat-Lösung getropft. Es wird 30 Minuten bei Raumtemperatur gerührt. Dann gibt man die Lösung von 1.9 g 11β-Fluor-7α-(9-iodnonyl)-3-(tetrahydropyran-2-yloxy)-estra-1,3,5(10)-trien-17-dion in 10 ml Dimethylformamid zu und rührt bei 50 °C. Nach 18 Stunden verdünnt man mit Ether, wäscht mit gesättigter Natriumchlorid-Lösung und Wasser neutral, trocknet und engt im Vak. ein. Das Rohprodukt wird an Kieselgel mit einem Hexan-Ether-Gradienten chromatographiert. Es werden 1.3 g.11β-Fluor-[9-(4,4,5,5,5-pentafluorpentylthio)-nonyl]-3-(tetrahydropyran-2-yloxy)-estra-1,3,5(10)-trien-17-on als Öl erhalten.

**1) 11β-Fluor-[9-(4,4,5,5,5-pentafluorpentylthio)-nonyl]-3-(tetrahydropyran-2-yloxy)-estra-1,3,5(10)-trien-17β-ol**

2.2 g 11β-Fluor-[9-(4,4,5,5,5-pentafluorpentylthio)-nonyl]-3-(tetrahydropyran-2-yloxy)-estra-1,3,5(10)-trien-17-on in einem Gemisch aus 45 ml Methanol, 7 ml Tetrahydrofuran und 4.5 ml Wasser werden portionsweise mit 450 mg Natriumborhydrid bei 0 °C versetzt. Nach 45 Minuten verdünnt man mit Essigester, wäscht mit Wasser, trocknet und engt im Vak. ein. Chromatographieren des Rohproduktes an Kieselgel mit einem Hexan-Essigester-Gradienten ergibt 1.8 g 11β-Fluor-[9-(4,4,5,5,5-pentafluorpentylthio)-nonyl]-3-(tetrahydropyran-2-yloxy)-estra-1,3,5(10)-trien-17β-ol als Öl.

**m) 11β-Fluor-[9-(4,4,5,5,5-pentafluorpentylthio)-nonyl]-estra-1,3,5(10)-trien-3,17β-ol**

1.8 g 11β-Fluor-[9-(4,4,5,5,5-pentafluorpentylthio)-nonyl]-3-(tetrahydropyran-2-yloxy)-estra-1,3,5(10)-trien-17β-ol in 68 ml Methanol und 6.8 ml Wasser läßt man bei 60 °C mit 900 mg Oxalsäure reagieren. Nach 2 Stunden verdünnt man mit Essigester, neutralisiert mit Natriumhydrogencarbonat-Lösung, wäscht mit Wasser, trocknet und engt im Vak. ein. Die Chromatographie des Rohproduktes an Kieselgel mit einem Hexan-Essigester-Gradienten ergibt 1.4 g 11β-Fluor-[9-(4,4,5,5,5-pentafluorpentylthio)-nonyl]-estra-1,3,5(10)-trien-3,17β-ol als Schaum.

**n) 11β-Fluor-[9-(4,4,5,5,5-pentafluorpentylsulfinyl)-nonyl]-estra-1,3,5(10)-trien-3,17β-ol**

300 mg 11β-Fluor-[9-(4,4,5,5,5-pentafluorpentylthio)-nonyl]-estra-1,3,5(10)-trien-3,17β-ol in 12 ml Methanol und 2.4 ml Wasser werden bei Raumtemperatur mit 241 mg Natriumperiodat versetzt. Nach 1.5 Stunden verdünnt man mit Essigester, wäscht mit gesättigter Natriumchlorid-Lösung und Wasser neutral, trocknet und engt im Vak. ein. Das Rohprodukt wird an Kieselgel mit einem Methylenchlorid-Aceton-Gradienten chromatographiert. Ausbeute: 287 mg 11β-Fluor-[9-(4,4,5,5,5-pentafluorpentylsulfinyl)-nonyl]-estra-1,3,5(10)-trien-3,17β-ol als Schaum.

**Beispiel 25**

**N-[4-(11β-Fluor-3,17-dihydroxy-estra-1,3,5(10)-tries-7α-yl)-butyl)-N-pentyl-benzamid**

**a) 3-Benzyloxy-7α-(4-chlorbutyl)-11β-fluor-estra-1,3,5(10)-trien-17-on**

Eine Lösung von 3,9 g 7α-(4-Chlorbutyl)-11β-fluor-3-hydroxy-estra-1,3,5(10)-trien-17-on, welches analog wie die Beispiel 4b) beschriebene 6-Chlorhexyl-Verbindung hergestellt wurde, in 100 ml Acetonitril wird mit 2,28 g Kaliumcarbonat und 2,11 g Benzylbromid 24 Stunden bei 80 °C Badtemperatur gerührt. Dann wird zur Trockne eingeengt, mit Wasser versetzt, zweimal mit Dichlormethan extrahiert, mit Wasser und gesättigter Kochsalzlösung gewaschen, über Natriumsulfat getrocknet, i.Vak. eingeengt und an Kieselgel mit Dichlormethan / Essigester chromatographiert. Man erhält 5,14 g 3-Benzyloxy-7α-(4-chlorbutyl)-11β-fluor-estra-1,3,5(10)-trien-17-on als Schaum.

**b) 7α-(5-Azadecyl)-3-benzyloxy-11β-fluor-estra-1,3,5(10)-trien-17-on**

Eine Lösung von 273 mg 3-Benzyloxy-7α-(4-chlorbutyl)-11β-fluor-estra-1,3,5(10)-trien-17-on in 7 ml Dimethylformamid wird mit 262 mg Natriumiodid und 0,5 ml n-Pentylamin 24 Stunden bei 80 °C gerührt. Dann gibt man das Reaktionsgemisch in Eis-Wasser, extrahiert mit Essigester, wäscht mit gesättigter Natriumchloridlösung, trocknet und engt im Vakuum ein. Nach Chromatographieren des Rohproduktes an Kieselgel mit einem Methylenchlorid-Methanol-Gradienten werden 275 mg 7α-(5-Azadecyl)-3-benzyloxy-11β-fluor-estra-1,3,5(10)-trien-17-on erhalten.

**c) N-[4-(3-Benzyloxy-11β-fluor-estra-1,3,5(10)-trien-17-on-7α-yl)-butyl]-N-pentylbenzamid**

Eine Lösung von 275 mg 7α-(5-Azadecyl)-3-benzyloxy-11β-fluor-estra-1,3,5(10)-trien-17-on in 7,8 ml Dimethylformamid wird mit 175 mg Benzoesäure und 250 mg 1-Hydroxybenzotriazol sowie 0,2 ml N,N'-Diisopropylcarbodiimid 24 Stunden bei Raumtemperatur gerührt. Dann gibt man das Reaktionsgemisch in Eis-Wasser, extrahiert mit Essigester, wäscht mit gesättigter Natriumchloridlösung, trocknet und engt im Vakuum ein. Nach Chromatographieren des Rohproduktes an Kieselgel mit einem Methylenchlorid-Methanol-Gradienten werden 197 mg N-[4-(3-Benzyloxy-11β-fluor-estra-1,3,5(10)-trien-17-on-7α-yl)-butyl]-N-pentyl-benzamid erhalten.

**d) N-[4-(11β-Fluor-3-hydroxy-estra-1,3,5(10)-trien-17-on-7α-yl)-butyl]-N-pentylbenzamid**

Eine Lösung von 197 mg N-[4-(3-Benzyloxy-11β-fluor-estra-1,3,5(10)-trien-17-on-7α-yl)-butyl]-N-pentyl-benzamid in 4,8 ml Dichlormethan wird mit 2 ml Thioanisol und 1,2 ml Trifluoressigsäure 3 Stunden bei Raumtemperatur gerührt. Dann wird mit Dichlormethan verdünnt, mit 1-molarer Natronlauge gewaschen, mit Wassser neutralgewaschen, über Natriumsulfat getrocknet, i.Vak. eingeengt und an Kieselgel mit Dichlormethan /Methyl*tertiär*butylether chromatographiert. Man erhält 147 mg N-[4-(11β-Fluor-3-hydroxyestra-1,3,5(10)-trien-17-on-7α-yl)-butyl]-N-pentyl-benzamid.

**e) N-[4-(11β-Fluor-3,17-dihydroxy-estra-1,3,5(10)-trien-7α-yl)-butyl]-N-pentylbenzamid**

Eine Lösung von 110 mg N-[4-(11β-Fluor-3-hydroxy-estra-1,3,5(10)-trien-17-on-7α-yl)-butyl]-N-pentyl-benzamid in 1,5 ml Dichlormethan und 4,5 ml Methanol wird bei Raumtemperatur mit 110 mg Natriumborhydrid versetzt und 0,5 Stunden gerührt. Dann wird mit Wasser verdünnt, mit 25 ml Essigester extrahiert, zweimal mit Wasser gewaschen, über Natriumsulfat getrocknet i.Vak. eingeengt und an Kieselgel mit Hexan / Essigester chromatographiert. Man erhält 112 mg reines N-[4-(11β-Fluor-3,17-dihydroxy-estra-1,3,5(10)-trien-7α-yl)-butyl]-N-pentyl-benzamid als Schaum. $[\alpha]_D^{22}$ = +42,3° (c = 0.51% in Chloroform).

Durch Auswahl je eines der folgenden ω-Chloride für den Reaktionsschritt a): 7α-(4-Chlorbutyl)-11β-fluor-3-hydroxy-estra-1,3,5(10)-trien-17-on, 7α-(5-Chlorpentyl)-11β-fluor-3-hydroxy-estra-1,3,5(10)-trien-17-on (Beispiel 2b)) und 7α-(6-Chlorhexyl)-11β-fluor-3-hydroxy-estra-1,3,5(10)-trien-17-on (Beispiel 4b))
sowie
je eines der folgenden Amine für den Reaktionsschritt b): Aminomethylcyclohexan, Benzylamin, 2-Methoxyethylamin, 3-Phenylpropylamin, Hexylamin und Octylamin
sowie
je einer der folgenden Säuren für den Reaktionsschritt c): 4-Acetaminobenzoesäure, 4-Cyanobenzoesäure, 3-Cyclohexylpropionsäure, 3,4-Dimethoxyphenylessigsäure, 4-Ethoxybenzoesäure, Laurinsäure, 2-Naphthylessigsäure, 4-Phenylbuttersäure, Propionsäure und 4-Biphenylessigsäure
erhält man durch analoge Ausführung der im Beispiel 25 beschriebenen Reaktionsschritte a-e die Verbindungen der folgenden Tabelle.

Anstelle der Umsetzung mit einer aktivierten Säure im Reaktionsschritt c) kann diese Umsetzung auch mit einem Alkylhalogenid (Halogen = Cl, Br) erfolgen, wobei dann anstelle des Amids direkt ein Amin erhalten wird.

In der Tabelle sind die $[M]^+$+1-Peaks der Massenspektren (MS) dieser Verbindungen angegeben; CI = Chemische Ionisation.

| Beispiel | Verbindung | MS (CI): $[M]^+$+1 [m/z] |
|---|---|---|
| 26 | 4-Acetylamino-N-cyclohexylmethyl-N-[4-(11β-fluor-3,17β-dihydroxy-estra-1,3,5(10)-trien-7α-yl)-butyl]-benzamid | 619 |
| 27 | 4-Acetylamino-N-benzyl-N-[4-(11β-fluor-3,17β-dihydroxy-estra-1,3,5(10)-trien-7α-yl)-butyl]-benzamid | 613 |
| 28 | 4-Acetylamino-N-[4-(11β-fluor-3,17β-dihydroxy-estra-1,3,5(10)-trien-7α-yl)-butyl] -N-(2-methoxy-ethyl)-benzamid | 581 |
| 29 | 4-Acetylamino-N-[4-(11β-fluor-3,17β-dihydroxy-estra-1,3,5(10)-trien-7α-yl)-butyl] -N-(3-phenyl-propyl)-benzamid | 641 |

(fortgesetzt)

| Beispiel | Verbindung | MS (CI): $[M]^{+}$+1 [m/z] |
|---|---|---|
| 30 | 4-Acetylamino-N-[4-(11β-fluor-3,17β-dihydroxy-estra-1,3,5(10)-trien-7α-yl)-butyl]-N-hexyl-benzamid | 607 |
| 31 | 4-Acetylamino-N-[4-(11β-fluor-3,17β-dihydroxy-estra-1,3,5(10)-trien-7α-yl)-butyl]-N-octyl-benzamid | 635 |
| 32 | 4-Cyano-N-cyclohexylmethyl-N-[4-(11β-fluor-3,17β-dihydroxy-estra-1,3,5(10)-trien-7α-yl)-butyl]-benzamid | 587 |
| 33 | N-Benzyl-4-cyano-N-[4-(11β-fluor-3,17β-dihydroxy-estra-1,3,5(10)-trien-7α-yl)-butyl]-benzamid | 581 |
| 34 | 4-Cyano-N-[4-(11β-fluor-3,17β-dihydroxy-estra-1,3,5(10)-trien-7α-yl)-butyl]-N-(2-methoxy-ethyl)-benzamid | 549 |
| 35 | 4-Cyano-N-[4-(11β-fluor-3,17β-dihydroxy-estra-1,3,5(10)-trien-7α-yl)-butyl]-N-(3-phenyl-propyl)-benzamid | 609 |
| 36 | 4-Cyano-N-[4-(11β-fluor-3,17β-dihydroxy-estra-1,3,5(10)-trien-7α-yl)-butyl]-N-hexyl-benzamid | 575 |
| 37 | 4-Cyano-N-[4-(11β-fluor-3,17β-dihydroxy-estra-1,3,5(10)-trien-7α-yl)-butyl]-N-octyl-benzamid | 603 |
| 38 | 3-Cyclohexyl-N-cyclohexylmethyl-N-[4-(11β-fluor-3,17β-dihydroxy-estra-1,3,5(10)-trien-7α-yl)-butyl]-propionamid | 596 |
| 39 | N-Benzyl-3-cyclohexyl-N-[4-(11β-fluor-3,17β-dihydroxy-estra-1,3,5(10)-trien-7α-yl)-butyl]-propionamid | 590 |
| 40 | 3-Cyclohexyl-N-[4-(11β-fluor-3,17β-dihydroxy-estra-1,3,5(10)-trien-7α-yl)-butyl] -N-(2-methoxy-ethyl)-propionamid | 558 |
| 41 | 3-Cyclohexyl-N-[4-(11β-fluor-3,17β-dihydroxy-estra-1,3,5(10)-trien-7α-yl)-butyl] -N-(3-phenyl-propyl)-propionamid | 618 |
| 42 | 3-Cyclohexyl-N-[4-(11β-fluor-3,17β-dihydroxy-estra-1,3,5(10)-trien-7α-yl)-butyl]-N-hexyl-propionamid | 584 |
| 43 | 3-Cyclohexyl-N-[4-(11β-fluor-3,17β-dihydroxy-estra-1,3,5(10)-trien-7α-yl)-butyl]-N-octyl-propionamid | 612 |
| 44 | N-Cyclohexylmethyl-N-[4-(11β-fluor-3,17β-dihydroxy-estra-1,3,5(10)-trien-7α-yl)-butyl] -(3,4-dimethoxyphenyl)-acetamid | 636 |
| 45 | N-Benzyl-N-[4-(11β-fluor-3,17β-dihydroxy-estra-1,3,5(10)-trien-7α-yl)-butyl] -(3,4-dimethoxyphenyl)-acetamid | 630 |
| 46 | N-[4-(11β-Fluor-3,17β-dihydroxy-estra-1,3,5(10)-trien-7α-yl)-butyl]-N-(2-methoxy-ethyl) -(3,4-dimethoxyphenyl)-acetamid | 598 |
| 47 | N-[4-(11β-Fluor-3,17β-dihydroxy-estra-1,3,5(10)-trien-7α-yl)-butyl]-(3,4-dimethoxyphenyl) -N-(3 -phenyl-propyl)-acetamid | 658 |
| 48 | N-[4-(11β-Fluor-3,17β-dihydroxy-estra-1,3,5(10)-trien-7α-yl)-butyl]-N-hexyl-(3,4-dimethoxyphenyl)-acetamid | 624 |
| 49 | N-[4-(11β-Fluor-3,17β-dihydroxy-estra-1,3,5(10)-trien-7α-yl)-butyl]-(3,4-dimethoxyphenyl) -N-octyl-acetamid | 652 |
| 50 | N-Cyclohexylmethyl-4-ethoxy-N-[4-(11β-fluor-3,17β-dihydroxy-estra-1,3,5(10)-trien-7α-yl)-butyl]-benzamid | 606 |

(fortgesetzt)

| Beispiel | Verbindung | MS (CI): $[M]^{+}+1$ [m/z] |
|---|---|---|
| 51 | N-Benzyl-4-ethoxy-N-[4-(11β-fluor-3,17β-dihydroxy-estra-1,3,5(10)-trien-7α-yl)-butyl]-benzamid | 600 |
| 52 | 4-Ethoxy-N-[4-(11β-fluor-3,17β-dihydroxy-estra-1,3,5(10)-trien-7α-yl)-butyl]-N-(2-methoxy-ethyl)-benzamid | 568 |
| 53 | 4-Ethoxy-N-[4-(11β-fluor-3,17β-dihydroxy-estra-1,3,5(10)-trien-7α-yl)-butyl]-N-(3-phenyl-propyl)-benzamid | 628 |
| 54 | 4-Ethoxy-N-[4-(11β-fluor-3,17β-dihydroxy-estra-1,3,5(10)-trien-7α-yl)-butyl]-N-hexyl-benzamid | 594 |
| 55 | 4-Ethoxy-N-[4-(11β-fluor-3,17β-dihydroxy-estra-1,3,5(10)-trien-7α-yl)-butyl]-N-octyl-benzamid | 622 |
| 56 | N-Cyclohexylmethyl-N-[4-(11β-fluor-3,17β-dihydroxy-estra-1,3,5(10)-trien-7α-yl)-butyl]-dodecanamid | 640 |
| 57 | N-Benzyl-N-[4-(11β-fluor-3,17β-dihydroxy-estra-1,3,5(10)-trien-7α-yl)-butyl]-dodecanamid | 634 |
| 58 | N-[4-(11β-Fluor-3,17β-dihydroxy-estra-1,3,5(10)-trien-7α-yl)-butyl]-N-(2-methoxy-ethyl)-dodecanamid | 602 |
| 59 | N-[4-(11β-Fluor-3,17β-dihydroxy-estra-1,3,5(10)-trien-7α-yl)-butyl]-N-(3-phenyl-propyl)-dodecanamid | 662 |
| 60 | N-[4-(11β-Fluor-3,17β-dihydroxy-estra-1,3,5(10)-trien-7α-yl)butyl]-N-hexyl-dodecanamid | 628 |
| 61 | N-[4-(11β-Fluor-3,17β-dihydroxy-estra-1,3,5(10)-trien-7α-yl)butyl]-N-octyl -dodecanamid | 657 |
| 62 | N-Cyclohexylmethyl-N-[4-(11β-fluor-3,17β-dihydroxy-estra-1,3,5(10)-trien-7α-yl)-butyl]-naphth-2-yl-acetamid | 626 |
| 63 | N-Benzyl-N-[4-(11β-fluor-3,17β-dihydroxy-estra-1,3,5(10)-trien-7α-yl)-butyl]-naphth-2-yl-acetamid | 620 |
| 64 | N-[4-(11β-Fluor-3,17β-dihydroxy-estra-1,3,5(10)-trien-7α-yl)-butyl]-N-(2-methoxy-ethyl)-naphth-2-yl-acetamid | 588 |
| 65 | N-[4-(11β-Fluor-3,17β-dihydroxy-estra-1,3,5(10)-trien-7α-yl)-butyl]-naphth-2-yl-N-(3-phenyl-propyl)-acetamid | 648 |
| 66 | N-[4-(11β-Fluor-3,17β-dihydroxy-estra-1,3,5(10)-trien-7α-yl)-butyl]-N-hexyl-naphth-2-yl-acetamid | 614 |
| 67 | N-[4-(11β-Fluor-3,17β-dihydroxy-estra-1,3,5(10)-trien-7α-yl)-butyl]-naphth-2-yl-N-octyl-acetamid | 642 |
| 68 | N-Cyclohexylmethyl-N-[4-(11β-fluor-3,17β-dihydroxy-estra-1,3,5(10)-trien-7α-yl)-butyl]-4-phenyl-butyramid | 604 |
| 69 | N-Benzyl-N-[4-(11β-fluor-3,17β-dihydroxy-estra-1,3,5(10)-trien-7α-yl)-butyl]-4-phenyl-butyramid | 598 |
| 70 | N-[4-(11β-Fluor-3,17β-dihydroxy-estra-1,3,5(10)-trien-7α-yl)-butyl]-N-(2-methoxy-ethyl)-4-phenyl-butyramid | 566 |
| 71 | N-[4-(11β-Fluor-3,17β-dihydroxy-estra-1,3,5(10)-trien-7α-yl)-butyl]-4-phenyl-N-(3-phenyl-propyl)-butyramid | 626 |
| 72 | N-[4-(11β-Fluor-3,17β-dihydroxy-estra-1,3,5(10)-trien-7α-yl)-butyl]-N-hexyl-4-phenyl-butyramid | 592 |

(fortgesetzt)

| Beispiel | Verbindung | MS (CI): $[M]^{+}+1$ [m/z] |
|---|---|---|
| 73 | N-[4-(11β-Fluor-3,17β-dihydroxy-estra-1,3,5(10)-trien-7α-yl)-butyl]-N-octyl-4-phenyl-butyramid | 620 |
| 74 | N-Cyclohexylmethyl-N-[4-(11β-fluor-3,17β-dihydroxy-estra-1,3,5(10)-trien-7α-yl)-butyl]-propionamid | 514 |
| 75 | N-Benzyl-N-[4-(11β-fluor-3,17β-dihydroxy-estra-1,3,5(10)-trien-7α-yl)-butyl]-propionamid | 508 |
| 76 | N-[4-(11β-Fluor-3,17β-dihydroxy-estra-1,3,5(10)-trien-7α-yl)-butyl]-N-(2-methoxy-ethyl)-propionamid | 476 |
| 77 | N-[4-(11β-Fluor-3,17β-dihydroxy-estra-1,3,5(10)-trien-7α-yl)-butyl]-N-(3-phenyl-propyl)-propionamid | 536 |
| 78 | N-[4-(11β-Fluor-3,17β-dihydroxy-estra-1,3,5(10)-trien-7α-yl)-butyl]-N-hexyl-propionamid | 502 |
| 79 | N-[4-(11β-Fluor-3,17β-dihydroxy-estra-1,3,5(10)-trien-7α-yl)-butyl]-N-octyl-propionamid | 530 |
| 80 | 4-Biphenyl-N-cyclohexylmethyl-N-[4-(11β-fluor-3,17β-dihydroxy-estra-1,3,5(10)-trien-7α-yl)-butyl]-acetamid | 652 |
| 81 | 4-Biphenyl-N-benzyl-N-[4-(11β-fluor-3,17β-dihydroxy-estra-1,3,5(10)-trien-7α-yl)-butyl]-acetamid | 646 |
| 82 | 4-Biphenyl-N-[4-(11β-fluor-3,17β-dihydroxy-estra-1,3,5(10)-trien-7α-yl)-butyl] -N-(2-methoxy-ethyl)-acetamid | 614 |
| 83 | 4-Biphenyl-N-[4-(11β-fluor-3,17β-dihydroxy-estra-1,3,5(10)-trien-7α-yl)-butyl]-N-(3-phenyl-propyl)-acetamid | 674 |
| 84 | 4-Biphenyl-N-[4-(11β-fluor-3,17β-dihydroxy-estra-1,3,5(10)-trien-7α-yl)-butyl]-N-hexyl-acetamid | 640 |
| 85 | 4-Biphenyl-N-[4-(11β-fluor-3,17β-dihydroxy-estra-1,3,5(10)-trien-7α-yl)-butyl]-N-octyl-acetamid | 668 |
| 86 | 4-Acetylamino-N-cyclohexylmethyl-N-[5-(11β-fluor-3,17β-dihydroxy-estra-1,3,5(10)-trien-7α-yl)-pentyl]-benzamid | 633 |
| 87 | 4-Acetylamino-N-benzyl-N-[5-(11β-fluor-3,17β-dihydroxy-estra-1,3,5(10)-trien-7α-yl)-pentyl]-benzamid | 627 |
| 88 | 4-Acetylamino-N-[5-(11β-fluor-3,17β-dihydroxy-estra-1,3,5(10)-trien-7α-yl)-pentyl] -N-(2-methoxy-ethyl)-benzamid | 595 |
| 89 | 4-Acetylamino-N-[5-(11β-fluor-3,17β-dihydroxy-estra-1,3,5(10)-trien-7α-yl)-pentyl] -N-(3-phenyl-propyl)-benzamid | 655 |
| 90 | 4-Acetylamino-N-[5-(11β-fluor-3,17β-dihydroxy-estra-1,3,5(10)-trien-7α-yl)-pentyl]-N-hexyl-benzamid | 621 |
| 91 | 4-Acetylamino-N-[5-(11β-fluor-3,17β-dihydroxy-estra-1,3,5(10)-trien-7α-yl)-pentyl]-N-octyl-benzamid | 649 |
| 92 | 4-Cyano-N-cyclohexylmethyl-N-[5-(11β-fluor-3,17β-dihydroxy-estra-1,3,5(10)-trien-7α-yl)-pentyl]-benzamid | 601 |
| 93 | N-Benzyl-4-cyano-N-[5-(11β-fluor-3,17β-dihydroxy-estra-1,3,5(10)-trien-7α-yl)-pentyl]-benzamid | 595 |
| 94 | 4-Cyano-N-[5-(11β-fluor-3,17β-dihydroxy-estra-1,3,5(10)-trien-7α-yl)-pentyl] -N-(2-methoxy-ethyl)-benzamid | 563 |

(fortgesetzt)

| Beispiel | Verbindung | MS (CI): $[M]^+$+1 [m/z] |
|---|---|---|
| 95 | 4-Cyano-N-[5-(11β-fluor-3,17β-dihydroxy-estra-1,3,5(10)-trien-7α-yl)-pentyl]-N-(3-phenyl-propyl)-benzamid | 623 |
| 96 | 4-Cyano-N-[5-(11β-fluor-3,17β-dihydroxy-estra-1,3,5(10)-trien-7α-yl)-pentyl]-N-hexyl-benzamid | 589 |
| 97 | 4-Cyano-N-[5-(11β-fluor-3,17β-dihydroxy-estra-1,3,5(10)-trien-7α-y])-pentyl]-N-octyl-benzamid | 617 |
| 98 | 3-Cyclohexyl-N-cyclohexylmethyl-N-[5-(11β-fluor-3,17β-dihydroxy-estra-1,3,5(10)-trien-7α-yl)-pentyl]-propionamid | 610 |
| 99 | N-Benzyl-3-cyclohexyl-N-[5-(11β-fluor-3,17β-dihydroxy-estra-1,3,5(10)-trien-7α-yl)-pentyl]-propionamid | 604 |
| 100 | 3-Cyclohexyl-N-[5-(11β-fluor-3,17β-dihydroxy-estra-1,3,5(10)-trien-7α-yl)-pentyl] -N-(2-methoxy-ethyl)-propionamid | 572 |
| 101 | 3-Cyclohexyl-N-[5-(11β-fluor-3,17β-dihydroxy-estra-1,3,5(10)-trien-7α-yl)-pentyl] -N-(3-phenyl-propyl)-propionamid | 632 |
| 102 | 3-Cyclohexyl-N-[5-(11β-fluor-3,17β-dihydroxy-estra-1,3,5(10)-trien-7α-yl)-pentyl]-N-hexyl-propionamid | 598 |
| 103 | 3-Cyclohexyl-N-[5-(1-1β-fluor-3,17β-dihydroxy-estra-1,3,5(10)-trien-7α-yl)-pentyl]-N-octyl-propionamid | 626 |
| 104 | N-Cyclohexylmethyl-N-[5-(11β-fluor-3,17β-dihydroxy-estra-1,3,5(10)-trien-7α-yl)-pentyl] -(3,4-dimethoxyphenyl)-acetamid | 650 |
| 105 | N-Benzyl-N-[5-(11β-fluor-3,17β-dihydroxy-estra-1,3,5(10)-trien-7α-yl)-pentyl] -(3,4-dimethoxyphenyl)-acetamid | 644 |
| 106 | N-[5-(11β-Fluor-3,17β-dihydroxy-estra-1,3,5(10)-trien-7α-yl)-pentyl]-N-(2-methoxy-ethyl) -(3,4-dimethoxyphenyl)-acetamid | 612 |
| 107 | N-[5-(11β-Fluor-3,17β-dihydroxy-estra-1,3,5(10)-trien-7α-yl)-pentyl]-(3,4-dimethoxyphenyl) -N-(3-phenyl-propyl)-acetamid | 672 |
| 108 | N-[5-(11β-Fluor-3,17β-dihydroxy-estra-1,3,5(10)-trien-7α-yl)-pentyl]-N-hexyl-(3,4-dimethoxyphenyl)-acetamid | 638 |
| 109 | N-[5-(11β-Fluor-3,17β-dihydroxy-estra-1,3,5(10)-trien-7α-yl)-pentyl]-(3,4-dimethoxyphenyl) -N-octyl-acetamid | 666 |
| 110 | N-Cyclohexylmethyl-4-ethoxy-N-[5-(11β-fluor-3,17β-dihydroxy-estra-1,3,5(10)-trien-7α-yl)-pentyl]-benzamid | 620 |
| 111 | N-Benzyl-4-ethoxy-N-[5-(11β-fluor-3,17β-dihydroxy-estra-1,3,5(10)-trien-7α-yl)-pentyl]-benzamid | 614 |
| 112 | 4-Ethoxy-N-[5-(11β-fluor-3,17β-dihydroxy-estra-1,3,5(10)-trien-7α-yl)-pentyl] -N-(2-methoxy-ethyl)-benzamid | 582 |
| 113 | 4-Ethoxy-N-[5-(11β-fluor-3,17β-dihydroxy-estra-1,3,5(10)-trien-7α-yl)-pentyl]-N-(3-phenyl-propyl)-benzamid | 642 |
| 114 | 4-Ethoxy-N-[5-(11β-fluor-3,17β-dihydroxy-estra-1,3,5(10)-trien-7α-yl)-pentyl]-N-hexyl-benzamid | 608 |
| 115 | 4-Ethoxy-N-[5-(11β-fluor-3,17β-dihydroxy-estra-1,3,5(10)-trien-7α-yl)-pentyl]-N-octyl-benzamid | 636 |

(fortgesetzt)

| Beispiel | Verbindung | MS (CI): $[M]^{+}$+1 [m/z] |
|---|---|---|
| 116 | N-Cyclohexylmethyl-N-[5-(11β-fluor-3,17β-dihydroxy-estra-1,3,5(10)-trien-7α-yl)-pentyl]-dodecanamid | 654 |
| 117 | N-Benzyl-N-[5-(11β-fluor-3,17β-dihydroxy-estra-1,3,5(10)-trien-7α-yl)-pentyl]-dodecanamid | 648 |
| 118 | N-[5-(11β-Fluor-3,17β-dihydroxy-estra-1,3,5(10)-trien-7α-yl)-pentyl]-N-(2-methoxy-ethyl)-dodecanamid | 616 |
| 119 | N-[5-(11β-Fluor-3,17β-dihydroxy-estra-1,3,5(10)-trien-7α-yl)-pentyl]-N-(3-phenyl-propyl)-dodecanamid | 676 |
| 120 | N-[5-(11β-F]uor-3,17β-dihydroxy-estra-1,3,5(10)-trien-7α-yl)-pentyl]-N-hexyl-dodecanamid | 642 |
| 121 | N-[5-(11β-Fluor-3,17β-dihydroxy-estra-1,3,5(10)-trien-7α-yl)-pentyl]-N-octyl-dodecanamid | 671 |
| 122 | N-Cyclohexylmethyl-N-[5-(11β-fluor-3,17β-dihydroxy-estra-1,3,5(10)-trien-7α-yl)-pentyl]-naphth-2-yl-acetamid | 640 |
| 123 | N-Benzyl-N-[5-(11β-fluor-3,17β-dihydroxy-estra-1,3,5(10)-trien-7α-yl)-pentyl]-naphth-2-yl-acetamid | 634 |
| 124 | N-[5-(11β-Fluor-3,17β-dihydroxy-estra-1,3,5(10)-trien-7α-yl)-pentyl]-N-(2-methoxy-ethyl)-naphth-2-yl-acetamid | 602 |
| 125 | N-[5-(11β-Fluor-3,17β-dihydroxy-estra-1,3,5(10)-trien-7α-yl)-pentyl]-naphth-2-yl-N-(3-phenyl-propyl)-acetamid | 662 |
| 126 | N-[5-(11β-Fluor-3,17β-dihydroxy-estra-1,3,5(10)-trien-7α-yl)-pentyl]-N-hexyl-naphth-2-yl-acetamid | 628 |
| 127 | N-[5-(11β-Fluor-3,17β-dihydroxy-estra-1,3,5(10)-trien-7α-yl)-pentyl]-naphth-2-yl-N-octyl-acetamid | 656 |
| 128 | N-Cyclohexylmethyl-N-[5-(11β-fluor-3,17β-dihydroxy-estra-1,3,5(10)-trien-7α-yl)-pentyl]-4-phenyl-butyramid | 618 |
| 129 | N-Benzyl-N-[5-(11β-fluor-3,17β-dihydroxy-estra-1,3,5(10)-trien-7α-yl)-pentyl]-4-phenyl-butyramid | 612 |
| 130 | N-[5-(11β-Fluor-3,17β-dihydroxy-estra-1,3,5(10)-trien-7α-yl)-pentyl]-N-(2-methoxy-ethyl)-4-phenyl-butyramid | 580 |
| 131 | N-[5-(11β-Fluor-3,17β-dihydroxy-estra-1,3,5(10)-trien-7α-yl)-pentyl]-4-phenyl-N-(3-phenyl-propyl)-butyramid | 640 |
| 132 | N-[5-(11β-Fluor-3,17β-dihydroxy-estra-1,3,5(10)-trien-7α-yl)-pentyl]-N-hexyl-4-phenyl-butyramid | 606 |
| 133 | N-[5-(11β-Fluor-3,17β-dihydroxy-estra-1,3,5(10)-trien-7α-yl)-pentyl]-N-octyl-4-phenyl-butyramid | 634 |
| 134 | N-Cyclohexylmethyl-N-[5-(11β-fluor-3,17β-dihydroxy-estra-1,3,5(10)-trien-7α-yl)-pentyl]-propionamid | 528 |
| 135 | N-Benzyl-N-[5-(11β-fluor-3,17β-dihydroxy-estra-1,3,5(10)-trien-7α-yl)-pentyl]-propionamid | 522 |
| 136 | N-[5-(11β-Fluor-3,17β-dihydroxy-estra-1,3,5(10)-trien-7α-yl)-pentyl]-N-(2-methoxy-ethyl)-propionamid | 490 |
| 137 | N-[5-(11β-Fluor-3,17β-dihydroxy-estra-1,3,5(10)-trien-7α-yl)-pentyl]-N-(3-phenyl-propyl)-propionamid | 550 |
| 138 | N-[5-(11β-Fluor-3,17β-dihydroxy-estra-1,3,5(10)-trien-7α-yl)-pentyl]-N-hexyl-propionamid | 516 |

(fortgesetzt)

| Beispiel | Verbindung | MS (CI): $[M]^{+}+1$ [m/z] |
|---|---|---|
| 139 | N-[5-(11β-Fluor-3,17β-dihydroxy-estra-1,3,5(10)-trien-7α-yl)-pentyl]-N-octyl-propionamid | 544 |
| 140 | 4-Biphenyl-N-cyclohexylmethyl-N-[5-(11β-fluor-3,17β-dihydroxy-estra-1,3,5(10)-trien-7α-yl)-pentyl]-acetamid | 666 |
| 141 | 4-Biphenyl-N-benzyl-N-[5-(11β-fluor-3,17β-dihydroxy-estra-1,3,5(10)-trien-7α-yl)-pentyl]-acetamid | 660 |
| 142 | 4-Biphenyl-N-[5-(11β-fluor-3,17β-dihydroxy-estra-1,3,5(10)-trien-7α-yl)-pentyl] -N-(2-methoxy-ethyl)-acetamid | 628 |
| 143 | 4-Biphenyl-N-[5-(11β-fluor-3,17β-dihydroxy-estra-1,3,5(10)-trien-7α-yl)-pentyl] -N-(3-phenyl-propyl)-acetamid | 688 |
| 144 | 4-Biphenyl-N-[5-(11β-fluor-3,17β-dihydroxy-estra-1,3,5(10)-trien-7α-yl)-pentyl]-N-hexyl-acetamid | 654 |
| 145 | 4-Biphenyl-N-[5-(11β-fluor-3,17β-dihydroxy-estra-1,3,5(10)-trien-7α-yl)-pentyl]-N-octyl-acetamid | 682 |
| 146 | 4-Acetylamino-N-cyclohexylmethyl-N-[6-(11β-fluor-3,17β-dihydroxy-estra-1,3,5(10)-tri en-7α-yl)-hexyl]-benzamid | 647 |
| 147 | 4-Acetylamino-N-benzyl-N-[6-(11β-fluor-3,17β-dihydroxy-estra-1,3,5(10)-trien-7α-yl)-hexyl]-benzamid | 641 |
| 148 | 4-Acetylamino-N-[6-(11β-fluor-3,17β-dihydroxy-estra-1,3,5(10)-trien-7α-yl)-hexyl] -N-(2-methoxy-ethyl)-benzamid | 609 |
| 149 | 4-Acetylamino-N-[6-(11β-fluor-3,17β-dihydroxy-estra-1,3,5(10)-trien-7α-yl)-hexyl] -N-(3-phenyl-propyl)-benzamid | 669 |
| 150 | 4-Acetylamino-N-[6-(11β-fluor-3,17β-dihydroxy-estra-1,3,5(10)-trien-7α-yl)-hexyl]-N-hexyl-benzamid | 635 |
| 151 | 4-Acetylamino-N-[6-(11β-fluor-3,17β-dihydroxy-estra-1,3,5(10)-trien-7α-yl)-hexyl]-N-octyl-benzamid | 663 |
| 152 | 4-Cyano-N-cyclohexylmethyl-N-[6-(11β-fluor-3,17β-dihydroxy-estra-1,3,5(10)-trien-7α-yl)-hexyl]-benzamid | 615 |
| 153 | N-Benzyl-4-cyano-N-[6-(11β-fluor-3,17β-dihydroxy-estra-1,3,5(10)-trien-7α-yl)-hexyl]-benzamid | 609 |
| 154 | 4-Cyano-N-[6-(11β-fluor-3,17β-dihydroxy-estra-1,3,5(10)-trien-7α-yl)-hexyl]-N-(2-methoxy-ethyl)-benzamid | 577 |
| 155 | 4-Cyano-N-[6-(11β-fluor-3,17β-dihydroxy-estra-1,3,5(10)-trien-7α-yl)-hexyl]-N-(3-phenyl-propyl)-benzamid | 637 |
| 156 | 4-Cyano-N-[6-(11β-fluor-3,17β-dihydroxy-estra-1,3,5(10)-trien-7α-yl)-hexyl]-N-hexyl-benzamid | 603 |
| 157 | 4-Cyano-N-[6-(11β-fluor-3,17β-dihydroxy-estra-1,3,5(10)-trien-7α-yl)-hexyl]-N-octyl-benzamid | 631 |
| 158 | 3-Cyclohexyl-N-cyclohexylmethyl-N-[6-(11β-fluor-3,17β-dihydroxy-estra-1,3,5(10)-trien-7α-yl)-hexyl]-propionamid | 624 |
| 159 | N-Benzyl-3-cyclohexyl-N-[6-(11β-floor-3,17β-dihydroxy-estra-1,3,5(10)-trien-7α-yl)-hexyl]-propionamid | 618 |
| 160 | 3-Cyclohexyl-N-[6-(11β-fluor-3,17β-dihydroxy-estra-1,3,5(10)-trien-7α-yl)-hexyl] -N-(2-methoxy-ethyl)-propionamid | 586 |

(fortgesetzt)

| Beispiel | Verbindung | MS (CI): $[M]^+$+1 [m/z] |
|---|---|---|
| 161 | 3-Cyclohexyl-N-[6-(11β-fluor-3,17β-dihydroxy-estra-1,3,5(10)-trien-7α-yl)-hexyl] -N-(3-phenyl-propyl)-propionamid | 646 |
| 162 | 3-Cyclohexyl-N-[6-(11β-fiuor-3,17β-dihydroxy-estra-1,3,5(10)-trien-7α-yl)-hexyl]-N-hexyl-propionamid | 612 |
| 163 | 3-Cyclohexyl-N-[6-(11β-fluor-3,17β-dihydroxy-estra-1,3,5(10)-trien-7α-yl)-hexyl]-N-octyl-propionamid | 640 |
| 164 | N-Cyclohexylmethyl-N-[6-(11β-fluor-3,17β-dihydroxy-estra-1,3,5(10)-trien-7α-yl)-hexyl] -(3,4-dimethoxyphenyl)-acetamid | 664 |
| 165 | N-Benzyl-N-[6-(11β-fluor-3,17β-dihydroxy-estra-1,3,5(10)-trien-7α-yl)-hexyl] -(3,4-dimethoxyphenyl)-acetamid | 658 |
| 166 | N-[6-(11β-Fluor-3,17β-dihydroxy-estra-1,3,5(10)-trien-7α-yl)-hexyl]-N-(2-methoxy-ethyl) -(3,4-dimethoxyphenyl)-acetamid | 626 |
| 167 | N-[6-(11β-Fluor-3,17β-dihydroxy-estra-1,3,5(10)-trien-7α-yl)-hexyl]-(3,4-dimethoxyphenyl) -N-(3-phenyl-propyl)-acetamid | 686 |
| 168 | N-[6-(11β-Fluor-3,17β-dihydroxy-estra-1,3,5(10)-trien-7α-yl)-hexyl]-N-hexyl-(3,4-dimethoxyphenyl)-acetamid | 652 |
| 169 | N-[6-(11β-Fluor-3,17β-dihydroxy-estra-1,3,5(10)-trien-7α-yl)-hexyl]-(3,4-dimethoxyphenyl) -N-octyl-acetamid | 680 |
| 170 | N-Cyclohexylmethyl-4-ethoxy-N-[6-(11β-fluor-3,17β-dihydroxy-estra-1,3,5(10)-trien-7α-yl)-hexyl]-benzamid | 634 |
| 171 | N-Benzyl-4-ethoxy-N-[6-(11β-fluor-3,17β-dihydroxy-estra-1,3,5(10)-trien-7α-yl)-hexyl]-benzamid | 628 |
| 172 | 4-Ethoxy-N-[6-(11β-fluor-3,17β-dihydroxy-estra-1,3,5(10)-trien-7α-yl)-hexyl]-N-(2-methoxy-ethyl)-benzamid | 596 |
| 173 | 4-Ethoxy-N-[6-(11β-fluor-3,17β-dihydroxy-estra-1,3,5(10)-trien-7α-yl)-hexyl]-N-(3-phenyl-propyl)-benzamid | 656 |
| 174 | 4-Ethoxy-N-[6-(11β-floor-3,17β-dihydroxy-estra-1,3,5(10)-trien-7α-yl)-hexyl]-N-hexyl-benzamid | 622 |
| 175 | 4-Ethoxy-N-[6-(11β-fluor-3,17β-dihydroxy-estra-1,3,5(10)-trien-7α-yl)-hexyl]-N-octyl-benzamid | 650 |
| 176 | N-Cyclohexylmethyl-N-[6-(11β-fluor-3,17β-dihydroxy-estra-1,3,5(10)-trien-7α-yl)-hexyl]-dodecanamid | 668 |
| 178 | N-Benzyl-N-[6-(11β-fluor-3,17β-dihydroxy-estra-1,3,5(10)-trien-7α-yl)-hexyl]-dodecanamid | 662 |
| 179 | N-[6-(11β-Fluor-3,17β-dihydroxy-estra-1,3,5(10)-trien-7α-yl)-hexyl]-N-(2-methoxy-ethyl)-dodecanamid | 630 |
| 180 | N-[6-(11β-Fluor-3,17β-dihydroxy-estra-1,3,5(10)-trien-7α-yl)-hexyl]-N-(3-phenyl-propyl)-dodecanamid | 690 |
| 181 | N-[6-(11β-Fluor-3,17β-dihydroxy-estra-1,3,5(10)-trien-7α-yl)-hexyl]-N-hexyl-dodecanamid | 656 |
| 182 | N-[6-(11β-Fluor-3,17β-dihydroxy-estra-1,3,5(10)-trien-7α-yl)-hexyl]-N-octyl-dodecanamid | 685 |
| 183 | N-Cyclohexylmethyl-N-[6-(11β-fluor-3,17β-dihydroxy-estra-1,3,5(10)-trien-7α-yl)-hexyl]-naphth-2-yl-acetamid | 654 |

(fortgesetzt)

| Beispiel | Verbindung | MS (CI): $[M]^{+}+1$ [m/z] |
|---|---|---|
| 184 | N-Benzyl-N-[6-(11β-fluor-3,17β-dihydroxy-estra-1,3,5(10)-trien-7α-yl)-hexyl]-naphth-2-yl-acetamid | 648 |
| 185 | N-[6-(11β-Fluor-3,17β-dihydroxy-estra-1,3,5(10)-trien-7α-yl)-hexyl]-N-(2-methoxy-ethyl)-naphth-2-yl-acetamid | 616 |
| 186 | N-[6-(11β-Fluor-3,17β-dihydroxy-estra-1,3,5(10)-trien-7α-yl)-hexyl]-naphth-2-yl-N-(3-phenyl-propyl)-acetamid | 676 |
| 187 | N-[6-(11β-Fluor-3,17β-dihydroxy-estra-1,3,5(10)-trien-7α-yl)-hexyl]-N-hexyl-naphth-2-yl-acetamid | 642 |
| 188 | N-[6-(11β-Fluor-3,17β-dihydroxy-estra-1,3,5(10)-trien-7α-yl)-hexyl]-naphth-2-yl-N-octyl-acetamid | 670 |
| 189 | N-Cyclohexylmethyl-N-[6-(11β-fluor-3,17β-dihydroxy-estra-1,3,5(10)-trien-7α-yl)-hexyl]-4-phenyl-butyramid | 632 |
| 190 | N-Benzyl-N-[6-(11β-fluor-3,17β-dihydroxy-estra-1,3,5(10)-trien-7α-yl)-hexyl]-4-phenyl-butyramid | 626 |
| 191 | N-[6-(11β-Fluor-3,17β-dihydroxy-estra-1,3,5(10)-trien-7α-yl)-hexyl]-N-(2-methoxy-ethyl)-4-phenyl-butyramid | 594 |
| 192 | N-[6-(11β-Fluor-3,17β-dihydroxy-estra-1,3,5(10)-trien-7α-yl)-hexyl]-4-phenyl-N-(3-phenyl-propyl)-butyramid | 654 |
| 193 | N-[6-(11β-Fluor-3,17β-dihydroxy-estra-1,3,5(10)-trien-7α-yl)-hexyl]-N-hexyl-4-phenyl-butyramid | 620 |
| 194 | N-[6-(11β-Fluor-3,17β-dihydroxy-estra-1,3,5(10)-trien-7α-yl)-hexyl]-N-octyl-4-phenyl-butyramid | 648 |
| 195 | N-Cyclohexylmethyl-N-[6-(11β-fluor-3,17β-dihydroxy-estra-1,3,5(10)-trien-7α-yl)-hexyl]-propionamid | 542 |
| 196 | N-Benzyl-N-[6-(11β-fluor-3,17β-dihydroxy-estra-1,3,5(10)-trien-7α-yl)-hexyl]-propionamid | 536 |
| 197 | N-[6-(11β-Fluor-3,17β-dihydroxy-estra-1,3,5(10)-trien-7α-yl)-hexyl]-N-(2-methoxy-ethyl)-propionamid | 504 |
| 198 | N-[6-(11β-Fluor-3,17β-dihydroxy-estra-1,3,5(10)-trien-7α-yl)-hexyl]-N-(3-phenyl-propyl)-propionamid | 564 |
| 199 | N-[6-(11β-Fluor-3,17β-dihydroxy-estra-1,3,5(10)-trien-7α-yl)-hexyl]-N-hexyl-propionamid | 530 |
| 200 | N-[6-(11β-Fluor-3,17β-dihydroxy-estra-1,3,5(10)-trien-7α-yl)-hexyl]-N-octyl-propionamid | 558 |
| 201 | 4-Biphenyl-N-cyclohexylmethyl-N-[6-(11β-fluor-3,17β-dihydroxy-estra-1,3,5(10)-trien-7α-yl)-hexyl]-acetamid | 680 |
| 202 | 4-Biphenyl-N-benzyl-N-[6-(11β-fluor-3,17β-dihydroxy-estra1,3,5(10)-trien-7α-yl)-hexyl]-acetamid | 674 |
| 203 | 4-Biphenyl-N-[6-(11β-fluor-3,17β-dihydroxy-estra-1,3,5(10)-trien-7α-yl)-hexyl] -N-(2-methoxy-ethyl)-acetamid | 642 |
| 204 | 4-Biphenyl-N-[6-(11β-fluor-3,17β-dihydroxy-estra-1,3,5(10)-trien-7α-yl)-hexyl]-N-(3-phenyl-propyl)-acetamid | 702 |
| 205 | 4-Biphenyl-N-[6-(11β-fluor-3,17β-dihydroxy-estra-1,3,5(10)-trien-7α-yl)-hexyl] -N-hexyl-acetamid | 668 |

(fortgesetzt)

| Beispiel | Verbindung | MS (CI): $[M]^{+}+1$ [m/z] |
|---|---|---|
| 206 | 4-Biphenyl-N-[6-(11β-fluor-3,17β-dihydroxy-estra-1,3,5(10)-trien-7α-yl)-hexyl]-N-octyl-acetamid | 696 |

**Beispiel 207**

**11β-Fluor-7α-{5-[methyl-(2-p-tolyl-ethyl)-amino]-pentyl}-estra-1,3,5(10)-trien-3,17β-diol**

660 mg 7α-(5-Brompentyl)-11β-fluor-estra-1,3,5(10)-trien-3,17β-diol werden in 10 ml N-Methylpyrrolidon gelöst, mit 1,2 g Methyl-(2-p-tolyl-ethyl)-amin versetzt und 3 Stunden bei 80 °C Badtemperatur gerührt. Zur Aufarbeitung wird der Ansatz auf gesättigte Natriumchloridlösung gegeben, mit Essigester extrahiert, über Natriumsulfat getrocknet und i. Vak. zur Trockne eingeengt. Säulenchromatographische Reinigung an Kieselgel mit einem Essigester-Methanol-Ammoniak-Gradienten und anschließende Kristallisation aus Essigester/Methylenchlorid/Hexan liefert 277 mg 11β-Fluor-7α-{5-[methyl-(2-p-tolylethyl)-amino]-pentyl}-estra-1,3,5(10)-trien-3,17β-diol vom Schmelzpunkt 142-143 °C.

**Herstellung der Ausgangsverbindungen:**

**a) 2-p-Tolyl-ethyltosylat**

2,8 ml 2-p-Tolyl-ethanol werden bei Eisbadkühlung in 40 ml Pyridin vorgelegt, mit 4,96 g p-Toluolsulfonylchlorid versetzt, 30 Minuten bei 0°C und 2 Stunden bei Raumtemperatur gerührt. Anschließend wird das Reaktionsgemisch in ca. 150g Eis/Wasser langsam eingerührt. Das ausgefallene Produkt wird abgesaugt, mit Wasser mehrfach nachgewaschen, in Methylenchlorid gelöst, über Magnesiumsulfat getrocknet und im Vakuum eingeengt. Man erhält 5,8 g 2-p-Tolyl-ethyltosylat, welches ohne weitere Aufreinigung in der nächsten Stufe eingesetzt wird.

**b) Methyl-(2-p-tolyl-ethyl)-amin**

In eine Lösung von 5,8 g 2-p-Tolyl-ethyltosylat in 15 ml abs. Tetrahydrofuran werden bei - 20 °C 9,2 g Methylamin kondensiert und über Nacht im Druckgefäß bei Raumtemperatur gerührt. Nachdem das Druckgefäß bei -20 °C geöffnet wurde, läßt man auf Raumtemperatur kommen, um überschüssiges Methylamin abdampfen zu lassen. Die Reaktionslösung wird in Ether aufgenommen, mit Wasser und gesättigter Natriumchloridlösung gewaschen, über Magnesiumsulfat getrocknet und i. Vak. zur Trockne eingeengt. Man erhält 5,39 g Methyl-(2-p-tolyl-ethyl)-amin, welches als Rohprodukt weiter umgesetzt wird.

**Beispiel 208**

**7α-(5-{[2-(4-Ethoxy-phenyl)-ethyl]-methyl-ammo}-pentyl)-11β-fluor-estra-1,3,5(10)-trien-3,17β-diol**

660 mg 7α-(5-Brompentyl)-11β-fluor-estra-1,3,5(10)-trien-3,17β-diol werden analog Beispiel 207 mit 1,06 g [2-(4-Ethoxy-phenyl)ethyl]-methylamin zu 7α-(5-{[2-(4-Ethoxyphenyl)-ethyl]-methyl-amino}-pentyl)-11β-fluor-estra-1,3,5(10)-trien-3,17β-diol vom Schmelzpunkt 94-95 °C umgesetzt. Die Ausbeute beträgt 464 mg.

**Herstellung der Ausgangsverbindungen:**

**a) 2-(4-Ethoxy-phenyl)-ethyltosylat**

3,32 g 2-(4-Ethoxy-phenyl)-ethanol werden analog Beispiel 207 a (Herstellung der Ausgangsverbindungen) zu 5,61 g 2-(4-Ethoxy-phenyl)-ethyltosylat als Rohprodukt umgesetzt.

**b) [2-(4-Ethoxy-phenyl)-ethyl]-methylamin**

5,61 g 2-(4-Ethoxy-phenyl)-ethyltosylat werden analog Beispiel 207 b (Herstellung der Ausgangsverbindungen) zu 4,14 g [2-(4-Ethoxy-phenyl)-ethyl]-methylamin als Rohprodukt umgesetzt.

**Beispiel 209**

**11β-Fluor-7α-{5-[methyl-(3-phenyl-propyl)-amino]-pentyl}-estra-1,3,5(10)-trien-3,17βdiol**

1,67 g 11β-Fluor-7α-[5-(methyl-amino)-pentyl]-estra-1,3,5(10)-trien-3,17β-diol wird in 60 ml Ethylmethylketon gelöst, nacheinander mit 1,64 g Kaliumcarbonat und 1,82 ml 3-Phenylpropylbromid versetzt und 2 Stunden unter Rückfluß erhitzt. Zur Aufarbeitung wird das Reaktionsgemisch mit Essigester verdünnt, mit Wasser und gesättigter Natriumchloridlösung gewaschen, über Magnesiumsulfat getrocknet und i. Vak. zur Trockne eingeengt. Säulenchromatographische Reinigung an Kieselgel mit einem Methylenchlorid-Methanol-Ammoniak-Gradienten und anschließende Kristallisation aus Essigester/Methylenchlorid/Hexan liefert 369 mg 11β-Fluor-7α-{5-[methyl-(3-phenylpropyl)-amino]-pentyl}-estra-1,3,5(10)-trien-3,17β-diol vom Schmelzpunkt123-124 °C.

**Beispiel 210**

**11β-Fluor-7α-{5-[methyl-(3-pyridin-3-yl-propyl)-amino]-pentyl}-estra-1,3,5(10)-trien-3,17β-diol**

779 mg 11β-Fluor-7α-[5-(methyl-amino)-pentyl]-estra-1,3,5(10)-trien-3,17β-diol werden analog Beispiel 209 mit 778 mg 3-(3-Chlor-propyl)-pyridin umgesetzt. Man erhält 207 mg 11β-Fluor-7α-{5-[methyl-(3-pyridin-3-yl-propyl)-amino]-pentyl}-estra-1,3,5(10)-trien-3,17β -diol als Schaum.

**Herstellung der Ausgangsverbindung:**

**3-(3-Chlor-propyl)-pyridin**

3,58 ml 3-(3-Hydroxy-propyl)-pyridin werden in 50 ml abs. Methylenchlorid vorgelegt und bei 0 °C tropfenweise mit einer Lösung von 4,4 ml Thionylchlorid in 50 ml abs. Methylenchlorid versetzt. Nach beendeter Zugabe wird 3 Stunden bei Raumtemperatur nachgerührt. Anschließend rührt man die Reaktionslösung vorsichtig in ein Gemisch aus 1N Natronlauge und Eis (1:1) ein, extrahiert mehrfach mit Methylenchlorid, wäscht mit gesättigter Natriumchloridlösung, trocknet über Natriumsulfat und engt i. Vak. ein. Man erhält 3,48 g 3-(3-Chlor-propyl)-pyridin, welches ohne weitere Aufreinigung in der nächsten Stufe eingesetzt wird.

**Beispiel 211**

**11β-Fluor-7α-{5-[methyl-(3-p-tolyl-propyl)-amino]-pentyl}-estra-1,3,5(10)-trien-3,17βdiol**

486 mg 11β-Fluor-7α-(5-iodpentyl)-estra-1,3,5(10)-trien-3,17β-diol werden in 10 ml N-Methylpyrrolidon gelöst, mit 0,71 g Methyl-(3-p-tolyl-propyl)-amin versetzt und 3 Stunden bei 90 °C Badtemperatur gerührt. Zur Aufarbeitung wird der Ansatz auf gesättigte Natriumchloridlösung gegeben. mit Essigester extrahiert, über Natriumsulfat getrocknet und i. Vak. zur Trockne eingeengt. Säulenchromatographische Reinigung an Kieselgel mit einem Essigester-Methanol-Ammoniak-Gradienten liefert 506 mg 11β-Fluor-7α-{5-[methyl-(3-p-tolyl-propyl)-amino)-pentyl}-estra-1,3,5(10)-trien-3,17β-diol als Schaum, $[\alpha]_D$ = +44,0° (c=0,5 in $CHCl_3$).

**Herstellung der Ausgangsverbindungen:**

**a) 3-p-Tolyl-propan-1-ol**

3,28 g 3-p-Tolylpropinsäure werden in 80 ml Ether gelöst und bei Raumtemperatur portionsweise mit dreimal 0,5 g Lithiumaluminiumhydrid versetzt. Nach jeder Zugabe wird für zirka 30 Minuten unter Rückfluß erhitzt. Zur Aufarbeitung wird das Reaktionsgemisch auf -5 °C gekühlt und tropfenweise mit 40 ml Wasser und 60 ml 1N Schwefelsäure versetzt. Anschließend wird mit Essigester verdünnt, mit gesättigter Natriumchloridlösung gewaschen, über Magnesiumsulfat getrocknet und i. Vak. zur Trockne eingeengt. Präparative Säulenchromatographie mit einem Hexan / tert. -Butyl-methylether-Gradienten liefert 2,8 g 3-p-Tolyl-propan-1-ol als klares Öl.

**b) 3-p-Tolyl-propyltosylat**

2,8 g 3-p-Tolyl-propan-1-ol werden in 50 ml Methylenchlorid gelöst und mit 3,14g 1,4-Diazabicyclo-2,2,2-octan versetzt. Bei Eisbadkühlung werden vorsichtig 4,62 g p-Toluolsulfonylchlorid hinzugefügt, 5 Minuten bei 0°C und dann bei Raumtemperatur gerührt. Zur Aufarbeitung wird das Reaktionsgemisch auf ca. 150g Eis gegeben und 30 Minuten

gerührt. Es wird dreimal mit Methylenchlorid extrahiert, mit Wasser gewaschen, über Magnesiumsulfat getrocknet und im Vakuum eingeengt. Man erhält 4,74 g 3-p-Tolyl-propyltosylat, welches ohne weitere Aufreinigung in der nächsten Stufe eingesetzt wird.

**c) Methyl-(3-p-tolyl-propyl)-amin**

In eine Lösung von 2,13 g 3-p-Tolyl-propyltosylat in 15 ml abs. Tetrahydrofuran werden bei - 20 °C 3,34 g Methylamin kondensiert und über Nacht im Druckgefäß bei Raumtemperatur gerührt. Nachdem das Druckgefäß bei -20 °C geöffnet wurde, läßt man auf Raumtemperatur kommen, um überschüssiges Methylamin abdampfen zu lassen. Die Reaktionslösung wird in Ether aufgenommen, mit Wasser und gesättigter Natriumchloridlösung gewaschen, über Magnesiumsulfat getrocknet und i. Vak. zur Trockne eingeengt. Man erhält 1,42 g Methyl-(3-p-tolyl-propyl)-amin, welches als Rohprodukt weiter umgesetzt wird.

**Beispiel 212**

**7α-(5-{[3-(4-Chlor-phenyl)-propyl]-methyl-amino}-pentyl)-11β-fluor-estra-1,3,5(10)-trien-3,17β-diol**

486 mg 11β-Fluor-7α-(5-iodpentyl)-estra-1,3,5(10)-trien-3,17β-diol werden analog zu Beispiel 211 mit 0.89 g [3-(4-Chlor-phenyl)-propyl]-methylamin zu 343 mg 7α-(5-{[3-(4-Chlor-phenyl)-propyl]-methyl-amino}-pentyl)-11β-fluor-estra-1,3,5(10)-trien-3,17β-diol, $[\alpha]_D$ = +42,8° (c=0,5 in $CHCl_3$), umgesetzt.

**Herstellung der Ausgangsverbindungen:**

**a) 3-(4-Chlor-phenyl)-propan-1-ol**

5,0 g 3-(4-Chlor-phenyl)-propionsäure werden analog Beispiel 211 a (Herstellung der Ausgangsverbindungen) zu 3,31 g 3-(4-Chlor-phenyl)-propan-1-ol umgesetzt.

**b) 3-(4-Chlor-phenyl)-propyltosylat**

2,8 g 3-(4-Chlor-phenyl)-propan-1-ol werden analog Beispiel 211 b (Herstellung der Ausgangsverbindungen) zu 5,11 g 3-(4-Chlor-phenyl)-propyltosylat als Rohprodukt umgesetzt.

**c) [3-(4-Chlor-phenyl)-propyl]-methylamin**

2,27 g 3-(4-Chlor-phenyl)-propyltosylat werden analog Beispiel 211 c (Herstellung der Ausgangsverbindungen) zu 1.78 g [3-(4-Chlor-phenyl)-propyl]-methylamin als Rohprodukt umgesetzt.

**Beispiel 213**

**7α-(5-{[3-(4-Ethoxy-phenyl)-propyl]-methyl-amino}-pentyl)-11β-fluor-estra-1,3,5(10)-trien-3,17β-diol**

486 mg 11β-Fluor-7α-(5-iodpentyl)-estra-1,3,5(10)-trien-3,17β-diol werden analog zu Beispiel 211 mit 0,78 g [3-(4-Ethoxy-phenyl)-propyl]-methylamin zu 345 mg 7α-(5-{[3-(4-Ethoxy-phenyl)-propyl]-methyl-amino}-pentyl)-11β-fluor-estra-1,3,5(10)-trien-3,17β-diol, $[\alpha]_D$ = +43,0° (c=0,5 in $CHCl_3$), umgesetzt.

**Herstellung der Ausgangsverbindungen:**

**a) 3-(4-Ethoxy-phenyl)-propan-1-ol**

5,0 g 3-(4-Ethoxy-phenyl)-propionsäure werden analog Beispiel 211 a (Herstellung der Ausgangsverbindungen) zu 5,08 g 3-(4-Ethoxy-phenyl)-propan-1-ol als Rohprodukt umgesetzt.

**b) 3-(4-Ethoxy-phenyl)-propyltosylat**

5,08 g 3-(4-Ethoxy-phenyl)-propan-1-ol werden analog Beispiel 211 b (Herstellung der Ausgangsverbindungen) zu 9,7 g 3-(4-Ethoxy-phenyl)-propyltosylat als Rohprodukt umgesetzt.

**c) [3-(4-Ethoxy-phenyl)-propyl]-methylamin**

2,34 g 3-(4-Ethoxy-phenyl)-propyltosylat werden analog Beispiel 211 c (Herstellung der Ausgangsverbindungen) zu 1,55 g [3-(4-Ethoxy-phenyl)-propyl]-methylamin als Rohprodukt umgesetzt.

**Beispiel 214**

**11β-Fluor-7α-{5-[methyl-(4-methyl-pentyl)-amino]-pentyl}-estra-1,3,5(10)-trien-3,17β-diol**

486 mg 11β-Fluor-7α-(5-iodpentyl)-estra-1,3,5(10)-trien-3,17β-diol werden analog zu Beispiel 211 mit 518 mg Methyl-(4-methyl-pentyl)-amin zu 263 mg 11β-Fluor-7α-{5-[methyl-(4-methyl-pentyl)-amino]-pentyl}-estra-1,3,5(10)-trien-3,17β-diol vom Schmelzpunkt 166 °C umgesetzt.

**Herstellung der Ausgangsverbindungen:**

**a) 4-Methyl-pentyltosylat**

6,28 ml g 4-Methyl-pentan-1-ol werden analog Beispiel 211 b (Herstellung der Ausgangsverbindungen) zu 8,43 g 4-Methyl-pentyltosylat umgesetzt.

**b) Methyl-(4-methyl-pentyl)-amin**

1,65 g 4-Methyl-pentyltosylat werden analog Beispiel 211 c (Herstellung der Ausgangsverbindungen) zu 518 mg Methyl-(4-methyl-pentyl)-amin als Rohprodukt umgesetzt.

**Patentansprüche**

1. 11β-Halogen-7α-substituierte-Estratriene der allgemeinen Formel I

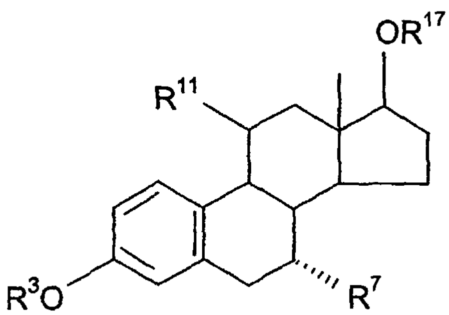

(I)

worin

$R^3$ ein Wasserstoffatom, einen Kohlenwasserstoffrest mit bis zu 8 Kohlenstoffatomen oder einen Rest der Teilformel $R^{3'}$—C(O)—, worin $R^{3'}$ ein Wasserstoffatom oder ein Kohlenwasserstoffrest mit bis zu 8 Kohlenstoffatomen oder ein Phenylrest ist, bedeutet,
$R^7$ einen Rest der Formel -A-B-Z-$R^{20}$ bedeutet,
worin

A fiir eine direkte Bindung oder einen Benzylidenrest, wobei die Methylengruppe an das 7-Kohlenstoffatom des Steroids gebunden ist, oder einen Phenylenrest,
B für eine gerad- oder verzweigtkettige Alkylen-, Alkenylen- oder Alkinylengruppe mit 3 bis 14 Kohlenstofatomen,

stehen, und

Z für-$NR^{21}$- und $R^{21}$ für eine $C_1$-$C_3$-Alkylgruppe stehen,
wobei dann $R^{20}$

ein Wasserstoffatom,
eine gerad- oder verzweigtkettige Alkyl-, Alkenyl- oder Alkinylgruppe mit bis zu 10 Kohlenstofatomen, oder eine der Gruppierungen
-D-$C_nF_{2n+1}$, wobei D eine gerad- oder verzweigtkettige Alkylen-, Alkenylen- oder Alkinylengruppe mit bis zu 8 Kohlenstofatomen und n eine ganze Zahl von 1 bis 8 ist,
-L-CH=CF-$C_pF_{2p+1}$, wobei L eine gerad- oder verzweigtkettige Alkylen-, Alkenylen- oder Alkinylengruppe mit 2 bis 7 Kohlenstofatomen und p eine ganze Zahl von 2 bis 7 ist,
-D-O-$(CH_2)_q$-Aryl, wobei D die bereits angegebenen Bedeutung hat, q 0, 1, 2 oder 3 ist und Aryl für einen gegebenenfalls ein- oder zweifach substituierten Phenyl-, 1- oder 2-Naphthyl- oder einen Heteroarylrest steht,
-D-O-$(CH_2)_r$-$C_nF_{2n+1}$, wobei D und n die bereits angegebenen Bedeutungen haben und r für eine ganze Zahl von 1 bis 5 steht,
bedeutet, oder

$R^{20}$ und $R^{21}$ mit dem Stickstoffatom, an das sie gebunden sind, einen gesättigten oder ungesättigten Heterocyclus mit 5 oder 6 Kettengliedern, der gegebenenfalls ein oder zwei weitere Heteroatome, ausgewählt aus Stickstoff, Sauerstoff und Schwefel, enthält und gegebenenfalls substituiert ist,
bilden, oder

Z fiir -$SO_x$- und x für 0, 1 oder 2 stehen,
wobei $R^{20}$ dann

eine gerad- oder verzweigtkettige Alkyl-, Alkenyl- oder Alkinylgruppe mit bis zu 10 Kohlenstofatomen, oder eine der Gruppierungen
-D-$C_nF_{2n+1}$, wobei D eine gerad- oder verzweigtkettige Alkylen-, Alkenylen- oder Alkinylengruppe mit bis zu 8 Kohlenstofatomen und n eine ganze Zahl von 1 bis 8 ist,
-L-CH=CF-$C_pF_{2p+1}$, wobei L eine gerad- oder verzweigtkettige Alkylen-, Alkenylen- oder Alkinylengruppe mit 2 bis 7 Kohlenstofatomen und p eine ganze Zahl von 2 bis 7 ist,
-D-O-$(CH_2)_q$-Aryl, wobei D die bereits angegebenen Bedeutung hat, q 0, 1, 2 oder 3 ist und Aryl für einen gegebenenfalls ein- oder zweifach substituierten Phenyl-, 1- oder 2-Naphthyl- oder einen Heteroarylrest steht,
-D-O-$(CH_2)_r$-$C_nF_{2n+1}$, wobei D und n die bereits angegebenen Bedeutungen haben und r fiir eine ganze Zahl von 1 bis 5 steht,
bedeutet, oder

Z für -$NR^{31}$- steht,
wobei dann $R^{20}$ ein gerad- oder verzweigtkettiger Alkyl-, Alkenyl- oder Alkinylrest mit bis zu 14 Kohlenstoffatomen, der durch ein bis drei Heteroatome -O- und -S- und Gruppierungen -$NR^{32}$-, worin $R^{32}$ ein Wasserstoffatom oder ein $C_1$-$C_3$-Alkylrest ist, unterbrochen und/oder teilweise fluoriert sein kann, ein gegebenenfalls ein- oder zweifach substituierter Aryl- oder Heteroarylrest, ein gegebenenfalls ein- oder zweifach substituierter $C_3$-$C_{10}$-Cycloalkylrest, ein gegebenenfalls ein- oder zweifach substituierter $C_4$-$C_{15}$-Cycloalkylalkylrest, ein gegebenenfalls ein- oder zweifach substituierter $C_7$-$C_{20}$-Aralkylrest, ein gegebenenfalls ein- oder zweifach substituierter Heteroaryl-$C_1$-$C_6$-alkylrest oder ein gegebenenfalls substituerter Aminoalkylrest, ist, und
$R^{31}$ ein Rest der Formel -C(O)$R^{33}$ oder -$CH_2$-$R^{33}$ ist,
wobei dann $R^{33}$ ein gerad- oder verzweigtkettiger Alkyl-, Alkenyl- oder Alkinylrest mit bis zu 14 Kohlenstoffatomen, der durch ein bis drei Heteroatome -O- und -S- und Gruppierungen -$NR^{32}$-, worin $R^{32}$ ein Wasserstoffatom oder ein $C_1$-$C_3$-Alkylrest ist, unterbrochen und/oder teilweise fluoriert sein kann, ein gegebenenfalls ein- oder zweifach substituierter Aryl- oder Heteroarylrest, ein gegebenenfalls ein- oder zweifach substituierter $C_3$-$C_{10}$-Cycloalkylrest, ein gegebenenfalls ein- oder zweifach substituierter $C_4$-$C_{15}$-Cycloalkylalkylrest, ein gegebenenfalls ein- oder zweifach substituierter $C_7$-$C_{20}$-Aralkylrest, ein gegebenenfalls ein- oder zweifach substituierter Heteroaryl-$C_1$-$C_6$-alkylrest, ein gegebenenfalls substituerter Aminoalkylrest oder ein Biphenylenrest ist,
ausgenommen der Verbindungen
11 β-Fluor-7α-{5-[N-methyl-N-3-(4,4,5,5,5-pentafluorpentylthio)-propylamino]-pentyl}estra-1,3,5(10)-trien-3,17β-diol

11β-Fluor-7α-{5-[2-(4,4,5,5,5-pentafluorpentylthiomethyl)-pyrrolidin-1-yl]-pentyl}-estra-1,3,5(10)-trien-3,17β-diol

11 β-Fluor-7α-{6-[N-methyl-N-3-(4,4,5,5,5-pentafluorpentylthio)-propylamino]-hexyl} -estral,3,5(10)-trien-3,17β-diol

11β-Fluor-7α-{5-[(2S)-2-(4-trifluormethylphenylthiomethyl)-pyrrolidin-1-yl]-pentyl}-estra-1,3,5(10)-trien-3,17β-diol

11β-Fluor-7α-{5-[(2S)-2-(4,4,5,5,5-pentafluorpentylthiomethyl)-pyrrolidin-1-yl]-pentyl}estra-1,3,5(10)-trien-3,17β-diol

11β-Fluor-7α-{5-[(2S)-2-(4,4,5,5,5-pentafluorpentansulfinylmethyl)-pyrrolidin-1-yl]-pentyl}estra-1,3,5(10)-trien-3,17β-diol

11 β-Fluor-7α-{5-[(2S)-2-(4,4,5,5,5-pentafluorpentansulfonylmethyl)-pyrrolidin-1-yl]-pentyl}-estra-1,3,5(10)-trien-3,17β-diol

11β-Fluor-7α-{5-[N-methyl-N-3-(4,4,5,5,5-pentafluorpentansulfinylmethyl)-propylamino]-pentyl}-estra-1,3,5 (10)-trien-3,17β-diol

11β-Fluor-7α-{5-[N-methyl-N-3-(4,4,5,5,5-pentafluorpentansulfonylmethyl)-propylamino]-pentyl}-estra-1,3,5 (10)-trien-3,17β-diol

11β-Fluor-7α-{5-[N-methyl-N-2-(4,4,5,5,5-pentfluorpentansulfonyl)-ethylamino]-pentyl}-estra-1,3,5(10)-trien-3,17β-diol

11β-Fluor-7α-{6-[N-methyl-N-3(4,4,5,5,5-pentafluorpentansulfinyl)propylamino]-hexyl}-estra-1,3,5(10)-trien-3,17-diol

$R^{11}$ ein Fluor- oder Chloratom,

$R^{17}$ ein Wasserstoffatom oder einen Rest der Teilformel $R^{17'}$—C(O)—, worin $R^{17'}$ ein Wasserstoffatom oder ein Kohlenwasserstoffrest mit bis zu 8 Kohlenstoffatomen ist.

**2.** 11β-Halogen-7α-substituierte-Estratriene nach Anspruch 1, worin $R^3$ ein Wasserstoffatom ist.

**3.** 11β-Halogen-7α-substituierte-Estratriene nach Anspruch 1, worin $R^3$ eín Benzoylrest ist.

**4.** 11β-Halogen-7α-substituierte-Estratriene nach Anspruch 1, worin $R^{11}$ eín Fluoratom ist.

**5.** 11β-Halogen-7α-substituierte-Estratriene nach Anspruch 1, worin $R^{17}$ eín Wasserstoffatom ist

**6.** 11β-Halogen-7α-substituierte-Estratriene nach Anspruch 1, worin A eine direkte Bindung ist.

**7.** 11β-Halogen-7α-substituierte-Estratriene nach Anspruch 6, worin für $R^7$ ein Rest der Formel $-(CH_2)_s$-Z-$R^{20}$, wobei
s eine ganze Zahl von 3 bis 8 ist,
Z für -$NR^{21}$ und $R^{21}$ für eine $C_1$-$C_3$-Alkylgruppe steht,
worin $R^{20}$
ein Wasserstoffatom,
eine $C_1$-$C_9$-Alkylgruppe, oder
eine der Gruppierungen
$-(CH_2)_m-C_nF_{2n+1}$, wobei m und n unabhängig voneinander jeweils eine ganze Zahl von 1 bis 8 ist,
$-(CH_2)_0-CH=CF-C_pF_{2p+1}$, wobei o 1, 2 oder 3 und p eine ganze Zahl von 2 bis 7 ist,
$-(CH_2)_m-O-(CH_2)_q$-Aryl, wobei m die bereits angegebenen Bedeutung hat, q 0, 1, 2 oder 3 ist und Aryl für einen gegebenenfalls ein- oder zweifach substituierten Phenyl- oder Heteroarylrest steht,
$-(CH_2)_m-O-(CH_2)_r-C_nF_{2n+1}$, wobei m und n die bereits angegebenen Bedeutungen haben und r für eine ganze Zahl von 1 bis 5 steht,
bedeutet;
steht.

**8.** 11β-Halogen-7α-substituierte-Estratriene nach Anspruch 6, worin für $R^7$
ein Rest der Formel $-(CH_2)_s$-Z-$R^{20}$,
wobei
s eine ganze Zahl von 3 bis 8 ist,
Z für -$NR^{21}$ und $R^{21}$ für eine $C_1$-$C_3$-Alkylgruppe steht,
worin $R^{20}$ und $R^{21}$
mit dem Stickstoffatom, an das sie gebunden sind, einen gesättigten oder ungesättigten Heterocyclus mit 5 oder

6 Kettengliedern bilden, der gegebenenfalls ein oder zwei weitere Heteroatome, ausgewählt aus Stickstoff, Sauerstoff und Schwefel, enthält und gegebenenfalls substituiert ist,
bedeutet,
steht.

**9.** 11β-Halogen-7α-substituierte-Estratriene nach Anspruch 6, worin für $R^7$
ein Rest der Formel $-(CH_2)_s-Z-R^{20}$,
wobei
s eine ganze Zahl von 3 bis 8 ist,
Z für $-SO_x-$ und x für 0, 1 oder 2 steht,
wobei $R^{20}$
$-(CH_2)_m-O-(CH_2)_r-C_nF_{2n+1}$, wobei m und n die bereits angegebenen Bedeutungen haben und r für eine ganze Zahl von 1 bis 5 steht,
bedeutet,
steht.

**10.** 11β-Halogen-7α-substituierte-Estratriene nach Anspruch 1, worin $R^7$ aus der Gruppe der folgenden Seitenketten ausgewählt ist

$-(CH_2)_5N(CH_3)(CH_2)_3C_2F_5$
$-(CH_2)_5N(CH_3)(CH_2)_6C_2F_5$
$-(CH_2)_5N(CH_3)(CH_2)_7C_2F_5$
$-(CH_2)_5N(CH_3)(CH_2)_8C_2F_5$
$-(CH_2)_6N(CH_3)(CH_2)_6C_2F_5$
$-(CH_2)_6N(CH_3)(CH_2)_7C_2F_5$
$-(CH_2)_6N(CH_3)(CH_2)_8C_2F_5$
$-(CH_2)_5N(CH_3)(CH_2)_2C_4F_9$
$-(CH_2)_5N(CH_3)(CH_2)_3C_6F_{13}$
$-(CH_2)_5N(CH_3)(CH_2)_3C_8F_{17}$
$-(CH_2)_5N(CH_3)(CH_2)_6C_4F_9$
$-(CH_2)_5N(CH_3)(CH_2)_6C_6F_{13}$
$-(CH_2)_5N(CH_3)(CH_2)_6C_8F_{17}$
$-(CH_2)_5N(CH_3)H$
$-(CH_2)_5N(CH_3)(CH_2)_9H$
$-(CH_2)_5N(CH_3)CH_2CH=CF-C_2F_5$
$-(CH_2)_5N(CH_3)CH_2CH=CF-C_3F_7$
$-(CH_2)_5N(CH_3)CH_2CH=CF-C_5F_{11}$
$-(CH_2)_5N(CH_3)CH_2CH=CF-C_7F_{15}$
$-(CH_2)_5$-1-Pyrrolidinyl
$-(CH_2)_5N(CH_3)(CH_2)_3$OPhenyl
$-(CH_2)_5N(CH_3)(CH_2)_3$OBenzyl
$-(CH_2)_5N(CH_3)(CH_2)_3O(CH_2)_3C_2F_5$
$-(CH_2)_9S(CH_2)_3C_2F_5$
$-(CH_2)_9SO(CH_2)_3C_2F_5$
$-(CH_2)_9SO_2(CH_2)_3C_2F_5$
$(CH_2)_5N(CH_3)(CH_2)_3CH(CH_3)_2$
$-(CH_2)_5N(CH_3)(CH_2)_3$-Pyridyl
$-(CH_2)_5N(CH_3)(CH_2)_3$-Phenyl
$-(CH_2)_5N(CH_3)(CH_2)_2$-p-Tolyl
$-(CH_2)_5N(CH_3)(CH_2)_2$-p-Ethoxyphenyl
$-(CH_2)_5N(CH_3)(CH_2)_3$-p-Tolyl
$-(CH_2)_5N(CH_3)(CH_2)_3$-p-Chlorphenyl
$-(CH_2)_5N(CH_3)(CH_2)_3-O-CH_2$-Phenyl
$-(CH_2)_5N(CH_3)(CH_2)_2$-O-p-Br-Phenyl
$-(CH_2)_5N(CH_3)(CH_2)_2-O-p-CF_3$-Phenyl

**11.** Verbindungen der allgemeinen Formel I, nämlich

11β-Fluor-7α-{5-[methyl-(8,8,9,9,9-pentafluor-nonyl)-amino]-pentyl}-estra-1,3,5(10)-trien-3,17β-diol
11β-Fluor-7α-{5-[methyl-nonyl-amino]-pentyl}-estra-1,3,5(10)-trien-3,17β-diol
11 β-Fluor-7α-{5-[methyl-(9,9,10,10,10-pentafluor-decyl)-amino]-pentyl}-estra-1,3,5(10)-trien-3,17β-diol
11 β-Fluor-7α-{6-[methyl-(8,8,9,9,9-pentafluor-nonyl)-amino]-hexyl} -estra-1,3,5(10)-trien-3,17β-diol
11 β-Fluor-7α-{6-[methyl-(9,9,10,10,10-pentafluor-decyl)-amino]-hexyl}-estra-1,3,5(10)-trien-3,17β-diol
11β-Fluor-7α-(5-(methyl-amino)-pentyl)-estra-1,3,5(10)-trien-3,17β-diol
11 β-Fluor-7α-(5-pyrrolidin-1-yl-pentyl)-estra-1,3,5(10)-trien-3,17β-diol
11β-Fluor-7α-{5-[methyl-(4,4,5,5,5-pentafluor-pentyl)-amino]-pentyl}-estra-1,3,5(10)-trien-3,17β-diol
11β-Fluor-7α-{5-[methyl-(4,4,5,5,6,6,7,7,8,8,9,9,9-tridecafluor-nonyl)-amino]-pentyl}-estra-1,3,5(10)-trien-3,17β-diol
11β-Fluor-7α-{5-[(4,4,5,5,6,6,7,7,8,8,9,9,10,10,11,11,11-heptadecafluor-undecyl)-methylamino]-pentyl}-estra-1,3,5(10)-trien-3,17β-diol
11 β-Fluor-7α-{5-[methyl-(3,3,4,4,5,5,6,6,6-nonafluor-hexyl)-amino]-pentyl} -estra-1,3,5(10)-trien-3,17β-diol
11β-Fluor-7α-{5-[methyl-(7,7,8,8,8-pentafluor-octyl)-amino]-pentyl}-estra-1,3,5(10)-trien-3,17β-diol
11 β-Fluor-7α-{6-[methyl-(7,7,8,8,8-pentafluor-octyl)-amino]-hexyl} -estra-1,3,5(10)-trien-3,17β-diol
11β-Fluor-7α-{5-[methyl-(7,7,8,8,9,9,10,10,10-nonafluor-decyl)-amino]-pentyl} -estra-1,3,5(10)-trien-3,17β-diol
11β-Fluor-7α-{5-[methyl-(7,7,8,8,9,9,10,10,11,11,12,12,12-tridecafluor-dodecyl)-amino]-pentyl}-estra-1,3,5(10)-trien-3,17β-diol
11β-Fluor-7α-{5-[(7,7,8,8,9,9,10,10,11,11,12,12,13,13,14,14,14-heptadecafluor-tetradecyl)-methyl-amino]-pentyl}-estra-1,3,5(10)-trien-3,17β-diol
11β-Fluor-7α-{5-[(3,4,4,5,5,5-hexafluor-pent-2-enyl)-methyl-amino]-pentyl} -estra-1,3,5(10)-trien-3,17β-diol
7α-{5-[(3,4,4,5,5,6,6,7,7,8,8,8-Dodecafluor-oct-2-enyl)-methyl-amino]-pentyl}-11β-fluorestra-1,3,5(10)-trien-3,17β-diol
11β-Fluor-7α-{ 5-[(3,4,4,5,5,6,6,7,7,8,8,9,9,10,10,10-hexadecafluor-dec-2-enyl)-methylamino]-pentyl) }-esrra-1,3,5(10)-trien-3,17β-diol
11 β-Fluor-7α-{5-[methyl-(3-phenoxy-propyl)-amino]-pentyl} -estra-1,3,5(10)-trien-3,17β-diol
7α-{5-[(3-Benzyloxy-propyl)-methyl-amino]-pentyl}-11β-fluor-estra-1,3,5(10)-trien-3,17βdiol
11β-Fluor-7α-{5-[N-methyl-N-3-(4,4,5,5,5-pentafluorpentyloxy)-propylamino]-pentyl}-estra-1,3,5(10)-trien-3,17β-diol
11β-Fluor-7α-[9-(4,4,5,5,5-pentafluorpentylsulfinvl)-nonyl]-estra-1,3,5(10)-trien-3,17β-diol N-[4-(11β-Fluor-3,17-dihydroxy-estra-1,3,5(10)-trien-7α-yl)-butyl]-N-pentyl-benzamid
4-Acetylamino-N-cyclohexylmethyl-N-[4-(11β-fluor-3,17β-dihydroxy-estra-1,3,5(10)-trien-7α-yl)-butyl]-benzamid
4-Acetylamino-N-benzyl-N-[4-(11β-fluor-3,17β-dihydroxy-estra-1,3,5(10)-trien-7α-yl)-butyl]-benzamid
4-Acetylamino-N-[4-(11β-fluor-3,17β-dihydroxy-estra-1,3,5(10)-trien-7α-yl)-butyl]-N-(2-methoxy-ethyl)-benzamid
4-Acetylamino-N-[4-(11β-fluor-3,17β-dihydroxy-estra-1,3,5(10)-trien-7α-yl)-butyl]-N-(3-phenyl-propyl)-benzamid
4-Acetylamino-N-[4-(11β-fluor-3,17β-dihydroxy-estra-1,3,5(10)-trien-7α-yl)-butyl]-N-hexylbenzamid
4-Acetylamino-N-[4-(11β-fluor-3,17β-dihydroxy-estra-1,3,5(10)-trien-7α-yl)-butyl]-N-octylbenzamid
4-Cyano-N-cyclohexylmethyl-N-[4-(11β-fluor-3,17β-dihydroxy-estra-1,3,5(10)-trien-7α-yl)-butyl]-benzamid
N-Benzyl-4-cyano-N-[4-(11β-fluor-3,17β-dihydroxy-estra-1,3,5(10)-trien-7α-yl)-butyl]-benzamid
4-Cyano-N-[4-(11β-fluor-3,17β-dihydroxy-estra-1,3,5(10)-trien-7α-yl)-butyl]-N-(2-methoxyethyl)-benzamid
4-Cyano-N-[4-(11β-fluor-3,17β-dihydroxy-estra-1,3,5(10)-trien-7α-yl)-butyl]-N-(3-phenylpropyl)-benzamid
4-Cyano-N-[4-(11β-fluor-3,17β-dihydroxy-estra-1,3,5(10)-trim-7α-yl)-butyl]-N-hexylbenzamid
4-Cyano-N-[4-(11β-fluor-3,17β-dihydroxy-estra-1,3,5(10)-trien-7α-yl)-butyl]-N-octylbenzamid
3-Cyclohexyl-N-cyclohexylmethyl-N-[4-(11β-fluor-3,17β-dihydroxy-estra-1,3,5(10)-trien-7α-yl)-butyl]-propionamid
N-Benzyl-3-cyclohexyl-N-[4-(11β-fluor-3,17β-dihydroxy-estra-1,3,5(10)-trien-7α-yl)-butyl]-propionamid
3-Cyclobexyl-N-[4-(11β-fluor-3,17β-dihydroxy-estra-1,3,5(10)-trien-7α-yl)-butyl]-N-(2-methoxy-ethyl)-propionamid
3-Cyclohexyl-N-[4-(11β-fluor-3,17β-dihydroxy-estra-1,3,5(10)-trien-7α-yl)-butyl]-N-(3-phenyl-propyl)-propionamid
3-Cyclohexyl-N-[4-(11β-fluor-3,17β-dihydroxy-estra-1,3,5(10)-trien-7α-yl)-butyl]-N-hexylpropionamid
3-Cyclohexyl-N-[4-(11β-fluor-3,17β-dihydroxy-estra-1,3,5(10)-trien-7α-yl)-butyl]-N-octylpropionamid
N-Cyclohexylmethyl-N-[4-(11β-fluor-3,17β-dihydroxy-estra-1,3,5(10)-trien-7α-yl)-butyl]-(3,4-dimethoxyphenyl)-acetamid

N-Benzyl-N-[4-(11β-fluor-3,17β-dihydroxy-estra-1,3,5(10)-trien-7α-yl)-butyl]-(3,4-dimethoxyphenyl)-acetamid
N-[4-(11β-Fluor-3,17β-dihydroxy-estra-1,3,5(10)-trien-7α-yl)-butyl]-N-(2-methoxy-ethyl)-(3,4-dimethoxyphenyl)-acetamid
N-[4-(11β-Fluor-3,17β-dihydroxy-estra-1,3,5(10)-trien-7α-yl)-butyl]-(3,4-dimethoxyphenyl)-N-(3-phenyl-propyl)-acetamid
N-[4-(11β-Fluor-3,17β-dihydroxy-estra-1,3,5(10)-trien-7α-yl)-butyl]-N-hexyl-(3,4-dimethoxyphenyl)-acetamid
N-[4-(11β-Fluor-3,17β-dihydroxy-estra-1,3,5(10)-trien-7α-yl)-butyl]-(3,4-dimethoxyphenyl)-N-octyl-acetamid
N-Cyclohexylmethyl-4-ethoxy-N-[4-(11β-fluor-3,17β-dihydroxy-estra-1,3,5(10)-trien-7α-yl)-butyl]-benzamid
N-Benzyl-4-ethoxy-N-[4-(11β-fluor-3,17β-dihydroxy-estra-1,3,5(10)-trien-7α-yl)-butyl]-benzamid
4-Ethoxy-N-[4-(11β-fluor-3,17β-dihydroxy-estra-1,3,5(10)-trien-7α-yl)-butyl]-N-(2-methoxy-ethyl)-benzamid
4-Ethoxy-N-[4-(11β-fluor-3,17β-dihydroxy-estra-1,3,5(10)-trien-7α-yl)-butyl]-N-(3-phenylpropyl)-benzamid
4-Ethoxy-N-[4-(11β-fluor-3,17β-dihydroxy-estra-1,3,5(10)-trien-7α-yl)-butyl]-N-hexylbenzamid
4-Ethoxy-N-[4-(11β-fluor-3,17β-dihydroxy-estra-1,3,5(10)-trien-7α-yl)-butyl]-N-octylbenzamid
N-Cyclohexylmethyl-N-[4-(11β-fluor-3,17β-dihydroxy-estra-1,3,5(10)-trien-7α-yl)-butyl]-dodecanamid
N-Benzyl-N-[4-(11β-fluor-3,17β-dihydroxy-estra-1,3,5(10)-trien-7α-yl)-butyl]-dodecanamid
N-[4-(11β-Fluor-3,17β-dihydroxy-estra-1,3,5(10)-trien-7α-yl)-butyl]-N-(2-methoxy-ethyl)-dodecanamid
N-[4-(11β-Fluor-3,17β-dihydroxy-estra-1,3,5(10)-trien-7α-yl)-butyl]-N-(3-phenyl-propyl)-dodecanamid
N-[4-(11β-Fluor-3,17β-dihydroxy-estra-1,3,5(10)-trien-7α-yl)-butyl]-N-hexyl-dodecanamid
N-[4-(11β-Fluor-3,17β-dihydroxy-estra-1,3,5(10)-trien-7α-yl)-butyl]-N-octyl-dodecanamid
N-Cyclohexylmethyl-N-[4-(11β-fluor-3,17β-dihydroxy-estra-1,3,5(10)-trien-7α-yl)-butyl]-naphth-2-yl-acetamid
N-Benzyl-N-[4-(11β-fluor-3,17β-dihydroxy-estra-1,3,5(10)-trien-7α-yl)-butyl]-naphth-2-ylacetamid
N-[4-(11β-Fluor-3,17β-dihydroxy-estra-1,3,5(10)-trien-7α-yl)-butyl]-N-(2-methoxy-ethyl)-naphth-2-yl-acetamid
N-[4-(11β-Fluor-3,17β-dihydroxy-estra-1,3,5(10)-trien-7α-yl)-butyl]-naphth-2-yl-N-(3-phenyl-propyl)-acetamid
N-[4-(11β-Fluor-3,17β-dihydroxy-estra-1,3,5(10)-trien-7α-yl)-butyl]-N-hexyl-naphth-2-ylacetamid
N-[4-(11β-Fluor-3,17β-dihydroxy-estra-1,3,5(10)-trien-7α-yl)-butyl]-naphth-2-yl-N-octylacetamid
N-Cyclohexylmethyl-N-[4-(11β-fluor-3,17β-dihydroxy-estra-1,3,5(10)-trien-7α-yl)-butyl]-4-phenyl-butyramid
N-Benzyl-N-[4-(11β-fluor-3,17β-dihydroxy-estra-1,3,5(10)-trien-7α-yl)-butyl]-4-phenylbutyramid
N-[4-(11β-Fluor-3,17β-dihydroxy-estra-1,3,5(10)-trien-7α-yl)-butyl]-N-(2-methoxy-ethyl)-4-phenyl-butyramid
N-[4-(11β-Fluor-3,17β-dihydroxy-estra-1,3,5(10)-trien-7α-yl)-butyl]-4-phenyl-N-(3-phenylpropyl)-butyramid
N-[4-(11β-Fluor-3,17β-dihydroxy-estra-1,3,5(10)-trien-7α-yl)-butyl]-N-hexyl-4-phenylbutyramid
N-[4-(11β-Fluor-3,17β-dihydroxy-estra-1,3,5(10)-trien-7α-yl)-butyl]-N-octyl-4-phenylbutyramid
N-Cyclohexylmethyl-N-[4-(111β-fluor-3,17β-dihydroxy-estra-1,3,5(10)-trien-7α-yl)-butyl]-propionamid
N-Benzyl-N-[4-(11β-fluor-3,17β-dihydroxy-estra-1,3,5(10)-trien-7α-yl)-butyl]-propionamid
N-[4-(11β-Fluor-3,17β-dihydroxy-estra-1,3,5(10)-trien-7α-yl)-butyl]-N-(2-methoxy-ethyl)-propionamid
N-[4-(11β-Fluor-3,17β-dihydroxy-estra-1,3,5(10)-trien-7α-yl)-butyl]-N-(3-phenyl-propyl)-propionamid
N-[4-(11β-Fluor-3,17β-dihydroxy-estra-1,3,5(10)-trien-7α-yl)-butyl]-N-hexyl-propionamid
N-[4-(11β-Fluor-3,17β-dihydroxy-estra-1,3,5(10)-trien-7α-yl)-butyl]-N-octyl-propionamid
4-Biphenyl-N-cyclohexylmethyl-N-[4-(11β-fluor-3,17β-dihydroxy-estra-1,3,5(10)-trien-7α-yl)-butyl]-acetamid
4-Biphenyl-N-benzyl-N-[4-(11β-fluor-3,17β-dihydroxy-estra-1,3,5(10)-trien-7α-yl)-butyl)-acetamid
4-Biphenyl-N-[4-(11β-fluor-3,17β-dihydroxy-estra-1,3,5(10)-trien-7α-yl)-butyl]-N-(2-methoxy-ethyl)-acetamid
4-Biphenyl-N-[4-(11β-fluor-3,17β-dihydroxy-estra-1,3,5(10)-trien-7α-yl)-butyl]-N-(3-phenyl-propyl)-acetamid
4-Biphenyl-N-[4-(11β-fluor-3,17β-dihydroxy-estra-1,3,5(10)-trien-7α-yl)-butyl]-N-hexylacetamid
4-Biphenyl-N-[4-(11β-fluor-3,17β-dihydroxy-estra-1,3,5(10)-trien-7α-yl)-butyl]-N-octylacetamid
4-Acetylamino-N-cyclohexylmethyl-N-[5-(11β-fluor-3,17β-dihydroxy-estra-1,3,5(10)-trien-7α-yl)-pentyl]-benzamid
4-Acetylamino-N-benzyl-N-[5-(11β-fluor-3,17β-dihydroxy-estra-1,3,5(10)-trien-7α-yl)-pentyl]-benzamid
4-Acetylamino-N-[5-(11β-fluor-3,17β-dihydroxy-estra-1,3,5(10)-trien-7α-yl)-pentyl]-N-(2-methoxy-ethyl)-benzamid
4-Acetylamino-N-[5-(11β-fluor-3,17β-dihydroxy-estra-1,3,5(10)-trien-7α-yl)-pentyl]-N-(3-phenyl-propyl)-benzamid
4-Acetylamino-N-[5-(11β-fluor-3,17β-dihydroxy-estra-1,3,5(10)-trien-7α-yl)-pentyl]-N-hexyl-benzamid
4-Acetylamino-N-[5-(11β-fluor-3,17β-dihydroxy-estra-1,3,5(10)-trien-7α-yl)-pentyl]-N-octyl-benzamid
4-Cyano-N-cyclohexylmethyl-N-[5-(11β-fluor-3,17β-dihydroxy-estra-1,3,5(10)-trien-7α-yl)-pentyl]-benzamid
N-Benzyl-4-cyano-N-[5-(11β-fluor-3,17β-dihydroxy-estra-1,3,5(10)-trien-7α-yl)-pentyl]-benzamid
4-Cyano-N-[5-(11β-fluor-3,17β-dihydroxy-estra-1,3,5(10)-trien-7α-yl)-pentyl]-N-(2-methoxy-ethyl)-benzamid

4-Cyano-N-[5-(11β-fluor-3,17β-dihydroxy-estra-1,3,5(10)-trien-7α-yl)-pentyl]-N-(3-phenylpropyl)-benzamid
4-Cyano-N-[5-(11β-fluor-3,17β-dihydroxy-estra-1,3,5(10)-trien-7α-yl)-pentyl]-N-hexylbenzamid
4-Cyano-N-[5-(11β-fluor-3,17β-dihydroxy-estra-1,3,5(10)-trien-7α-yl)-pentyl]-N-octylbenzamid
3-Cyclohexyl-N-cyclohexylmethyl-N-[5-(11β-fluor-3,17β-dihydroxy-estra-1,3,5(10)-trien-7α-yl)-pentyl]-propionamid
N-Benzyl-3-cyclohexyl-N-[5-(11β-fluor-3,17β-dihydroxy-estra-1,3,5(10)-trien-7α-yl)-pentyl]-propionamid
3-Cyclohexyl-N-[5-(11β-fluor-3,17β-dihydroxy-estra-1,3,5(10)-trien-7α-yl)-pentyl]-N-(2-methoxy-ethyl)-propionamid
3-Cyclohexyl-N-[5-(11β-fluor-3,17β-dihykoxy-estra-1,3,5(10)-trien-7α-yl)-pentyl]-N-(3-phenyl-propyl)-propionamid
3-Cyclohexyl-N-[5-(11β-fluor-3,17β-dihydroxy-estra-1,3,5(10)-trien-7α-yl)-pentyl]-N-hexylpropionamid
3-Cyclohexyl-N-[5-(11β-fluor-3,17β-dihydroxy-estra-1,3,5(10)-trien-7α-yl)-pentyl]-N-octylpropionamid
N-Cyclohexylmethyl-N-[5-(11β-fluor-3,17β-dihydroxy-estra-1,3,5(10)-trien-7α-yl)-pentyl]-(3,4-dimethoxyphenyl)-acetamid
N-Benzyl-N-[5-(11β-fluor-3,17β-dihydroxy-estra-1,3,5(10)-trien-7α-yl)-pentyl]-(3,4-dimethoxyphenyl)-acetamid
N-[5-(11β-Fluor-3,17β-dihydroxy-estra-1,3,5(10)-trien-7α-yl)-pentyl)-N-(2-methoxy-ethyl)-(3,4-dimethoxyphenyl)-acetamid
N-[5-(11β-Fluor-3,17β-dihydroxy-estra-1,3,5(10)-trien-7α-yl)-pentyl]-(3,4-dimethoxyphenyl)-N-(3-phenyl-propyl)-acetamid
N-[5-(11β-Fluor-3,17β-dihydroxy-estra-1,3,5(10)-trien-7α-yl)-pentyl]-N-hexyl-(3,4-dimethoxyphenyl)-acetamid
N-[5-(11β-Fluor-3,17β-dihydroxy-estra-1,3,5(10)-trien-7α-yl)-pentyl]-(3,4-dimethoxyphenyl)-N-octyl-acetamid
N-Cyclohexylmethyl-4-ethoxy-N-[5-(11β-fluor-3,17β-dihydroxy-estra-1,3,5(10)-trien-7α-yl)-pentyl]-benzamid
N-Benzyl-4-ethoxy-N-[5-(11β-fluor-3,17β-dihydroxy-estra-1,3,5(10)-trien-7α-yl)-pentyl]-benzamid
4-Ethoxy-N-[5-(11β-fluor-3,17β-dihydroxy-estra-1,3,5(10)-trien-7α-yl)-pentyl]-N-(2-methoxy-ethyl)-benzamid
4-Ethoxy-N-[5-(11β-fluor-3,17β-dihydroxy-estra-1,3,5(10)-trien-7α-yl)-pentyl]-N-(3-phenylpropyl)-benzamid
4-Ethoxy-N-[5-(11β-fluor-3,17β-dihydroxy-estra-1,3,5(10)-trien-7α-yl)-pentyl]-N-hexylbenzamid
4-Ethoxy-N-[5-(11β-fluor-3,17β-dihydroxy-estra-1,3,5(10)-trien-7α-yl)-pentyl)-N-octylbenzamid
N-Cyclohexylmethyl-N-[5-(11β-fluor-3,17β-dihydroxy-estra-1,3,5(10)-trien-7α-yl)-pentyl]-dodecanamid
N-Benzyl-N-[5-(11β-fluor-3,17β-dihydroxy-estra-1,3,5(10)-trien-7α-yl)-pentyl]-dodecanamid
N-[5-(11β-Fluor-3,17β-dihydroxy-esrra-1,3,5(10)-trien-7α-yl)-pentyl]-N-(2-methoxy-ethyl)-dodecanamid
N-[5-(11β-Fluor-3,17β-dihydroxy-estra-1,3,5(10)-trien-7α-yl)-pentyl]-N-(3-phenyl-propyl)-dodecanamid
N-[5-(11β-Fluor-3,17β-dihydroxy-estra-1,3,5(10)-trien-7α-yl)-pentyl] -N-hexyl-dodecanamid
N-[5-(11β-Fluor-3,17β-dihydroxy-estra-1,3,5(10)-trien-7α-yl)-pentyl]-N-octyl-dodecanamid
N-Cyclohexylmethyl-N-[5-(11β-fluor-3,17β-dihydroxy-extra-1,3,5(10)-trien-7α-yl)-pentyl]-naphth-2-yl-acetamid
N-Benzyl-N-[5-(11β-fluor-3,17β-dihydroxy-estra-1,3,5(10)-trien-7α-yl)-pentyl]-naphth-2-ylacetamid
N-[5-(11β-Fluor-3,17β-dihydroxy-estra-1,3,5(10)-trien-7α-yl)-pentyl]-N-(2-methoxy-ethyl)-naphth-2-yl-acetamid
N-[5-(11β-Fluor-3,17β-dihydroxy-estra-1,3,5(10)-trien-7α-yl)-pentyl]-naphth-2-yl-N-(3-phenyl-propyl)-acetamid
N-[5-(11β-Fluor-3,17β-dihydroxy-estra-1,3,5(10)-trien-7α-yl)-pentyl]-N-hexyl-naphth-2-ylacetamid
N-[5-(11β-Fluor-3,17β-dihydroxy-estra-1,3,5(10)-trien-7α-yl)-pentyl]-naphth-2-yl-N-octylacetamid
N-Cyclohexylmethyl-N-[5-(11β-fluor-3,17β-dihydroxy-estra-1,3,5(10)-trien-7α-yl)-pentyl]-4-phenyl-butyramid
N-Benzyl-N-[5-(11β-fluor-3,17β-dihydroxy-estra-1,3,5(10)-trien-7α-yl)-pentyl]-4-phenylbutyramid
N-[5-(11β-Fluor-3,17β-dihydroxy-estra-1,3,5(10)-trien-7α-yl)-pentyl]-N-(2-methoxy-ethyl)-4-phenyl-butyramid
N-[5-(11β-Fluor-3,17β-dihydroxy-estra-1,3,5(10)-trien-7α-yl)-pentyl]-4-phenyl-N-(3-phenylpropyl)-butyramid
N-[5-(11β-Fluor-3,17β-dihydroxy-estra-1,3,5(10)-trien-7α-yl)-pentyl]-N-hexyl-4-phenylbutyramid
N-[5-(11β-Fluor-3,17β-dihydroxy-estra-1,3,5(10)-trien-7α-yl)-pentyl]-N-octyl-4-phenylbutyramid
N-Cyclohexylmethyl-N-[5-(11β-fluor-3,17β-dihydroxy-estra-1,3,5(10)-trien-7α-yl)-pentyl]-propionamid
N-Benzyl-N-[5-(11β-fluor-3,17β-dihydroxy-estra-1,3,5(10)-trien-7α-yl)-pentyl]-propionamid
N-[5-(11β-Fluor-3,17β-dihydroxy-estra-1,3,5(10)-trien-7α-yl)-pentyl]-N-(2-methoxy-ethyl)-propionamid
N-[5-(11β-Fluor-3,17β-dihydroxy-estra-1,3,5(10)-trien-7α-yl)-pentyl]-N-(3-phenyl-propyl)-propionamid
N-[5-(11β-Fluor-3,17β-dihydroxy-estra-1,3,5(10)-trien-7α-yl)-pentyl]-N-hexyl-propionamid
N-[5-(11β-Fluor-3,17β-dihydroxy-estra-1,3,5(10)-trien-7α-yl)-pentyl]-N-octyl-propionamid

4-Biphenyl-N-cyclohexylmethyl-N-[5-(11β-fluor-3,17β-dihydroxy-estra-1,3,5(10)-trien-7α-yl)-pentyl]-acetamid
4-Biphenyl-N-benzyl-N-[5-(11β-fluor-3,17β-dihydroxy-estra-1,3,5(10)-trien-7α-yl)-pentyl]-acetamid
4-Biphenyl-N-[5-(11β-fluor-3,17β-dihydroxy-estra-1,3,5(10)-trien-7α-yl)-pentyl]-N-(2-methoxy-ethyl)-acetamid
4-Biphenyl-N-[5-(11β-fluor-3,17β-dihydroxy-estra-1,3,5(10)-trien-7α-yl)-pentyl]-N-(3-phenyl-propyl)-acetamid
4-Biphenyl-N-[5-(11β-fluor-3,17β-dihydroxy-estra-1,3,5(10)-trien-7α-yl)-pentyl]-N-hexylacetamid
4-Biphenyl-N-[5-(11β-fluor-3,17β-dihydroxy-estra-1,3,5(10)-trien-7α-yl)-pentyl]-N-octylacetamid
4-Acetylamino-N-cyclohexylmethyl-N-[6-(11β-fluor-3,17β-dihydroxy-estra-1,3,5(10)-trien-7α-yl)-hexyl]-benzamid
4-Acetylamino-N-benzyl-N-[6-(11β-fluor-3,17β-dihydroxy-estra-1,3,5(10)-trien-7α-yl)-hexyl]-benzamid
4-Acetylamino-N-[6-(11β-fluor-3,17β-dihydroxy-estra-1,3,5(10)-trien-7α-yl)-hexyl]-N-(2-methoxy-ethyl)-benzamid
4-Acetylamino-N-[6-(11β-fluor-3,17β-dihydroxy-estra-1,3,5(10)-trien-7α-yl)-hexyl]-N-(3-phenyl-propyl)-benzamid
4-Acetylamino-N-[6-(11β-fluor-3,17β-dihydroxy-estra-1,3,5(10)-trien-7α-yl)-hexyl]-N-hexyl-benzamid
4-Acetylamino-N-[6-(11β-fluor-3,17β-dihydroxy-estra-1,3,5(10)-trien-7α-yl)-hexyl]-N-octylbenzamid
4-Cyano-N-cyclohexylmethyl-N-[6-(11β-fluor-3,17β-dihydroxy-estra-1,3,5(10)-trien-7α-yl)-hexyl]-benzamid
N-Benzyl-4-cyano-N-[6-(11β-fluor-3,17β-dihydroxy-estra-1,3,5(10)-trien-7α-yl)-hexyl]-benzamid
4-Cyano-N-[6-(11β-fluor-3,17β-dihydroxy-estra-1,3,5(10)-trien-7α-yl)-hexyl]-N-(2-methoxy-ethyl)-benzamid
4-Cyano-N-[6-(11β-fluor-3,17β-dihydroxy-estra-1,3,5(10)-trien-7α-yl)-hexyl]-N-(3-phenylpropyl)-benzamid
4-Cyano-N-[6-(11β-fluor-3,17β-dihydroxy-estra-1,3,5(10)-trien-7α-yl)-hexyl]-N-hexylbenzamid
4-Cyano-N-[6-(11β-fluor-3,17β-dihydroxy-estra-1,3,5(10)-trien-7α-yl)-hexyl]-N-octylbenzamid
3-Cyclohexyl-N-cyclohexylmethyl-N-[6-(11β-fluor-3,17β-dihydroxy-estra-1,3,5(10)-trien-7α-yl)-hexyl]-propionamid
N-Benzyl-3-cyclohexyl-N-[6-(11β-fluor-3,17β-dihydroxy-estra-1,3,5(10)-trien-7α-yl)-hexyl]-propionamid
3-Cyclohexyl-N-[6-(11β-fluor-3,17β-dihydroxy-estra-1,3,5(10)-trien-7α-yl)-hexyl]-N-(2-methoxy-ethyl)-propionamid
3-Cyclohexyl-N-[6-(11β-fluor-3,17β-dihydroxy-estra-1,3,5(10)-trien-7α-yl)-hexyl]-N-(3-phenyl-propyl)-propionamid
3-Cyclohexyl-N-[6-(11β-fluor-3,17β-dihydroxy-estra-1,3,5(10)-trien-7α-yl)-hexyl]-N-hexylpropionamid
3-Cyclohexyl-N-[6-(11β-fluor-3,17β-dihydroxy-estra-1,3,5(10)-trien-7α-yl)-hexyl]-N-octylpropionamid
N-Cyclohexylmethyl-N-[6-(11β-fluor-3,17β-dihydroxy-estra-1,3,5(10)-trien-7α-yl)-hexyl]-(3,4-dimethoxyphenyl)-acetamid
N-Benzyl-N-[6-(11β-fluor-3,17β-dihydroxy-estra-1,3,5(10)-trien-7α-yl)-hexyl]-(3,4-dimethoxyphenyl)-acetamid
N-[6-(11β-Fluor-3,17β-dihydroxy-estra-1,3,5(10)-trien-7α-yl)-hexyl]-N-(2-methoxy-ethyl)-(3,4-dimethoxyphenyl)-acetamid
N-[6-(11β-Fluor-3,17β-dihydroxy-estra-1,3,5(10)-trien-7α-yl)-hexyl]-(3,4-dimethoxyphenyl)-N-(3-phenyl-propyl)-acetamid
N-[6-(11β-Fluor-3,17β-dihydroxy-estra-1,3,5(10)-trien-7α-yl)-hexyl]-N-hexyl-(3,4-dimethoxyphenyl)-acetamid
N-[6-(11β-Fluor-3,17β-dihydroxy-estra-1,3,5(10)-trien-7α-yl)-hexyl]-(3,4-dimethoxyphenyl)-N-octyl-acetamid
N-Cyclohexylmethyl-4-ethoxy-N-[6-(11β-fluor-3,17β-dihydroxy-estra-1,3,5(10)-trien-7α-yl)-hexyl]-benzamid
N-Benzyl-4-ethoxy-N-[6-(11β-fluor-3,17β-dihydroxy-estra-1,3,5(10)-trien-7α-yl)-hexyl]-benzamid
4-Ethoxy-N-[6-(11β-fluor-3,17β-dihydroxy-estra-1,3,5(10)-trien-7α-yl)-hexyl]-N-(2-methoxy-ethyl)-benzamid
4-Ethoxy-N-[6-(11β-fluor-3,17β-dihydroxy-estra-1,3,5(10)-trien-7α-yl)-hexyl]-N-(3-phenylpropyl)-benzamid
4-Ethoxy-N-[6-(11β-fluor-3,17β-dihydroxy-estra-1,3,5(10)-trien-7α-yl)-hexyl]-N-hexylbenzamid
4-Ethoxy-N-[6-(11β-fluor-3,17β-dihydroxy-estra-1,3,5(10)-trien-7α-yl)-hexyl]-N-octylbenzamid
N-Cyclohexylmethyl-N-[6-(11β-fluor-3,17β-dihydroxy-estra-1,3,5(10)-trien-7α-yl)-hexyl]-dodecanamid
N-Benzyl-N-[6-(11β-fluor-3,17β-dihydroxy-estra-1,3,5(10)-trien-7α-yl)-hexyl]-dodecanamid
N-[6-(11β-Fluor-3,17β-dihydroxy-estra-1,3,5(10)-trien-7α-yl)-hexyl]-N-(2-methoxy-ethyl)-dodecanamid
N-[6-(11β-Fluor-3,17β-dihydroxy-estra-1,3,5(10)-trien-7α-yl)-hexyl]-N-(3-phenyl-propyl)-dodecanamid
N-[6-(11β-Fluor-3,17β-dihydroxy-estra-1,3,5(10)-trien-7α-yl)-hexyl]-N-hexyl-dodecanamid
N-[6-(11β-Fluor-3,17β-dihydroxy-estra-1,3,5(10)-trien-7α-yl)-hexyl]-N-octyl-dodecanamid
N-Cyclohexylmethyl-N-[6-(11β-fluor-3,17β-dihydroxy-estra-1,3,5(10)-trien-7α-yl)-hexyl]-naphth-2-yl-acetamid
N-Benzyl-N-[6-(11β-fluor-3,17β-dihydroxy-estra-1,3,5(10)-trien-7α-yl)-hexyl]-naphth-2-ylacetamid

N-[6-(11β-Fluor-3,17β-dihydroxy-estra-1,3,5(10)-trien-7α-yl)-hexyl]-N-(2-methoxy-ethyl)-naphth-2-yl-acetamid
N-[6-(11β-Fluor-3,17β-dihydroxy-estra-1,3,5(10)-trien-7α-yl)-hexyl]-naphth-2-yl-N-(3-phenyl-propyl)-acetamid
N-[6-(11β-Fluor-3,17β-dihydroxy-estra-1,3,5(10)-trien-7α-yl)-hexyl]-N-hexyl-naphth-2-ylacetamid
N-[6-(11β-Fluor-3,17β-dihydroxy-estra-1,3,5(10)-trien-7α-yl)-hexyl]-naphth-2-yl-N-octylacetamid
N-Cyclohexylmethyl-N-[6-(11β-fluor-3,17β-dihydroxy-estra-1,3,5(10)-trien-7α-yl)-hexyl]-4-phenyl-butyramid
N-Benzyl-N-[6-(11β-fluor-3,17β-dihydroxy-estra-1,3,5(10)-trien-7α-yl)-hexyl]-4-phenylbutyramid
N-[6-(11β-Fluor-3,17β-dihydroxy-estra-1,3,5(10)-trien-7α-yl)-hexyl]-N-(2-methoxy-ethyl)-4-phenyl-butyramid
N-[6-(11β-Fluor-3,17β-dihydroxy-estra-1,3,5(10)-trien-7α-yl)-hexyl]-4-phenyl-N-(3-phenylpropyl)-butyramid
N-[6-(11β-Fluor-3,17β-dihydroxy-estra-1,3,5(10)-trien-7α-yl)-hexyl]-N-hexyl-4-phenylbutyramid
N-[6-(11β-Fluor-3,17β-dihydroxy-estra-1,3,5(10)-trien-7α-yl)-hexyl]-N-octyl-4-phenylbutyramid
9-N-Cyclohexylmethyl-N-[6-(11β-fluor-3,17β-dihydroxy-estra-1,3,5(10)-trien-7α-yl)-hexyl]-propionamid
N-Benzyl-N-[6-(11β-fluor-3,17β-dihydroxy-estra-1,3,5(10)-trien-7α-yl)-hexyl]-propionamid
N-[6-(11β-Fluor-3,17β-dihydroxy-estra-1,3,5(10)-trien-7α-yl)-hexyl]-N-(2-methoxy-ethyl)-propionamid
N-[6-(11β-Fluor-3,17β-dihydroxy-estra-1,3,5(10)-trien-7α-yl)-hexyl]-N-(3-phenyl-propyl)-propionamid
N-[6-(11β-Fluor-3,17β-dihydroxy-estra-1,3,5(10)-trien-7α-yl)-hexyl]-N-hexyl-propionamid
N-[6-(11β-Fluor-3,17β-dihydroxy-estra-1,3,5(10)-trien-7α-yl)-hexyl]-N-octyl-propionamid 4-Biphenyl-N-cyclohexylmethyl-N-[6-(11β-fluor-3,17β-dihydroxy-estra-1,3,5(10)-trien-7α-yl)-hexyl]-acetamid
4-Biphenyl-N-benzyl-N-[6-(11β-fluor-3,17β-dihydroxy-estra-1,3,5(10)-trien-7α-yl)-hexyl]-acetamid
4-Biphenyl-N-[6-(11β-fluor-3,17β-dihydroxy-estra-1,3,5(10)-trien-7α-yl)-hexyl]-N-(2-methoxy-ethyl)-acetamid
4-Biphenyl-N-[6-(11β-fluor-3,17β-dihydroxy-estra-1,3,5(10)-trien-7α-yl)-hexyl]-N-(3-phenyl-propyl)-acetamid
4-Biphenyl-N-[6-(11β-fluor-3,17β-dihydroxy-estra-1,3,5(10)-trien-7α-yl)-hexyl]-N-hexylacetamid
4-Biphenyl-N-[6-(11β-fluor-3,17β-dihydroxy-estra-1,3,5(10)-trien-7α-yl)-hexyl]-N-octylacetamid
11β-Fluor-7α-{5-[methyl-(2-p-tolyl-ethyl)-amino]-pentyl) -estra-1,3,5(10)-trien-3,17β-diol 7α-(5-{[2-(4-Ethoxy-phenyl)-ethyl]-methyl-amino}-pentyl)-11β-fluor-estra-1,3,5(10)-trien-3,17β-diol
11 β-Fluor-7α-{5-[methyl-(3-phenyl-propyl)-amino]-pentyl}-estra-1,3,5(10)-trien-3,17β-diol
11β-Fluor-7α-{5-[methyl-(3-pyridin-3-yl-propyl)-amino]-pentyl}-estra-1,3,5(10)-trien-3,17β-diol
11 β-Fluor-7α-{5-[methyl-(3-p-tolyl-propyl)-amino]-pentyl} -estra-1,3,5(10)-trien-3,17β-diol 7α-(5-{[3-(4-Chlor-phenyl)-propyl]-methyl-amino} -pentyl)-11β-fluor-estra-1,3,5(10)-trien-3,17β-diol
7α-(5-{[3-(4-Ethoxy-phenyl)-propyl]-methyl-amino} -pentyl)-11β-fluor-estra-1,3,5(10)-trien-3,17β-diol
11β-Fluor-7α-{5-[methyl-(4-methyl-pentyl)-amino]-pentyl}-estra-1,3,5(10)-trien-3,17β-diol

12. Verwendung der Verbindungen der allgemeinen Formel I gemäß Anspruch 1 zur Herstellung von Arzneimitteln.

13. Pharmazeutische Präparate enthaltend mindestens eine Verbindung der allgemeinen Formel I gemäß Anspruch 1 sowie einen pharmazeutisch verträglichen Träger.

## Revendications

1. Estratriènes 11β-halogéno-7α-substitués de formule générale I

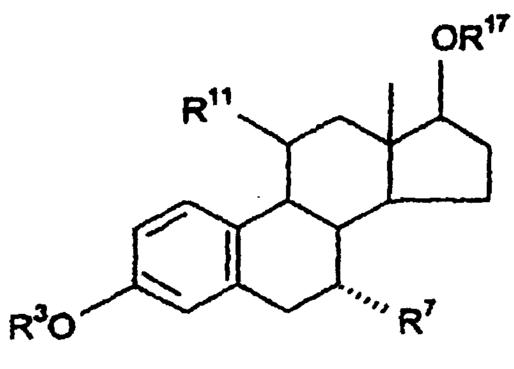

(I)

dans laquelle

$R^3$ représente un atome d'hydrogène, un radical hydrocarboné ayant jusqu'à 8 atomes de carbone ou un radical de formule partielle $R^{3'}$-C(O)-, dans laquelle $R^{3'}$ représente un atome d'hydrogène ou un radical hydrocarboné ayant jusqu'à 8 atomes de carbone ou un radical phényle,

$R^7$ représente un radical de formule -A-B-Z-$R^{20}$,
dans laquelle

A représente une liaison directe ou un radical benzylidène, où le groupe méthylène est lié à l'atome de carbone en 7 du stéroïde, ou un radical phénylène,

B représente un groupe alkylène, alcénylène ou alcynylène à chaîne droite ou ramifiée ayant de 3 à 14 atomes de carbone,

et

Z représente -$NR^{21}$- et $R^{21}$ représente un groupe alkyle en $C_1$-$C_3$,

où $R^{20}$ représente alors

un atome d'hydrogène,

un groupe alkyle, alcényle ou alcynyle à chaîne droite ou ramifiée ayant jusqu'à 10 atomes de carbone, ou l'un des groupements -D-$C_nF_{2n+1}$, où D représente un groupe alkylène, alcénylène ou alcynylène à chaîne droite ou ramifiée ayant jusqu'à 8 atomes de carbone et n représente un entier valant de 1 à 8,

-L-CH=CF-$C_pF_{2p+1}$, où L représente un groupe alkylène, alcénylène ou alcynylène à chaîne droite ou ramifiée ayant de 2 à 7 atomes de carbone et p représente un entier valant de 2 à 7,

-D-O-$(CH_2)_q$-aryle, où D a la signification déjà indiquée, q vaut 0, 1, 2 ou 3 et aryle représente un radical phényle, 1- ou 2-naphtyle ou hétéroaryle éventuellement mono- ou bisubstitué,

-D-O-$(CH_2)_r$-$C_nF_{2n+1}$, où D et n ont les significations déjà indiquées et r représente un entier valant de 1 à 5, ou

$R^{20}$ et $R^{21}$ forment, avec l'atome d'azote auquel ils sont liés, un hétérocycle saturé ou insaturé à 5 ou 6 chaînons, qui renferme éventuellement un ou deux autres hétéroatomes choisis parmi un azote, un oxygène et un soufre, et est éventuellement substitué,
ou

Z représente -$SO_x$- et x vaut 0, 1 ou 2,

où $R^{20}$ représente alors

un groupe alkyle, alcényle ou alcynyle à chaîne droite ou ramifiée ayant jusqu'à 10 atomes de carbone, ou l'un des groupements -D-$C_nF_{2n+1}$, où D représente un groupe alkylène, alcénylène ou alcynylène à chaîne droite ou ramifiée ayant jusqu'à 8 atomes de carbone et n représente un entier valant de 1 à 8,

-L-CH=CF-$C_pF_{2p+1}$, où L représente un groupe alkylène, alcénylène ou alcynylène à chaîne droite ou ramifiée ayant de 2 à 7 atomes de carbone et p représente un entier valant de 2 à 7,

-D-O-$(CH_2)_q$-aryle, où D a la signification déjà indiquée, q vaut 0, 1, 2 ou 3 et aryle représente un radical phényle, 1- ou 2-naphtyle ou hétéroaryle éventuellement mono- ou bisubstitué, -D-O-$(CH_2)_r$-$C_nF_{2n+1}$, où D et n ont les significations déjà indiquées et r représente un entier valant de 1 à 5,

ou

Z représente $-NR^{31}-$,

où $R^{20}$ représente alors un radical alkyle, alcényle ou alcynyle à chaîne droite ou ramifiée ayant jusqu'à 14 atomes de carbone, qui peut être interrompu par un à trois hétéroatomes -O- et -S- et des groupements $-NR^{32}-$, où $R^{32}$ représente un atome d'hydrogène ou un radical alkyle en $C_1$-$C_3$ et/ou peut être partiellement fluoré, un radical aryle ou hétéroaryle éventuellement mono- ou bisubstitué, un radical cycloalkyle en $C_3$-$C_{10}$ éventuellement mono- ou bisubstitué, un radical $C_4$-$C_{15}$-cycloalkylalkyle éventuellement mono- ou bisubstitué, un radical aralkyle en $C_7$-$C_{20}$ éventuellement mono- ou bisubstitué, un radical hétéroaryl-$C_1$-$C_6$-alkyle éventuellement mono- ou bisubstitué ou un radical aminoalkyle éventuellement substitué, et

$R^{31}$ représente un radical de formule $-C(O)R^{33}$ ou $-CH_2-R^{33}$,

où $R^{33}$ représente alors un radical alkyle, alcényle ou alcynyle à chaîne droite ou ramifiée ayant jusqu'à 14 atomes de carbone, qui peut être interrompu par un à trois hétéroatomes -O- et -S- et des groupements $-NR^{32}-$, où $R^{32}$ représente un atome d'hydrogène ou un radical alkyle en $C_1$-$C_3$ et/ou peut être partiellement fluoré, un radical aryle ou hétéroaryle éventuellement mono- ou bisubstitué, un radical cycloalkyle en $C_3$-$C_{10}$ éventuellement mono- ou bisubstitué, un radical $C_4$-$C_{15}$-cycloalkylalkyle éventuellement mono- ou bisubstitué, un radical aralkyle en $C_7$-$C_{20}$ éventuellement mono- ou bisubstitué, un radical hétéroaryl-$C_1$-$C_6$-alkyle éventuellement mono- ou bisubstitué, un radical aminoalkyle éventuellement substitué ou un radical biphénylène,

à l'exception des composés

11β-fluoro-7α-{5-[N-méthyl-N-3-(4,4,5,5,5-pentafluoropentylthio)propylamino]pentyl}estra-1,3,5(10)triène-3,17β-diol
11β-fluoro-7α-{5-[2-(4,4,5,5,5-pentafluoropentylthiométhyl)pyrrolidin-1-yl]pentyl}-estra-1,3,5(10)triène-3,17β-diol
11b-fluoro-7α-{6-[N-méthyl-N-3-(4,4,5,5,5-pentafluoropentylthio)propylamino]hexyl}estra-1,3,5(10)triène-3,17β-diol
11β-fluoro-7α-{5-[(2S)-2-(4-trifluorométhylphénylthiométhyl)pyrrolidin-1-yl]pentyl}estra-1,3,5(10)triène-3,17β-diol
11β-fluoro-7α-{5-[(2S)-2-(4,4,5,5,5-pentafluoropentylthiométhyl)pyrrolidin-1-yl]pentyl}estra-1,3,5(10)triène-3,17β-diol
11β-fluoro-7α-{5-[(2S)-2-(4,4,5,5,5-pentafluoropentanesulfinylméthyl)pyrrolidin-1-yl]pentyl}estra-1,3,5(10)triène-3,17β-diol
11β-fluoro-7α-{5-[(2S)-2-(4,4,5,5,5-pentafluoropentanesulfonylméthyl)pyrrolidin-1-yl]pentyl}estra-1,3,5 (10)triène-3,17β-diol
$R^{11}$ représente un atome de fluore ou de chlore,
$R^{17}$ représente un atome d'hydrogène ou un radical de formule partielle $R^{17'}$-C(O)-, dans laquelle $R^{17'}$ représente un atome d'hydrogène ou un radical hydrocarboné ayant jusqu'à 8 atomes de carbone.

2. Estratriènes 11β-halogéno-7α-substitués selon la revendication 1, dans laquelle $R^3$ représente un atome d'hydrogène.

3. Estratriènes 11β-halogéno-7α-substitués selon la revendication 1, dans laquelle $R^3$ représente un radical benzoyle.

4. Estratriènes 11β-halogéno-7α-substitués selon la revendication 1, dans laquelle $R^{11}$ représente un atome de fluor.

5. Estratriènes 11β-halogéno-7α-substitués selon la revendication 1, dans laquelle $R^{17}$ représente un atome d'hydrogène.

6. Estratriènes 11β-halogéno-7α-substitués selon la revendication 1, dans laquelle A représente une liaison directe.

7. Estratriènes 11β-halogéno-7α-substitués selon la revendication 6, dans laquelle $R^7$ représente un radical de formule-$(CH_2)_s$-Z-$R^{20}$,
dans laquelle

s représente un entier valant de 3 à 8,

Z représente $-NR^{21}$ et $R^{21}$ représente un groupe alkyle en $C_1$-$C_3$,

où $R^{21}$ représente

un atome d'hydrogène,

un groupe alkyle en $C_1$-$C_9$, ou

l'un des groupements

- $(CH_2)_m$-$C_nF_{2n+1}$, où m et n représentent chacun, indépendamment l'un de l'autre, un entier valant de 1 à 8,

-$(CH_2)_o$-CH=CF-$C_pF_{2p+1}$, où o vaut 1, 2 ou 3 et p représente un entier valant de 2 à 7,

-$(CH_2)_m$-O-$(CH_2)_q$-aryle, où m a la signification déjà indiquée, q vaut 0, 1, 2 ou 3 et aryle représente un radical phényle ou hétéroaryle éventuellement mono- ou bisubstitué,

- $(CH_2)_m$-O-$(CH_2)_r$-$C_nF_{2n+1}$, où m et n ont les significations déjà indiquées et r représente un entier valant de 1 à 5.

**8.** Estratriènes 11β-halogéno-7α-substitués selon la revendication 6, dans laquelle $R^7$ représente un radical de formule-$(CH_2)_s$-Z-$R^{20}$,
dans laquelle

s représente un entier valant de 3 à 8,

Z représente $-NR^{21}$ et $R^{21}$ représente un groupe alkyle en $C_1$-$C_3$,

où $R^{20}$ et $R^{21}$ forment, avec l'atome d'azote auquel ils sont liés, un hétérocycle saturé ou insaturé à 5 ou 6 chaînons, qui renferme éventuellement un ou deux autres hétéroatomes choisis parmi un azote, un oxygène et un soufre, et est éventuellement substitué.

**9.** Estratriènes 11β-halogéno-7α-substitués selon la revendication 6, dans laquelle $R^7$ représente un radical de formule -$(CH_2)_s$-Z-$R^{20}$,
dans laquelle

s représente un entier valant de 3 à 8,

Z représente $-SO_x$- et x vaut 0, 1 ou 2,

où $R^{20}$ représente

- $(CH_2)_m$-O-$(CH_2)_r$-$C_nF_{2n+1}$, où m et n ont les significations déjà indiquées et r représente un entier valant de 1 à 5.

**10.** Estratriènes 11β-halogéno-7α-substitués selon la revendication 1, dans laquelle $R^7$ est choisi parmi le groupe des chaînes latérales suivantes

$-(CH_2)_5N(CH_3)(CH_2)_3C_2F_5$

$-(CH_2)_5N(CH_3)(CH_2)_6C_2F_5$

$-(CH_2)_5N(CH_3)(CH_2)_7C_2F_5$

$-(CH_2)_5N(CH_3)(CH_2)_8C_2F_5$

$-(CH_2)_6N(CH_3)(CH_2)_6C_2F_5$

$-(CH_2)_6N(CH_3)(CH_2)_7C_2F_5$

$-(CH_2)_6N(CH_3)(CH_2)_8C_2F_5$

$-(CH_2)_5N(CH_3)(CH_2)_2C_4F_9$

$-(CH_2)_5N(CH_3)(CH_2)_3C_6F_{13}$

$-(CH_2)_5N(CH_3)(CH_2)_3C_8F_{17}$

$-(CH_2)_5N(CH_3)(CH_2)_6C_4F_9$

$-(CH_2)_5N(CH_3)(CH_2)_6C_6F_{13}$

$-(CH_2)_5N(CH_3)(CH_2)_6C_8F_{17}$

$-(CH_2)_5N(CH_3)H$

$-(R_2)_5N(CH_3)(CH_2)_9H$

$-(CH_2)_5N(CH_3)CH_2CH=CF-C_2F_5$

$-(R_2)_5N(CT_3)CH_2CH=CF-C_3F_7$

$-(CH_2)_5N(CH_3)CH_2CH=CF-C_5F_{11}$

$-(CH_2)_5N(CH_3)CH_2CH=CF-C_7F_{15}$

$-(CH_2)_5$-1-pyrrolidinyle

$-(CH_2)_5N(CH_3)(CH_2)_3$ ophényle

$-(CH_2)_5N(CH_3)(CH_2)_3$ obenzyle

$-(CH_2)_5N(CH_3)(CH_2)_3O(CH_2)_3C_2F_5$

$-(CH_2)_9S(CH_2)_3C_2F_5$

$-(CH_2)_9SO(CH_2)_3C_2F_5$

$-(CH_2)_9SO_2(CH_2)_3C_2F_5$

$-(CH_2)_5N(CH_3)(CH_2)_3CH(CH_3)_2$

$-(CH_2)_5N(CH_3)(CH_2)_3$- pyridyle

$-(CH_2)_5N(CH_3)(CH_2)_3$- phényle

$-(CH_2)_5N(CH_3)(CH_2)_2$-p- tolyle

$-(CH_2)_5N(CH_3)(CH_2)_2$-p- éthoxyphényle

$-(CH_2)_5N(CH_3)(CH_2)_3$-p- tolyle

$-(CH_2)_5N(CH_3)(CH_2)_3$-p- chlorophényle

$-(CH_2)_5N(CH_3)(CH_2)_3-CH_2-$ phényle

$-(CH_2)_5N(CH_3)(CH_2)_2-O-p-Br-$ phényle

$-(CH_2)_5N(CH_3)(CH_2)_2-O-p-CF_3-$phényle

**11.** Composés de formule générale I, à savoir

le 11β-fluoro-7α-{5-[méthyl(8,8,9,9,9-pentafluorononyl)amino]pentyl}estra-1,3,5(10)triène-3,17β-diol
le 11β-fluoro-7α-{5-[méthylnonylamino]pentyl}estra-1,3,5(10)triène-3,17β-diol
le 11β-fluoro-7α-{5-[méthyl(9,9,10,10,10-pentafluorodécyl)amino]pentyl}estra-1,3,5 (10) triène-3,17β-diol
le 11β-fluoro-7α-{6-[méthyl(8,8,9,9,9-pentafluorononyl)amino]hexyl}estra-1,3,5(10)triène-3,17β-diol
le 11β-fluoro-7α-{6-[méthyl(9,9,10,10,10-pentafluorodécyl)amino]hexyl}estra-1,3,5(10)triène-3,17β-diol
le 11β-fluoro-7α-(5-(méthylamino)pentyl)estra-1,3,5(10)triène-3,17β-diol
le 11β-fluoro-7α-(5-pyrrolidin-1-yl)estra-1,3,5(10)triène-3,17β-diol
le 11β-fluoro-7α-{5-[méthyl(4,4,5,5,5-pentafluoropentyl)amino]pentyl}estra-1,3,5(10)triène-3,17β-diol
le 11β-fluoro-7α-(5-[méthyl(4,4,5,5,6,6,7,7,8,8,9,9,9-tridécafluorononyl)amino]pentyl}estra-1,3,5(10)triène-3,17β-diol
le 11β-fluoro-7α-{5-[(4,4,5,5,6,6,7,7,8,8,9,9,10,10,11,11,11-heptadécafluoroundécyl)méthylamino]pentyl}estra-1,3,5(10)triène-3,17β-diol
le 11β-fluoro-7α-{5-[méthyl(3,3,4,4,5,5,6,6,6-nonafluorohexyl)amino]pentyl}estra-1,3,5(10)triène-3,17β-diol
le 11β-fluoro-7α-{5-[méthyl(7,7,8,8,8-pentafluorooctyl)amino]pentyl}estra-1,3,5(10)triène-3,17β-diol
le 11β-fluoro-7α-{6-[méthyl(7,7,8,8,8-pentafluorooctyl)amino]hexyl}estra-1,3,5(10)triène-3,17β-diol
le 11β-fluoro-7α-{5-[méthyl(7,7,8,8,9,9,10,10,10-nonafluorodécyl)amino]pentyl}estra-1,3,5(10)triène-3,17β-diol
le 11β-fluoro-7α-{5-[méthyl(7,7,8,8,9,9,10,10,11,11,12,12,12-tridécafluorododécyl)amino]pentyl}estra-1,3,5 (10)triène-3,17β-diol
le 11β-fluoro-7α-{5-[(7,7,8,8,9,9,10,10,11,11,12,12,13,13,14,14,14-heptadécafluorotétradécyl)méthylamino] pentyl}estra -1,3,5(10)triène-3,17β-diol
le 11β-fluoro-7α-{5-[(3,4,4,5,5,5-hexafluoropent-2-ényl)méthylamino]pentyl}estra-1,3,5(10)triène-3,17β-diol
le 7α-{5-[(3,4,4,5,5,6,6,7,7,8,8,8-dodécafluorooct-2-ényl)méthylamino]pentyl}-11β-fluoroestra-1,3,5(10)triène-3,17β-diol
le 11β-fluoro-7α-{5-[(3,4,4,5,5,6,6,7,7,8,8,9,9,10,10,10-hexadécafluorodéc-2-ényl)méthylamino]pentyl}estra-1,3,5(10)triène-3,17β-diol
le 11β-fluoro-7α-{5-[méthyl(3-phénoxypropyl)amino]pentyl}estra-1,3,5(10)triène-3,17β-diol
le 7α-{5-[(3-benzyloxypropyl)méthylamino]pentyl}-11β-fluoroestra-1,3,5(10)triène-3,17β-diol
le 11β-fluoro-7α-{5-[N-méthyl-N-3-(4,4,5,5,5-pentafluoropentyloxy)propylamino]pentyl}estra-1,3,5(10)triène-3,17β-diol
le 11β-fluoro-7α-[9-(4,4,5,5,5-pentafluoropentylsulfinyl)nonyl]estra-1,3,5(10)triène-3,17β-diol
le N-[4-(11β-fluoro-3,17-dihydroestra-1,3,5(10)trién-7α-yl)butyl]-N-pentyl-benzamide
le 4-acétylamino-N-cyclohexylméthyl-N-[4-(11β-fluoro-3,17β-dihydroxyestra-1,3, 5 (10) -trién-7α-yl)butyl]benzamide
le 4-acétylamino-N-benzyl-N-[4-(11β-fluoro-3,17β-dihydroxyestra-1,3,5(10)-trién-7α-yl)butyl]benzamide
le 4-acétylamino-N-[4-(11β-fluoro-3,17β-dihydroxyestra-1,3,5(10)-trién-7α-yl)butyl]-N-(2-méthoxyéthyl)benzamide
le 4-acétylamino-N-[4-(11β-fluoro-3,17β-dihydroxyestra-1,3,5(10)-trién-7α-yl)butyl]-N-(3-phénylpropyl)benzamide
le 4-acétylamino-N-[4-(11β-fluoro-3,17β-dihydroxyestra-1,3,5(10)-trién-7α-yl)butyl]-N-hexylbenzamide
le 4-acétylamino-N-[4-(11β-fluoro-3,17β-dihydroxyestra-1,3,5(10)-trién-7α-yl)butyl]-N-octylbenzamide
le 4-cyano-N-cyclohexylméthyl-N-[4-(11β-fluoro-3,17β-dihydroxyestra-1,3,5(10)-trién-7α-yl)butyl]benzamide
le N-benzyl-4-cyano-N-[4-(11β-fluoro-3,17β-dihydroxyestra-1,3,5(10)-trién-7α-yl)butyl]benzamide
le 4-cyano-N-[4-(11β-fluoro-3,17β-dihydroxyestra-1,3,5(10)-trién-7α-yl)butyl]-N-(2-méthoxyéthyl)benzamide
le 4-cyano-N-[4-(11β-fluoro-3,17β-dihydroxyestra-1,3,5(10)-trién-7α-yl)butyl]-N-(3-phénylpropyl)benzamide
le 4-cyano-N-[4-(11β-fluoro-3,17β-dihydroxyestra-1,3,5(10)-trién-7α-yl)butyl]-N-hexylbenzamide
le 4-cyano-N-[4-(11β-fluoro-3,17β-dihydroxyestra-1,3,5(10)-trién-7α-yl)butyl]-N-octylbenzamide
le 3-cyclohexyl-N-cyclohexylméthyl-N-[4-(11β-fluoro-3,17β-dihydroxyestra-1,3,5(10)-trién-7α-yl)butyl]propionamide
le N-benzyl-3-cyclohexyl-N-[4-(11β-fluoro-3,17β-dihydroxyestra-1,3,5(10)-trién-7α-yl)butyl]propionamide

le 3-cyclohexyl-N-[4-(11β-fluoro-3,17β-dihydroxyestra-1,3,5(10)-trién-7α-yl)butyl]-N-(2-méthoxyéthyl)propionamide
le 3-cyclohexyl-N-[4-(11β-fluoro-3,17β-dihydroxyestra-1,3,5(10)-trién-7α-yl)butyl]-N-(3-phénylpropyl)propionamide
le 3-cyclohexyl-N- [4- (11β-fluoro-3,17β-dihydroxyestra-1,3,5(10)-trién-7α-yl)butyl]-N-hexylpropionamide
le 3-cyclohexyl-N-[4-(11β-fluoro-3,17β-dihydroxyestra-1,3,5(10)-trién-7α-yl)butyl]-N-octylpropionamide
le N-cyclohexylméthyl-N-[4-(11β-fluoro-3,17β-dihydroxyestra-1,3,5(10)-trién-7α-yl)butyl]-(3,4-diméthoxyphényl)acétamide
le N-benzyl-N-[4-(11β-fluoro-3,17β-dihydroxyestra-1,3,5(10)-trién-7α-yl)butyl]-(3,4-diméthoxyphényl)acétamide
le N-[4-(11β-fluoro-3,17β-dihydroxyestra-1,3,5(10)-trién-7α-yl)butyl]-N-(2-méthoxyéthyl)-(3,4-diméthoxyphényl)acétamide
le N-[4-(11β-fluoro-3,17β-dihydroxyestra-1,3,5(10)-trién-7α-yl)butyl]-(3,4-diméthoxyphényl)-N-(3-phénylpropyl)acétamide
le N-[4-(11β-fluoro-3,17β-dihydroxyestra-1,3,5(10)-trién-7α-yl)butyl]-N-hexyl-(3,4-diméthoxyphényl)acétamide
le N-[4-(11β-fluoro-3,17β-dihydroxyestra-1,3,5(10)-trién-7α-yl)butyl]-(3,4-diméthoxyphényl)-N-octylacétamide
le N-cyclohexylméthyl-4-éthoxy-N-[4-(11β-fluoro-3,17β-dihydroxyestra-1,3,5(10)-trién-7α-yl)butyl]benzamide
le N-benzyl-4-éthoxy-N-(4-(11β-tluoro-3,17β-dihydroxyestra-1, 3,5(10)-trién-7α-yl)butyl]benzamide
le 4-éthoxy-N-[4-(11β-fluoro-3,17β-dihydroxyestra-1,3,5(10)-trién-7α-yl)butyl]-N-(2-méthoxyéthyl)benzamide
le 4-éthoxy-N-[4-(11β-fluoro-3,17β-dihydroxyestra-1,3,5(10)-trién-7α-yl)butyl]-N-(3-phénylpropyl)benzamide
le 4-éthoxy-N-[4-(11β-fluoro-3,17β-dihydroxyestra-1,3,5(10)-trién-7α-yl)butyl]-N-hexylbenzamide
le 4-éthoxy-N-[4-(11β-fluoro-3,17β-dihydroxyestra-1,3,5(10)-trién-7α-yl)butyl]-N-octylbenzamide
le N-cyclohexylméthyl-N-[4- (11β-fluoro-3,17β-dihydroxyestra-1,3,5(10)-trién-7α-yl)butyl]dodécanamide
le N-benzyl-N-[4-(11β-fluoro-3,17β-dihydroxyestra-1,3,5(10)-trién-7α-yl)butyl]dodécanamide
le N- [4- (11β-fluoro-3,17β-dihydroxyestra-1,3,5(10)-trién-7α-yl)butyl]-N-(2-méthoxyéthyl)dodécanamide
le N-[4-(11β-fluoro-3,17β-dihydroxyestra-1,3,5(10)-trién-7α-yl)butyl]-N-(3-phénylpropyl)dodécanamide
le N-[4-(11β-fluoro-3,17β-dihydroxyestra-1,3,5(10)-trién-7α-yl)butyl]-N-hexyldodécanamide
le N-[4-(11β-fluoro-3,17β-dihydroxyestra-1,3,5(10)-trién-7α-yl)butyl]-N-octyldodécanamide
le N-cyclohexylméthyl-N-[4-(11β-fluoro-3,17β-dihydroxyestra-1,3,5(10)-trién-7α-yl)butyl]napht-2-ylacétamide
le N-benzyl-N-[4-(11β-fluoro-3,17β-dihydroxyestra-1,3,5(10)-trién-7α-yl)butyl]napht-2-ylacétamide
le N-[4-(11β-fluoro-3,17β-dihydroxyestra-1,3,5(10)-trién-7α-yl)butyl]-N-(2-méthoxyéthyl)napht-2-ylacétamide
le N-[4-(11β-fluoro-3,17β-dihydroxyestra-1,3,5(10)-trién-7α-yl)butyl]napht-2-yl-N-(3-phénylpropyl)acétamide
le N-[4-(11β-fluoro-3,17β-dihydroxyestra-1,3,5(10)-trién-7α-yl)butyl]-N-hexylnapht-2-ylacétamide
le N-[4-(11β-fluoro-3,17β-dihydroxyestra-1,3,5(10)-trién-7α-yl)butyl]napht-2-yl-N-octylacétamide
le N-cyclohexylméthyl-N-[4-(11β-fluoro-3,17β-dihydroxyestra-1,3,5(10)-trién-7α-yl)butyl]-4-phénylbutyramide
le N-benzyl-N-[4-(11β-fluoro-3,17β-dihydroxyestra-1,3,5(10)-trién-7α-yl)butyl]-4-phénylbutyramide
le N- [4- (11β-fluoro-3,17β-dihydroxyestra-1,3,5(10)-trién-7α-yl)butyl]-N-(2-méthoxyéthyl)-4-phénylbutyramide
le N-[4-(11β-fluoro-3,17β-dihydroxyestra-1,3,5(10)-trién-7α-yl)butyl]-4-phényl-N-(3-phénylpropyl)butyramide
le N-[4-(11β-fluoro-3,17β-dihydroxyestra-1,3,5(10)-trién-7α-yl)butyl]-N-hexyl-4-phénylbutyramide
le N-[4-(11β-fluoro-3,17β-dihydroxyestra-1,3,5(10)-trién-7α-yl)butyl]-N-octyl-4-phénylbutyramide
le N-cyclohexylméthyl-N-[4-(11β-fluoro-3,17β-dihydroxyestra-1,3,5(10)-trién-7α-yl)butyl]propionamide
le N-benzyl-N-[4-(11β-fluoro-3,17β-dihydroxyestra-1,3,5(10)-trién-7α-yl)butyl]propionamide
le N-[4-(11β-fluoro-3,17β-dihydroxyestra-1,3,5(10)-trién-7α-yl)butyl]-N-(2-méthoxyéthyl)propionamide
le N-[4-(11β-fluoro-3,17β-dihydroxyestra-1,3,5(10)-trién-7α-yl)butyl]-N-(3-phénylpropyl)propionamide
le N-[4-(11β-fluoro-3,17β-dihydroxyestra-1,3,5(10)-trién-7α-yl)butyl]-N-hexylpropionamide
le N-[4-(11β-fluoro-3,17β-dihydroxyestra-1,3,5(10)-trién-7α-yl)butyl]-N-octylpropionamide
le 4-biphényl-N-cyclohexylméthyl-N-[4-(11β-fluoro-3,17β-dihydroxyestra-1,3,5(10)-trién-7α-yl)butyl]acétamide
le 4-biphényl-N-benzyl-N-[4-(11β-fluoro-3,17β-dihydroxyestra-1,3,5(10)-trién-7α-yl)butyl]acétamide
le 4-biphényl-N-[4- (11β-fluoro-3,17β-dihydroxyestra-1,3,5(10)-trién-7α-yl)butyl]-N-(2-méthoxyéthyl)acétamide
le 4-biphényl-N-[4-(11β-fluoro-3,17β-dihydroxyestra-1,3,5(10)-trién-7α-yl)butyl]-N-(3-phénylpropyl)acétamide
le 4-biphényl-N- [4- (11β-fluoro-3,17β-dihydroxyestra-1,3,5(10)-trién-7α-yl)butyl]-N-hexylacétamide
le 4-biphényl-N-[4-(11β-fluoro-3,17β-dihydroxyestra-1,3,5(10)-trién-7α-yl)butyl]-N-octylacétamide

le 4-acétylamino-N-cyclohexylméthyl-N-[5-(11β-fluoro-3,17β-dihydroxyestra-1,3,5(10)-trién-7α-yl)pentyl]benzamide
le 4-acétylamino-N-benzyl-N-[5-(11β-fluoro-3,17β-dihydroxyestra-1, 3,5(10)-trién-7α-yl)pentyl]benzamide
le 4-acétylamino-N-[5-(11β-fluoro-3,17β-dihydroxyestra-1,3,5(10)-trién-7α-yl)pentyl]-N-(2-méthoxyéthyl)benzamide
le 4-acétylamino-N-[5-(11β-fluoro-3,17β-dihydroxyestra-1,3,5(10)-trién-7α-yl)pentyl]-N-(3-phénylpropyl)benzamide
le 4-acétylamino-N-[5-(11β-fluoro-3,17β-dihydroxyestra-1,3,5(10)-trién-7α-yl)pentyl]-N-hexylbenzamide
le 4-acétylamino-N-[5-(11β-fluoro-3,17β-dihydroxyestra-1,3,5(10)-trién-7α-yl)pentyl]-N-octylbenzamide
le 4-cyano-N-cyclohexylméthyl-N-[5-(11β-fluoro-3,17β-dihydroxyestra-1,3,5(10)-trién-7α-yl)pentyl]benzamide
le N-benzyl-4-cyano-N-[5-(11β-fluoro-3,17β-dihydroxyestra-1,3,5(10)-trién-7α-yl)pentyl]benzamide
le 4-cyano-N-[5-(11β-fluoro-3,17β-dihydroxyestra-1,3,5(10)-trién-7α-yl)pentyl]-N-(2-méthoxyéthyl)benzamide
le 4-cyano-N-[5-(11β-fluoro-3,17β-dihydroxyestra-1,3,5(10)-trién-7α-yl)pentyl]-N-(3-phénylpropyl)benzamide
le 4-cyano-N-[5-(11β-fluoro-3,17β-dihydroxyestra-1,3,5(10)-trién-7α-yl)pentyl]-N-hexylbenzamide
le 4-cyano-N-[5-(11β-fluoro-3,17β-dihydroxyestra-1,3,5(10)-trién-7α-yl)pentyl]-N-octylbenzamide
le 3-cyclohexyl-N-cyclohexylméthyl-N-[5-(11β-fluoro-3,17β-dihydroxyestra-1,3,5(10)-trién-7α-yl)pentyl]propionamide
le N-benzyl-3-cyclohexyl-N-[5-(11β-fluoro-3,17β-dihydroxyestra-1,3,5(10)-trién-7α-yl)pentyl]propionamide
le 3-cyclohexyl-N-[5-(11β-fluoro-3,17β-dihydroxyestra-1,3,5(10)-trién-7α-yl)pentyl]-N- (2-méthoxyéthyl)propionamide
le 3-cyclohexyl-N-[5-(11β-fluoro-3,17β-dihydroxyestra-1,3,5(10)-trién-7α-yl)pentyl]-N-(3-phénylpropyl)propionamide
le 3-cyclohexyl-N-[5-(11β-fluoro-3,17β-dihydroxyestra-1,3,5(10)-trién-7α-yl)pentyl]-N-hexylpropionamide
le 3-cyclohexyl-N-[5-(11β-fluoro-3,17β-dihydroxyestra-1,3,5(10)-trién-7α-yl)pentyl]-N-octylpropionamide
le N-cyclohexylméthyl-N-[5-(11β-fluoro-3,17β-dihydroxyestra-1,3,5(10)-trién-7α-yl)pentyl]-(3,4-diméthoxyphényl)acétamide
le N-benzyl-N-[5-(11β-fluoro-3,17β-dihydroxyestra-1,3,5(10)-trién-7α-yl)pentyl]-(3,4-diméthoxyphényl)acétamide
leN-[5-(11β-fluoro-3,17β-dihydroxyestra-1,3,5(10)-trién-7α-yl)pentyl]-N-(2-méthoxyéthyl)-(3,4-diméthoxyphényl)acétamide
le N-[5-(11β-fluoro-3,17β-dihydroxyestra-1,3,5(10)-trién-7α-yl)pentyl]-(3,4-diméthoxyphényl)-N-(3-phénylpropyl)acétamide
le N-[5-(11β-fluoro-3,17β-dihydroxyestra-1,3,5(10)-trién-7α-yl)pentyl]-N-hexyl-(3,4-diméthoxyphényl)acétamide
le N-[5-(11β-fluoro-3,17β-dihydroxyestra-1,3,5(10)-trién-7α-yl)pentyl]-(3,4-diméthoxyphényl)-N-octylacétamide
le N-cyclohexylméthyl-4-éthoxy-N-[5-(11β-fluoro-3,17β-dihydroxyestra-1,3,5 (10) -trién-7α-yl)pentyl]benzamide
le N-benzyl-4-éthoxy-N-[5-(11β-fluoro-3,17β-dihydroxyestra-1,3,5(10)-trién-7α-yl)pentyl]benzamide
le 4-éthoxy-N- [5- (11β-fluoro-3,17β-dihydroxyestra-1,3,5(10)-trién-7α-yl)pentyl]-N-(2-méthoxyéthyl)benzamide
le 4-éthoxy-N-[5-(11β-fluoro-3,17β-dihydroxyestra-1,3,5(10)-trién-7α-yl)pentyl]-N-(3-phénylpropyl)benzamide
le 4-éthoxy-N-[5-(11β-fluoro-3,17β-dihydroxyestra-1,3,5(10)-trién-7α-yl)pentyl]-N-hexylbenzamide
le 4-éthoxy-N-[5-(11β-fluoro-3,17β-dihydroxyestra-1,3,5(10)-trién-7α-yl)pentyl]-N-octylbenzamide
le N-cyclohexylméthyl-N-[5-(11β-fluoro-3,17β-dihydroxyestra-1,3,5(10)-trién-7α-yl)pentyl]dodécanamide
le N-benzyl-N- [5- (11β-fluoro-3,17β-dihydroxyestra-1,3,5(10)-trién-7α-yl)pentyl]dodécanamide
1e N-[5-(11β-fluoro-3,17β-dihydroxyestra-1,3,5(10)-trién-7α-yl)pentyl]-N-(2-méthoxyéthyl)dodécanamide
le N-[5-(11β-fluoro-3,17β-dihydroxyestra-1,3,5(10)-trién-7α-yl)pentyl]-N-(3-phénylpropyl)dodécanamide
le N-[5-(11β-fluoro-3,17β-dihydroxyestra-1,3,5(10)-trién-7α-yl)pentyl]-N-hexyldodécanamide
le N-[5-(11β-fluoro-3,17β-dihydroxyestra-1,3,5(10)-trién-7α-yl)pentyl]-N-octyldodécanamide
le N-cyclohexylméthyl-N-[5-(11β-fluoro-3,17β-dihydroxyestra-1,3,5(10)-trién-7α-yl)pentyl]napht-2-ylacétamide
le N-benzyl-N-[5-(11β-fluoro-3,17β-dihydroxyestra-1,3,5(10)-trién-7α-yl)pentyl]napht-2-ylacétamide
le N-[5-(11β-fluoro-3,17β-dihydroxyestra-1,3,5(10)-trién-7α-yl)pentyl]-N-(2-méthoxyéthyl)napht-2-ylacétamide

le N- [5- (11β-fluoro-3,17β-dihydroxyestra-1,3,5(10)-trién-7α-yl)pentyl]napht-2-yl-N-(3-phénylpropyl)acétamide
le N- [5- (11β-fluoro-3,17β-dihydroxyestra-1,3,5(10)-trién-7α-yl)pentyl]-N-hexylnapht-2-ylacétamide
le N-[5-(11β-fluoro-3,17β-dihydroxyestra-1,3,5(10)-trién-7α-yl)pentyl]napht-2-yl-N-octylacétamide
le N-cyclohexylméthyl-N-[5-(11β-fluoro-3,17β-dihydroxyestra-1,3,5(10)-trién-7α-yl)pentyl]-4-phénylbutyramide
le N-benzyl-N-[5-(11β-fluoro-3,17β-dihydroxyestra-1,3,5(10)-trién-7α-yl)pentyl]-4-phénylbutyramide
le N-[5-(11β-fluoro-3,17β-dihydroxyestra-1,3,5(10)-trién-7α-yl)pentyl]-N-(2-méthoxyéthyl)-4-phénylbutyramide
le N-[5-(11β-fluoro-3,17β-dihydroxyestra-1,3,5(10)-trién-7α-yl)pentyl]-4-phényl-N-(3-phénylpropyl)butyramide
le N-[5-(11β-fluoro-3,17β-dihydroxyestra-1,3,5(10)-trién-7α-yl)pentyl]-N-hexyl-4-phénylbutyramide
le N-[5-(11β-fluoro-3,17β-dihydroxyestra-1,3,5(10)-trién-7α-yl)pentyl]-N-octyl-4-phénylbutyramide
le N-cyclohexylméthyl-N-[5-(11β-fluoro-3,17β-dihydroxyestra-1,3,5(10)-trién-7α-yl)pentyl]propionamide
le N-benzyl-N-[5-(11β-fluoro-3,17β-dihydroxyestra-1,3,5(10)-trién-7α-yl)pentyl]propionamide
le N-[5-(11β-fluoro-3,17β-dihydroxyestra-1,3,5(10)-trién-7α-yl)pentyl]-N-(2-méthoxyéthyl)propionamide
le N-[5-(11β-fluoro-3,17β-dihydroxyestra-1,3,5(10)-trién-7α-yl)pentyl]-N-(3-phénylpropyl)propionamide
le N-[5-(11β-fluoro-3,17β-dihydroxyestra-1,3,5(10)-trién-7α-yl)pentyl]-N-hexylpropionamide
le N-[5-(11β-fluoro-3,17β-dihydroxyestra-1,3,5(10)-trién-7α-yl)pentyl]-N-octylpropionamide
le 4-biphényl-N-cyclohexylméthyl-N-[5-(11β-fluoro-3,17β-dihydroxyestra-1,3,5(10)-trién-7α-yl)pentyl]acétamide
le 4-biphényl-N-benzyl-N-[5-(11β-fluoro-3,17β-dihydroxyestra-1,3,5(10)-trién-7α-yl)pentyl]acétamide
le 4-biphényl-N-[5-(11β-fluoro-3,17β-dihydroxyestra-1,3,5(10)-trién-7α-yl)pentyl]-N-(2-méthoxyéthyl)acétamide
le 4-biphényl-N-[5-(11β-fluoro-3,17β-dihydroxyestra-1,3,5(10)-trién-7α-yl)pentyl]-N-(3-phénylpropyl)acétamide
le 4-biphényl-N-[5-(11β-fluoro-3,17β-dihydroxyestra-1,3,5(10)-trién-7α-yl)pentyl]-N-hexylacétamide
le 4-biphényl-N-[5-(11β-fluoro-3,17β-dihydroxyestra-1,3,5(10)-trién-7α-yl)pentyl]-N-octylacétamide
le 4-acétylamino-N-cyclohexylméthyl-N-[6-(11β-fluoro-3,17β-dihydroxyestra-1,3, 5 (10)-trién-7α-yl)hexyl]benzarnide
le 4-acétylamino-N-benzyl-N-[6-(11β-fluoro-3,17β-dihydroxyestra-1,3,5(10)-trién-7α-yl)hexyl]benzamide
le 4-acétylamino-N-[6-(11β-fluoro-3,17β-dihydroxyestra-1,3,5(10)-trién-7α-yl)hexyl]-N-(2-méthoxyéthyl) benzamide
le 4-acétylamino-N- [6- (11β-fluoro-3,17β-dihydroxyestra-1,3,5(10)-trién-7α-yl)hexyl]-N-(3-phénylpropyl)benzamide
le 4-acétylamino-N-[6-(11β-fluoro-3,17β-dihydroxyestra-1,3,5(10)-trién-7α-yl)hexyl]-N-hexylbenzamide
le 4-acétylamino-N-[6-(11β-fluoro-3,17β-dihydroxyestra-1,3,5(10)-trién-7α-yl)hexyl]-N-octylbenzamide
le 4-cyano-N-cyclohexylméthyl-N-[6-(11β-fluoro-3,17β-dihydroxyestra-1,3,5(10)-trién-7α-yl)hexyl]benzamide
le N-benzyl-4-cyano-N-[6-(11β-fluoro-3,17β-dihydroxyestra-1,3,5(10)-trién-7α-yl)hexyl]benzamide
le 4-cyano-N-[6-(11β-fluoro-3,17β-dihydroxyestra-1,3,5(10)-trién-7α-yl)hexyl]-N-(2-méthoxyéthyl)benzamide
le 4-cyano-N-[6-(11β-fluoro-3,17β-dihydroxyestra-1,3,5(10)-trién-7α-yl)hexyl]-N-(3-phénylpropyl)benzamide
le 4-cyano-N-[6-(11β-fluoro-3,17β-dihydroxyestra-1,3,5(10)-trién-7α-yl)hexyl]-N-hexylbenzamide
le 4-cyano-N-[6-(11β-fluoro-3,17β-dihydroxyestra-1,3,5(10)-trién-7α-yl)hexyl]-N-octylbenzamide
le 3-cyclohexyl-N-cyclohexylméthyl-N-[6-(11β-fluoro-3,17β-dihydroxyestra-1,3,5(10)-trién-7α-yl)hexyl]propionamide
le N-benzyl-3-cyclohexyl-N-[6-(11β-fluoro-3,17β-dihydroxyestra-1,3,5(10)-trién-7α-yl)hexyl]propionamide
le 3-cyclohexyl-N-[6-(11β-fluoro-3,17β-dihydroxyestra-1,3,5(10)-trién-7α-yl)hexyl]-N-(2-méthoxyéthyl)propionamide
le 3-cyclohexyl-N-[6-(11β-fluoro-3,17β-dihydroxyestra-1,3,5(10)-trién-7α-yl)hexyl]-N-(3-phénylpropyl)propionamide
le 3-cyclohexyl-N- [6- (11β-fluoro-3,17β-dihydroxyestra-1,3,5(10)-trién-7α-yl)hexyl]-N-hexylpropionamide
le 3-cyclohexyl-N-[6-(11β-fluoro-3,17β-dihydroxyestra-1,3,5(10)-trién-7α-yl)hexyl]-N-octylpropionamide
le N-cyclohexylméthyl-N-[6-(11β-fluoro-3, 17β-dihydroxyestra-1,3,5(10)-trién-7α-yl)hexyl]-(3,4-diméthoxyphényl)acétamide
le N-benzyl-N-[6-(11β-fluoro-3,17β-dihydroxyestra-1,3,5(10)-trién-7α-yl)hexyl]-(3,4-diméthoxyphényl)acétamide
le N-[6-(11β-fluoro-3,17β-dihydroxyestra-1,3,5(10)-trién-7α-yl)hexyl]-N-(2-méthoxyéthyl)-(3,4-diméthoxyphényl)acétamide

le N-[6-(11β-fluoro-3,17β-dihydroxyestra-1,3,5(10)-trién-7α-yl)hexyl]-N-(3,4-diméthoxyphényl)-N-(3-phénylpropyl)acétamide
le N-[6-(11β-fluoro-3,17β-dihydroxyestra-1,3,5(10)-trién-7α-yl)hexyl]-N-hexyl-(3,4-diméthoxyphényl)acétamide
le N-[6-(11β-fluoro-3,17β-dihydroxyestra-1,3,5(10)-trién-7α-yl)hexyl]-(3,4-diméthoxyphényl)-N-octylacétamide
le N-cyclohexylméthyl-4-éthoxy-N-[6-(11β-fluoro-3,17β-dihydroxyestra-1,3,5(10)-trién-7α-yl)hexyl]benzamide
le N-benzyl-4-éthoxy-N-[6-(11β-fluoro-3,17β-dihydroxyestra-1,3,5(10)-trién-7α-yl)hexyl]benzamide
le 4-éthoxy-N-[6-(11β-fluoro-3,17β-dihydroxyestra-1,3,5(10)-trién-7α-yl)hexyl]-N-(2-méthoxyéthyl)benzamide
le 4-éthoxy-N-[6-(11β-fluoro-3,17β-dihydroxyestra-1,3,5(10)-trién-7α-yl)hexyl]-N-(3-phénylpropyl)benzamide
le 4-éthoxy-N-[6-(11β-fluoro-3,17β-dihydroxyestra-1,3,5(10)-trién-7α-yl)hexyl]-N-hexylbenzamide
le 4-éthoxy-N- [6-(11β-fluoro-3,17β-dihydroxyestra-1,3,5(10)-trién-7α-yl)hexyl]-N-octylbenzamide
le N-cyclohexylméthyl-N-[6-(11β-fluoro-3,17β-dihydroxyestra-1,3,5(10)-trién-7α-yl)hexyl]dodécanamide
le N-benzyl-N-[6-(11β-fluoro-3,17β-dihydroxyestra-1,3,5(10)-trién-7α-yl)hexyl]dodécanamide
le N-[6-(11β-fluoro-3,17β-dihydroxyestra-1,3,5(10)-trién-7α-yl)hexyl]-N-(2-méthoxyéthyl)dodécanamide
le N-[6-(11β-fluoro-3,17β-dihydroxyestra-1,3,5(10)-trién-7α-yl)hexyl]-N-(3-phénylpropyl)dodécanamide
le N-[6-(11β-fluoro-3,17β-dihydroxyestra-1,3,5(10)-trién-7α-yl)hexyl]-N-hexyldodécanamide
le N-[6-(11β-fluoro-3,17β-dihydroxyestra-1,3,5(10)-trién-7α-yl)hexyl]-N-octyldodécanamide
le N-cyclohexylméthyl-N-[6-(11β-fluoro-3,17β-dihydroxyestra-1,3,5(10)-trién-7α-yl)hexyl]napht-2-ylacétamide
le N-benzyl-N-[6-(11β-fluoro-3,17β-dihydroxyestra-1,3,5(10)-trién-7α-yl)hexyl]napht-2-ylacétamide
le N-[6-(11β-fluoro-3,17β-dihydroxyestra-1,3,5(10)-trién-7α-yl)hexyl]-N-(2-méthoxyéthyl)napht-2-ylacétamide
le N-[6-(11β-fluoro-3,17β-dihydroxyestra-1,3,5(10)-trién-7α-yl)hexyl]napht-2-yl-N-(3-phénylpropyl)acétamide
le N-[6-(11β-fluoro-3,17β-dihydroxyestra-1,3,5(10)-trién-7α-yl)hexyl]-N-hexylnapht-2-ylacétamide
le N-[6-(11β-fluoro-3,17β-dihydroxyestra-1,3,5(10)-trién-7α-yl)hexyl]napht-2-yl-N-octylacétamide
le N-cyclohexylméthyl-N- [6-(11β-fluoro-3,17β-dihydroxyestra-1,3,5(10)-trién-7α-yl)hexyl]-4-phénylbutyramide
le N-benzyl-N-[6-(11β-fluoro-3,17β-dihydroxyestra-1,3,5(10)-trién-7α-yl)hexyl]-4-phénylbutyramide
le N-[6-(11β-fluoro-3,17β-dihydroxyestra-1,3,5(10)-trién-7α-yl)hexyl]-N-(2-méthoxyéthyl)-4-phénylbutyramide
le N- [6-(11β-fluoro-3,17β-dihydroxyestra-1,3,5(10)-trién-7α-yl)hexyl]-4-phényl-N-(3-phénylpropyl)butyramide
le N-[6-(11β-fluoro-3,17β-dihydroxyestra-1,3,5(10)-trién-7α-yl)hexyl]-N-hexyl-4-phénylbutyramide
le N-[6-(11β-fluoro-3,17β-dihydroxyestra-1,3,5(10)-trién-7α-yl)hexyl]-N-octyl-4-phénylbutyramide
le 9-N-cyclohexylméthyl-N-[6-(11β-fluoro-3,17β-dihydroxyestra-1,3,5(10) -trién-7α-yl)hexyl]propionamide
le N-benzyl-N-[6-(11β-fluoro-3,17β-dihydroxyestra-1,3,5(10)-trién-7α-yl)hexyl]propionamide
le N-[6-(11β-fluoro-3,17β-dihydroxyestra-1,3,5(10)-trién-7α-yl)hexyl]-N-(2-méthoxyéthyl)propionamide
le N-[6-(11β-fluoro-3,17β-dihydroxyestra-1,3,5(10)-trién-7α-yl)hexyl]-N-(3-phénylpropyl)propionamide
le N-[6-(11β-fluoro-3,17β-dihydroxyestra-1,3,5(10)-trién-7α-yl)hexyl]-N-hexylpropionamide
le N-[6-(11β-fluoro-3,17β-dihydroxyestra-1,3,5(10)-trién-7α-yl)hexyl]-N-octylpropionamide
le 4-biphényl-N-cyclohexylméthyl-N-[6-(11β-fluoro-3,17β-dihydroxyestra-1,3,5(10)-trién-7α-yl)hexyl]acétamide
le 4-biphényl-N-benzyl-N-[6-(11β-fluoro-3,17β-dihydroxyestra-1,3,5(10)-trién-7α-yl)hexyl]acétamide
le 4-biphényl-N-[6-(11β-fluoro-3,17β-dihydroxyestra-1,3,5(10)-trién-7α-yl)hexyl]-N-(2-méthoxyéthyl)acétamide
le 4-biphényl-N-[6-(11β-fluoro-3,17β-dihydroxyestra-1,3,5(10)-trién-7α-yl)hexyl]-N-(3-phénylpropyl)acétamide
le 4-biphényl-N-[6-(11β-fluoro-3,17β-dihydroxyestra-1,3,5(10)-trién-7α-yl)hexyl]-N-hexylacétamide
le 4-biphényl-N-[6-(11β-fluoro-3,17β-dihydroxyestra-1,3,5(10)-trién-7α-yl)hexyl]-N-octylacétamide

le 11β-fluoro-7α-{5-[méthyl(2-p-tolyléthyl)amino]pentyl}estra-1,3,5(10)-triène-3,17β-diol
le 7α-(5-{[2-(4-éthoxyphényl)éthyl]méthylamino}pentyl)-11β-fluoroestra-1,3,5(10)-triène-3,17β-diol
le 11β-fluoro-7α-{5-[méthyl(3-phénylpropyl)amino]pentyl}estra-1,3,5(10)-triène-3,17β-diol
le 11β-fluoro-7α-{5-[méthyl(3-pyridin-3-ylpropyl)amino]pentyl}estra-1,3,5(10)-triène-3,17β-diol
le 11β-fluoro-7α-{5-[méthyl(3-p-tolylpropyl)amino]pentyl}estra-1,3,5(10)-triène-3,17β-diol

le 7α-(5-{[3-(4-chlorophényl)propyl]méthylamino}pentyl)-11β-fluoroestra-1,3,5(10)-triène-3,17β-diol
le 7α-(5-{[3-(4-éthoxyphényl)propyl]méthylamino}pentyl)-11β-fluoroestra-1,3,5(10)-triène-3,17β-diol
le 11β-fluoro-7α-{5-[méthyl (4-méthylpentyl)amino]pentyl}estra-1,3,5(10)-triène-3,17β-diol.

**12.** Utilisation des composés de formule générale I selon la revendication 1 pour la préparation de médicaments.

**13.** Préparations pharmaceutiques comprenant au moins un composé de formule générale I selon la revendication 1 ainsi qu'un support pharmaceutiquement acceptable.

**Claims**

**1.** 11β-Halogen-7α-substituted estratrienes of general formula I

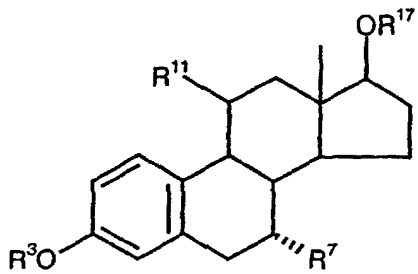

(I)

in which

$R^3$ means a hydrogen atom, a hydrocarbon radical with up to 8 carbon atoms or a radical of partial formula $R^{3'}$-C(O)-, in which $R^{3'}$ means a hydrogen atom or a hydrocarbon radical with up to 8 carbon atoms or a phenyl radical,

$R^7$ means a radical of formula -A-B-Z-$R^{20}$, in which

A stands for a direct bond or a benzylidene radical, where the methylene group is bonded to the 7-carbon atom of the steroid, or a phenylene radical,

B stands for a straight-chain or branched-chain alkylene, alkenylene or alkynylene group with 3 to 14 carbon atoms, and

Z stands for -$NR^{21}$- and $R^{21}$ stands for a $C_1$-$C_3$ alkyl group,

in which case $R^{20}$ means

a hydrogen atom,

a straight-chain or branched-chain alkyl, alkenyl or alkynyl group with up to 10 carbon atoms or one of the groupings

-D-$C_nF_{2n+1}$, where D is a straight-chain or branched-chain alkylene, alkenylene or alkynylene group with up to 8 carbon atoms and n is an integer from 1 to 8,

-L-CH=CF-$C_pF_{2p+1}$, where 1 is a straight-chain or branched-chain alkylene, alkenylene or alkynylene group with 2 to 7 carbon atoms and p is an integer from 2 to 7,

-D-O-$(CH_2)_q$-aryl, where D has the already indicated meaning, q is 0, 1, 2 or 3, and aryl stands for a phenyl radical, 1-or 2-naphthyl radical or a heteroaryl radical that is optionally substituted once or twice,

-D-O-$(CH_2)_r$-$C_nF_{2n+1}$, where D and n have the already indicated meanings and r stands for an integer from 1 to 5, or

$R^{20}$ and $R^{21}$ with the nitrogen atom to which they are bonded form a saturated or unsaturated heterocycle with 5 or 6 chain links, which optionally contains one or two additional heteroatoms, selected from nitrogen, oxygen and sulphur, and optionally is substituted, or

Z stands for $-SO_x-$ and x stands for 0, 1 or 2, in which case $R^{20}$ means

a straight-chain or branched-chain alkyl, alkenyl or alkynyl group with up to 10 carbon atoms, or one of the groupings
$-D-C_nF_{2n+1}$, where D is a straight-chain or branched-chain alkylene, alkenylene or alkynylene group with up to 8 carbon atoms and n is an integer from 1 to 8,
$-L-CH=CF-C_pF_{2p+1}$, where 1 is a straight-chain or branched-chain alkylene, alkenylene or alkynylene group with 2 to 7 carbon atoms and p is an integer from 2 to 7,
$-D-O-(CH_2)_q$-aryl, where D has the already indicated meaning, q is 0, 1, 2 or 3, and aryl stands for a phenyl radical, 1-or 2-naphthyl radical or a heteroaryl radical optionally substituted once or twice,
$-D-O-(CH_2)_r-C_nF_{2n+1}$, where D and n have the already indicated meanings and r stands for an integer from 1 to 5, or

Z stands for $-NR^{31}$, in which case $R^{20}$ is a straight-chain or branched-chain alkyl, alkenyl or alkynyl radical with up to 14 carbon atoms, which can be interrupted and/or partially fluorinated by one to three heteroatoms -O- and -S- and groupings - $NR^{32}$-, in which $R^{32}$ is a hydrogen atom or a $C_1$-$C_3$ alkyl radical, an aryl or heteroaryl radical that is optionally substituted once or twice, a $C_3$-$C_{10}$ cycloalkyl radical that is optionally substituted once or twice, a $C_4$-$C_{15}$ cycloalkylalkyl radical that is optionally substituted once or twice, a $C_7$-$C_{20}$ aralkyl radical that is optionally substituted once or twice, a heteroaryl-$C_1$-$C_6$ alkyl radical that is optionally substituted once or twice or an optionally substituted aminoalkyl radical, and
$R^{31}$ is a radical of formula $-C(O)R^{33}$ or $-CH_2-R^{33}$,
in which case $R^{33}$ is a straight-chain or branched-chain, alkyl, alkenyl or alkynyl radical with up to 14 carbon atoms, which can be interrupted and/or partially fluorinated by one to three heteroatoms -O- and -S- and groupings $-NR^{32}$-, in which $R^{32}$ is a hydrogen atom or a $C_1$-$C_3$ alkyl radical, an aryl or heteroaryl radical that is optionally substituted once or twice, a $C_3$-$C_{10}$ cycloalkyl radical that is optionally substituted once or twice, a $C_4$-$C_{15}$ cycloalkylalkyl radical that is optionally substituted once or twice, a $C_7$-$C_{20}$ aralkyl radical that is optionally substituted once or twice, a heteroaryl-$C_1$-$C_6$ alkyl radical that is optionally substituted once or twice, an optionally substituted aminoalkyl radical or a biphenylyl radical,

excluding the compounds

11β-fluoro-7α-{5-[N-methyl-N-3-(4,4,5,5,5-pentafluoropentylthio)-propylamino]-pentyl}-estra-1,3,5(10)-triene-3,17β-diol
11β-fluoro-7α-{5-[2-(4,4,5,5,5-pentafluoropentylthiomethyl)-pyrrolidin-1-yl]-pentyl}-estra-1,3,5(10)-triene-3,17β-diol
11β-fluoro-7α-{6-[N-methyl-N-3- (4,4,5,5, 5-pentafluoropentylthio)-propylamino]-hexyl}-estra-1,3,5(10)-triene-3,17β-diol
11β-fluoro-7α-{5-[(2S)-2-(4-trifluoromethylphenylthiomethyl)-pyrrolidin-1-yl]-pentyl}-estra-1,3,5(10)-triene-3,17β-diol
11β-fluoro-7α-{5-[(2S)-2-(4,4,5,5,5-pentafluoropentylthiomethyl)-pyrrolidin-1-yl]-pentyl}-estra-1,3,5(10)-triene-3,17β-diol
11β-fluoro-7α-{5-[(2S)-2-(4,4,5,5,5-pentafluoropentanesulphinylmethyl)-pyrrolidin-1-yl]-pentyl}-estra-1,3,5(10)-triene-3,17β-diol
11β-fluoro-7α-{5-[(2S)-2-(4,4,5,5,5-pentafluoropentanesulphonylmethyl)-pyrrolidin-1-yl]-pentyl}-estra-1,3,5(10)-triene-3,17β-diol

$R^{11}$ means a fluorine or chlorine atom,
$R^{17}$ means a hydrogen atom or a radical of partial formula $R^{17'}$-C(O)-, in which $R^{17'}$ is a hydrogen atom or a hydrocarbon radical with up to 8 carbon atoms.

**2.** 11β-Halogen-7α-substituted estratrienes according to Claim 1, in which $R^3$ is a hydrogen atom.

**3.** 11β-Halogen-7α-substituted estratrienes according to Claim 1, in which $R^3$ is a benzoyl radical.

**4.** 11β-Halogen-7α-substituted estratrienes according to Claim 1, in which $R^{11}$ is a fluorine atom.

**5.** 11β-Halogen-7α-substituted estratrienes according to Claim 1, in which $R^{17}$ is a hydrogen atom.

**6.** 11β-Halogen-7α-substituted estratrienes according to Claim 1, in which A is a direct bond.

**7.** 11β-Halogen-7α-substituted estratrienes according to Claim 6, in which
a radical of formula $-(CH_2)_5-Z-R^{20}$,
where

s is an integer from 3 to 8,
Z stands for $-NR^{21}$ and $R^{21}$ stands for a $C_1$-$C_3$ alkyl group,

in which $R^{20}$ means

a hydrogen atom,
a $C_1$-$C_9$ alkyl group, or
one of the groupings
$-(CH_2)_m-C_nF_{2n+1}$, where m and n, independently of one another, in each case are an integer from 1 to 8,
$-(CH_2)_o-CH=CF-C_pF_{2p+1}$, where o is 1, 2 or 3 and p is an integer from 2 to 7,
$-(CH_2)_m-O-(CH_2)_q$-aryl, where m has the already indicated meaning, q is 0, 1, 2 or 3 and aryl stands for a phenyl or heteroaryl radical that is optionally substituted once or twice,
$-(CH_2)_m-O-(CH_2)_r-C_nF_{2n+1}$, where m and n have the already indicated meanings and r stands for an integer from 1 to 5, stands for $R^7$.

**8.** 11β-Halogen-7α-substituted estratrienes according to Claim 6, in which
a radical of formula $-(CH_2)_s-Z-R^{20}$,
where
s is an integer from 3 to 8,
Z stands for $-NR^{21}$ and $R^{21}$ stands for a $C_1$-$C_3$ alkyl group,
in which $R^{20}$ and $R^{21}$
with the nitrogen atom, to which they are bonded, form a saturated or unsaturated heterocycle with 5 or 6 chain links, which optionally contains one or two additional heteroatoms, selected from nitrogen, oxygen and sulphur, and optionally is substituted,
stands for $R^7$.

**9.** 11β-Halogen-7α-substituted estratrienes according to Claim 6, in which
a radical of formula$-(CH_2)_s-Z-R^{20}$,
where
s is an integer from 3 to 8,
Z stands for $-SO_x-$ and x stands for 0, 1 or 2, where $R^{20}$ means
$-(CH_2)_m-O-(CH_2)_r-C_nF_{2n+1}$, where m and n have the already indicated meanings and r stands for an integer from 1 to 5,
stands for $R^7$.

**10.** 11β-Halogen-7α-substituted estratrienes according to Claim 1, in which $R^7$ is selected from the group of the following side chains

$-(CH_2)_5N(CH_3)(CH_2)_3C_2F_5$

$-(CH_2)_5N(CH_3)(CH_2)_6C_2F_5$

$-(CH_2)_5N(CH_3)(CH_2)_7C_2F_5$

$-(CH_2)_5N(CH_3)(CH_2)_8C_2F_5$

$-(CH_2)_6N(CH_3)(CH_2)_6C_2F_5$

$-(CH_2)_6N(CH_3)(CH_2)_7C_2F_5$

$-(CH_2)_6N(CH_3)(CH_2)_8C_2F_5$

$-(CH_2)_5N(CH_3)(CH_2)_2C_4F_9$

$-(CH_2)_5N(CH_3)(CH_2)_3C_6F_{13}$

$-(CH_2)_5N(CH_3)(CH_2)_3C_8F_{17}$

$-(CH_2)_5N(CH_3)(CH_2)_6C_4F_9$

$-(CH_2)_5N(CH_3)(CH_2)_6C_6F_{13}$

$-(CH_2)_5N(CH_3)(CH_2)_6C_8F_{17}$

$-(CH_2)_5N(CH_3)H$

$-(CH_2)_5N(CH_3)(CH_2)_9H$

$-(CH_2)_5N(CH_3)CH_2CH{=}CF{-}C_2F_5$

$-(CH_2)_5N(CH_3)CH_2CH{=}CF{-}C_3F_7$

$-(CH_2)_5N(CH_3)CH_2CH{=}CF{-}C_5F_{11}$

$-(CH_2)_5N(CH_3)CH_2CH{=}CF{-}C_7F_{15}$

$-(CH_2)_5$-1-pyrrolidinyl

$-(CH_2)_5N(CH_3)(CH_2)_3$Ophenyl

$-(CH_2)_5N(CH_3)(CH_2)_3$Obenzyl

$-(CH_2)_5N(CH_3)(CH_2)_3O(CH_2)_3C_1F_5$

$-(CH_2)_9S(CH_2)_3C_2F_5$

$-(CH_2)_9SO(CH_2)_3C_2F_5$

$-(CH_2)_9SO_2(CH_2)_3C_2F_5$

$-(CH_2)_5N(CH_3)(CH_2)_3CH(CH_3)_2$

$-(CH_2)_5N(CH_3)(CH_2)_3$-pyridyl

$-(CH_2)_5N(CH_3)(CH_2)_3$-phenyl

$-(CH_2)_5N(CH_3)(CH_2)_2$-p-tolyl

$-(CH_2)_5N(CH_3)(CH_2)_2$-p-ethoxyphenyl

$-(CH_2)_5N(CH_3)(CH_2)_3$-p-tolyl

$-(CH_2)_5N(CH_3)(CH_2)_3$-p-chlorophenyl

$-(CH_2)_5N(CH_3)(CH_2)_3$-O-$CH_2$-phenyl

$-(CH_2)_5N(CH_3)(CH_2)_2$-O-p-Br-phenyl

$-(CH_2)_5N(CH_3)(CH_2)_2$-O-P-$CF_3$-phenyl

**11.** Compounds of general formula I, namely

11β-Fluoro-7α-{5-[methyl-(8,8,9,9,9-pentafluorononyl)amino]-pentyl)-estra-1,3,5(10)-triene-3,17β-diol
11β-Fluoro-7α-{5-[methyl-nonyl-amino]-pentyl}-estra-1,3,5(10)-triene-3,17β-diol
11β-Fluoro-7α-{5-[methyl-(9,9,10,10,10-pentafluoro-decyl)-amino]-pentyl}-estra-1,3,5(10)-triene-3,17β-diol
11β-Fluoro-7α-{6-[methyl-(8,8,9,9,9-pentafluorononyl)-amino]-hexyl}-estra-1,3,5(10)-triene-3,17β-diol
11β-Fluoro-7α-{6-[methyl-(9,9,10,10,10-pentafluoro-decyl)-amino]-hexyl}-estra-1,3,5(10)-triene-3,17β-diol
11β-Fluoro-7α-(5-(methyl-amino)-pentyl)-estra-1,3,5(10)-triene-3,17β-diol
11β-Fluoro-7α-(5-pyrrolidin-1-yl-pentyl)-estra-1,3,5(10)-triene-3,17β-diol
11β-Fluoro-7α-{5-[methyl-(4,4,5,5,5-pentafluoropentyl)-amino]-pentyl}-estra-1,3,5(10)-triene-3,17β-diol
11β-Fluoro-7α-{5-[methyl-(4,4,5,5,6,6,7,7,8,8,9,9,9-tridecafluoro-nonyl)-amino]-pentyl}-estra-1,3,5(10)-triene-3,17β-diol
11β-Fluoro-7α-{5-[(4,4,5,5,6,6,7,7,8,8,9,9,10,10,11,11,11-heptadecafluoro-undecyl)-methyl-amino]-pentyl}-estra-1,3,5(10)-triene-3,17β-diol
11β-Fluoro-7α-{5-[methyl-(3,3,4,4,5,5,6,6,6-nonafluoro-hexyl)-amino]-pentyl}-estra-1,3,5(10)-triene-3,17β-diol
11β-Fluoro-7α-{5-[methyl-(7,7,8,8,8-pentafluorooctyl)-amino]-pentyl}-estra-1,3,5(10)-triene-3,17β-diol
11β-Fluoro-7α-{6-[methyl-(7,7,8,8,8-pentafluorooctyl)-amino]-hexyl}-estra-1,3,5(10)-triene-3,17β-diol
11β-Fluoro-7α-{5-(methyl-(7,7,8,8,9,9,10,10,10-nonafluoro-decyl)-amino]-pentyl}-estra-1,3,5(10)-triene-3,17β-diol
11β-Fluoro-7α-{5-[methyl-(7,7,8,8,9,9,10,10,11,11,12,12,12-tridecafluorododecyl)-amino]-pentyl}-estra-1,3,5(10)-triene-3,17β-diol
11β-Fluoro-7α-{5-[(7,7,8,8,9,9,10,10,11,11, 12,12,13,13,14,14,14-heptadecafluoro-tetradecyl)-methyl-amino]-pentyl}-estra-1,3,5(10)-triene-3,17β-diol
11β-Fluoro-7α-{5-[(3,4,4,5,5,5-hexafluoro-pent-2-enyl)-methyl-amino]-pentyl}-estra-1,3,5(10)-triene-3,17β-diol
7α-{5-[(3,4,4,5,5,6,6,7,7,8,8,8-Dodecafluoro-oct-2-enyl)-methyl-amino]-pentyl}-11β-fluoro-estra-1,3,5(10)-triene-3,17β-diol
11β-Fluoro-7α-{5-[(3,4,4,5,5,6,6,7,7,8,8,9,9,10,10,10-hexadecafluorodec-2-enyl)-methyl-amino]-pentyl}-estra-1,3,5(10)-triene-3,17β-diol
11β-Fluoro-7α-{5-[methyl-(3-phenoxy-propyl)-amino]-pentyl}-estra-1,3,5(10)-triene-3,17β-diol
7α-{5-[(3-Benzyloxy-propyl)-methyl-amino]-pentyl}-11β-fluoro-estra-1,3,5(10)-triene-3,17β-diol
11β-Fluoro-7α-{5-[N-methyl-N-3-(4,4,5,5,5-pentafluoropentyloxy)-propylamino]-pentyl}-estra-1,3,5(10)-triene-3,17β-diol
11β-Fluoro-7α-[9-(4,4,5,5,5-pentafluoropentylsulphinyl)-nonyl]-estra-1,3,5(10)-triene-3,17β-diol
N-[4-(11β-Fluoro-3,17-dihydroxy-estra-1,3,5(10)-trien-7α-yl)-butyl]-N-pentyl-benzamide
4-Acetylamino-N-cyclohexylmethyl-N-[4-(11β-fluoro-3,17β-dihydroxy-estra-1,3,5(10)-trien-7α-yl)-butyl]-benzamide
4-Acetylamino-N-benzyl-N-[4-(11β-fluoro-3,17β-dihydroxy-estra-1,3,5(10)-trien-7α-yl)-butyl]-benzamide
4-Acetylamino-N-[4-(11β-fluoro-3,17β-dihydroxyestra-1,3,5(10)-trien-7α-yl)-butyl]-N-(2-methoxyethyl)-benzamide
4-Acetylamino-N-[4-(11β-fluoro-3,17β-dihydroxyestra-1,3,5(10)-trien-7α-yl)-butyl]-N-(3-phenylpropyl)-benzamide
4-Acetylamino-N-[4-(11β-fluoro-3,17β-dihydroxyestra-1,3,5(10)-trien-7α-yl)-butyl]-N-hexyl-benzamide
4-Acetylamino-N-[4-(11β-fluoro-3,17β-dihydroxyestra-1,3,5(10)-trien-7α-yl)-butyl]-N-octyl-benzamide
4-Cyano-N-cyclohexylmethyl-N-[4-(11β-fluoro-3,17β-dihydroxy-estra-1,3,5(10)-trien-7α-yl)-butyl]-benzamide
N-Benzyl-4-cyano-N-[4-(11β-fluoro-3,17β-dihydroxyestra-1,3,5(10)-trien-7α-yl)-butyl]-benzamide
4-Cyano-N-[4-(11β-fluoro-3,17β-dihydroxy-estra-1,3,5(10)-trien-7α-yl)-butyl]-N-(2-methoxy-ethyl)-benzamide
4-Cyano-N-[4-(11β-fluoro-3,17β-dihydroxy-estra-1,3,5(10)-trien-7α-yl)-butyl]-N-(3-phenyl-propyl)-benzamide
4-Cyano-N-[4-(11β-fluoro-3,17β-dihydroxy-estra-1,3,5(10)-trien-7α-yl)-butyl]-N-hexyl-benzamide
4-Cyano-N-[4-(11β-fluoro-3,17β-dihydroxy-estra-1,3,5(10)-trien-7α-yl)-butyl]-N-octyl-benzamide
3-Cyclohexyl-N-cyclohexylmethyl-N-[4-(11β-fluoro-3,17β-dihydroxy-estra-1,3,5(10)-trien-7α-yl)-butyl]-propionamide
N-Benzyl-3-cyclohexyl-N-[4-(11β-fluoro-3,17β-dihydroxy-estra-1,3,5(10)-trien-7α-yl)-butyl]-propionamide
3-Cyclohexyl-N- [4- (11β-fluoro-3,17β-dihydroxyestra-1,3,5(10)-trien-7α-yl)-butyl]-N-(2-methoxyethyl)-propionamide
3-Cyclohexyl-N-[4-(11β-fluoro-3,17β-dihydroxyestra-1,3,5(10)-trien-7α-yl)-butyl]-N-(3-phenylpropyl)-propionamide
3-Cyclohexyl-N-[4-(11β-fluoro-3,17β-dihydroxyestra-1,3,5(10)-trien-7α-yl)-butyl]-N-hexylpropionamide
3-Cyclohexyl-N-[4-(11β-fluoro-3,17β-dihydroxyestra-1,3,5(10)-trien-7α-yl)-butyl]-N-octylpropionamide
N-Cyclohexylmethyl-N-[4-(11β-fluoro-3,17β-dihydroxy-estra-1,3,5(10)-trien-7α-yl)-butyl]-(3,4-dimethoxyphenyl) -acetamide
N-Benzyl-N-[4-(11β-fluoro-3,17β-dihydroxy-estra-1,3,5(10)-trien-7α-yl)-butyl]-(3,4-dimethoxyphenyl)-acetamide
N-[4-(11β-Fluoro-3,17β-dihydroxy-estra-1,3,5(10)-trien-7α-yl)-butyl]-N-(2-methoxy-ethyl)-(3,4-dimethoxyphenyl)-acetamide
N-[4-(11β-Fluoro-3,17β-dihydroxy-estra-1,3,5(10)-trien-7α-yl)-butyl]-(3,4-dimethoxyphenyl)-N-(3-phenylpropyl)-acetamide
N-[4-(11β-Fluoro-3,17β-dihydroxy-estra-1,3,5(10)-trien-7α-yl)-butyl]-N-hexyl-(3,4-dimethoxyphenyl)-acetamide
N-[4-(11β-Fluoro-3,17β-dihydroxy-estra-1,3,5(10)-trien-7α-yl)-butyl]-(3,4-dimethoxyphenyl)-N-octylacetamide
N-Cyclohexylmethyl-4-ethoxy-N-[4-(11β-fluoro-3,17β-dihydroxy-estra-1,3,5(10)-trien-7α-yl)-butyl]-benzamide
N-Benzyl-4-ethoxy-N-[4-(11β-fluoro-3,17β-dihydroxy-estra-1,3,5(10)-trien-7α-yl)-butyl]-benzamide
4-Ethoxy-N-[4-(11β-fluoro-3,17β-dihydroxy-estra-1,3,5(10)-trien-7α-yl)-butyl]-N-(2-methoxy-ethyl)-benzamide
4-Ethoxy-N-[4-(11β-fluoro-3,17β-dihydroxy-estra-1,3,5(10)-trien-7α-yl)-butyl]-N-(3-phenyl-propyl)-benzamide
4-Ethoxy-N-[4-(11β-fluoro-3,17β-dihydroxy-estra-1,3,5(10)-trien-7α-yl)-butyl]-N-hexyl-benzamide
4-Ethoxy-N-[4-(11β-fluoro-3,17β-dihydroxy-estra-1,3,5(10)-trien-7α-yl)-butyl]-N-octyl-benzamide
N-Cyclohexylmethyl-N-[4-(11β-fluoro-3,17β-dihydroxy-estra-1,3,5(10)-trien-7α-yl)-butyl]-dodecanamide
N-Benzyl-N-[4-(11β-fluoro-3,17β-dihydroxy-estra-1,3,5(10)-trien-7α-yl)-butyl]-dodecanamide
N-[4-(11β-Fluoro-3,17β-dihydroxy-estra-1,3,5(10)-trien-7α-yl)-butyl]-N-(2-methoxy-ethyl)-dodecanamide
N-[4-(11β-fluoro-3,17β-dihydroxy-estra-1,3,5(10)-trien-7α-yl)-butyl]-N-(3-phenyl-propyl)-dodecanamide
N- [4- (11β-Fluoro-3,17β-dihydroxy-estra-1,3,5(10)-trien-7α-yl)-butyl]-N-hexyl-dodecanamide
N-[4-(11β-Fluoro-3,17β-dihydroxy-estra-1,3,5(10)-trien-7α-yl)-butyl]-N-octyl-dodecanamide
N-Cyclohexylmethyl-N-[4-(11β-fluoro-3,17β-dihydroxy-estra-1,3,5(10)-trien-7α-yl)-butyl]-naphth-2-yl-acetamide
N-Benzyl-N-[4-(11β-fluoro-3,17β-dihydroxy-estra-1,3,5(10)-trien-7α-yl)-butyl]-naphth-2-yl-acetamide
N-[4-(11β-Fluoro-3,17β-dihydroxy-estra-1,3,5(10)-trien-7α-yl)-butyl]-N-(2-methoxy-ethyl)-naphth-2-ylacetamide
N-[4-(11β-Fluoro-3,17β-dihydroxy-estra-1,3,5(10)-trien-7α-yl)-butyl]-naphth-2-yl-N-(3-phenyl-propyl)-acetamide
N-[4-(11β-Fluoro-3,17β-dihydroxy-estra-1,3,5(10)-trien-7α-yl)-butyl]-N-hexyl-naphth-2-yl-acetamide
N-[4-(11β-Fluoro-3,17β-dihydroxy-estra-1,3,5(10)-trien-7α-yl)-butyl]-naphth-2-yl-N-octyl-acetamide
N-Cyclohexylmethyl-N-[4-(11β-fluoro-3,17β-dihydroxy-estra-1,3,5(10)-trien-7α-yl)-butyl]-4-phenylbutyramide
N-Benzyl-N-[4-(11β-fluoro-3,17β-dihydroxy-estra-1,3,5(10)-trien-7α-yl)-butyl]-4-phenyl-butyramide
N-[4-(11β-Fluoro-3,17β-dihydroxy-estra-1,3,5(10)-trien-7α-yl)-butyl]-N-(2-methoxy-ethyl)-4-phenylbutyramide
N-[4-(11β-Fluoro-3,17β-dihydroxy-estra-1,3,5(10)-trien-7α-yl)-butyl]-4-phenyl-N-(3-phenyl-propyl)-butyramide
N-[4-(11β-Fluoro-3,17β-dihydroxy-estra-1,3,5(10)-trien-7α-yl)-butyl]-N-hexyl-4-phenyl-butyramide
N-[4-(11β-Fluoro-3,17β-dihydroxy-estra-1,3,5(10)-trien-7α-yl)-butyl]-N-octyl-4-phenyl-butyramide
N-Cyclohexylmethyl-N-[4-(11β-fluoro-3,17β-dihydroxy-estra-1,3,5(10)-trien-7α-yl)-butyl]-propionamide
N-Benzyl-N-[4-(11β-fluoro-3,17β-dihydroxy-estra-1,3,5(10)-trien-7α-yl)-butyl]-propionamide
N-[4-(11β-Fluoro-3,17β-dihydroxy-estra-1,3,5(10)-trien-7α-yl)-butyl]-N-(2-methoxy-ethyl)-propionamide
N-[4-(11β-Fluoro-3,17β-dihydroxy-estra-1,3,5(10)-trien-7α-yl)-butyl]-N-(3-phenyl-propyl)-propionamide
N-[4-(11β-Fluoro-3,17β-dihydroxy-estra-1,3,5(10)-trien-7α-yl)-butyl]-N-hexyl-propionamide
N-[4-(11β-Fluoro-3,17β-dihydroxy-estra-1,3,5(10)-trien-7α-yl)-butyl]-N-octyl-propionamide
4-Biphenyl-N-cyclohexylmethyl-N-[4-(11β-fluoro-3,17β-dihydroxy-estra-1,3,5(10)-trien-7α-yl)-butyl]-acetamide
4-Biphenyl-N-benzyl-N-[4-(11β-fluoro-3,17β-dihydroxy-estra-1,3,5(10)-trien-7α-yl)-butyl]-acetamide

4-Biphenyl-N-[4-(11β-fluoro-3,17β-dihydroxy-estra-1,3,5(10)-trien-7α-yl)-butyl]-N-(2-methoxy-ethyl)-acetamide
4-Biphenyl-N-[4-(11β-fluoro-3,17β-dihydroxy-estra-1,3,5(10)-trien-7α-yl)-butyl]-N-(3-phenyl-propyl)-acetamide
4-Biphenyl-N-[4-(11β-fluoro-3,17β-dihydroxy-estra-1,3,5(10)-trien-7α-yl)-butyl]-N-hexyl-acetamide
4-Biphenyl-N-[4-(11β-fluoro-3,17β-dihydroxy-estra-1,3,5(10)-trien-7α-yl)-butyl]-N-octyl-acetamide
4-Acetylamino-N-cyclohexylmethyl-N-[5-(11β-fluoro-3,17β-dihydroxy-estra-1,3,5(10)-trien-7α-yl)-pentyl]-benzamide
4-Acetylamino-N-benzyl-N-[5-(11β-fluoro-3,17β-dihydroxy-estra-1,3,5(10)-trien-7α-yl)-pentyl]-benzamide
4-Acetylamino-N-[5-(11β-fluoro-3,17β-dihydroxyestra-1,3,5(10)-trien-7α-yl)-pentyl]-N-(2-methoxyethyl)-benzamide
4-Acetylamino-N-[5-(11β-fluoro-3,17β-dihydroxyestra-1,3,5(10)-trien-7α-yl)-pentyl]-N-(3-phenylpropyl)-benzamide
4-Acetylamino-N-[5-(11β-fluoro-3,17β-dihydroxyestra-1,3,5(10)-trien-7α-yl)-pentyl]-N-hexyl-benzamide
4-Acetylamino-N-[5-(11β-fluoro-3,17β-dihydroxyestra-1,3,5(10)-trien-7α-yl)-pentyl]-N-octyl-benzamide
4-Cyano-N-cyclohexylmethyl-N-[5-(11β-fluoro-3,17β-dihydroxy-estra-1,3,5(10)-trien-7α-yl)-pentyl]-benzamide
N-Benzyl-4-cyano-N-[5-(11β-fluoro-3,17β-dihydroxyestra-1,3,5(10)-trien-7α-yl)-pentyl]-benzamide
4-Cyano-N-[5-(11β-fluoro-3,17β-dihydroxy-estra-1,3,5(10)-trien-7α-yl)-pentyl]-N-(2-methoxy-ethyl)-benzamide
4-Cyano-N-[5-(11β-fluoro-3,17β-dihydroxy-estra-1,3,5(10)-trien-7α-yl)-pentyl]-N-(3-phenyl-propyl)-benzamide
4-Cyano-N-[5-(11β-fluoro-3,17β-dihydroxy-estra-1,3,5(10)-trien-7α-yl)-pentyl]-N-hexyl-benzamide
4-Cyano-N-[5-(11β-fluoro-3,17β-dihydroxy-estra-1,3,5(10)-trien-7α-yl)-pentyl]-N-octyl-benzamide
3-Cyclohexyl-N-cyclohexylmethyl-N-[5-(11β-fluoro-3,17β-dihydroxy-estra-1,3,5(10)-trien-7α-yl)-pentyl]-propionamide
N-Benzyl-3-cyclohexyl-N-[5-(11β-fluoro-3,17β-dihydroxy-estra-1,3,5(10)-trien-7α-yl)-pentyl]-propionamide
3-Cyclohexyl-N-[5-(11β-fluoro-3,17β-dihydroxyestra-1,3,5(10)-trien-7α-yl)-pentyl]-N-(2-methoxyethyl)-propionamide
3-Cyclohexyl-N-[5-(11β-fluoro-3,17β-dihydroxyestra-1,3,5(10)-trien-7α-yl)-pentyl]-N-(3-phenylpropyl)-propionamide
3-Cyclohexyl-N-[5-(11β-fluoro-3,17β-dihydroxyestra-1,3,5(10)-trien-7α-yl)-pentyl]-N-hexylpropionamide
3-Cyclohexyl-N-[5-(11β-fluoro-3,17β-dihydroxyestra-1,3,5(10)-trien-7α-yl)-pentyl]-N-octylpropionamide
N-Cyclohexylmethyl-N-[5-(11β-fluoro-3,17β-dihydroxy-estra-1,3,5(10)-trien-7α-yl)-pentyl]-(3,4-dimethoxyphenyl)-acetamide
N-Benzyl-N-[5-(11β-fluoro-3,17β-dihydroxy-estra-1,3,5(10)-trien-7α-yl)-pentyl]-(3,4-dimethoxyphenyl)-acetamide
N-[5-(11β-Fluoro-3,17β-dihydroxy-estra-1,3,5(10)-trien-7α-yl)-pentyl]-N-(2-methoxy-ethyl)-(3,4-dimethoxyphenyl)-acetamide
N-[5-(11β-Fluoro-3,17β-dihydroxy-estra-1,3,5(10)-trien-7α-yl)-pentyl]-(3,4-dimethoxyphenyl)-N-(3-phenylpropyl)-acetamide
N-[5-(11β-Fluoro-3,17β-dihydroxy-estra-1,3,5(10)-trien-7α-yl)-pentyl]-N-hexyl-(3,4-dimethoxyphenyl)-acetamide
N-[5-(11β-Fluoro-3,17β-dihydroxy-estra-1,3,5(10)-trien-7α-yl)-pentyl]-(3,4-dimethoxyphenyl)-N-octylacetamide
N-Cyclohexylmethyl-4-ethoxy-N-[5-(11β-fluoro-3,17β-dihydroxy-estra-1,3,5(10)-trien-7α-yl)-pentyl]-benzamide
N-Benzyl-4-ethoxy-N-[5-(11β-fluoro-3,17β-dihydroxy-extra-1,3,5(10)-trien-7α-yl)-pentyl]-benzamide
4-Ethoxy-N- [5-(11β-fluoro-3,17β-dihydroxy-estra-1,3,5(10)-trien-7α-yl)-pentyl)-N-(2-methoxy-ethyl)-benzamide
4-Ethoxy-N-[5-(11β-fluoro-3,17β-dihydroxy-estra-1,3,5(10)-trien-7α-yl)-pentyl]-N-(3-phenyl-propyl)-benzamide
4-Ethoxy-N-[5-(11β-fluoro-3,17β-dihydroxy-estra-1,3,5(10)-trien-7α-yl)-pentyl]-N-hexyl-benzamide
4-Ethoxy-N-[5-(11β-fluoro-3,17β-dihydroxy-estra-1,3,5(10)-trien-7α-yl)-pentyl]-N-octyl-benzamide
N-Cyclohexylmethyl-N-[5-(11β-fluoro-3,17β-dihydroxy-estra-1,3,5(10)-trien-7α-yl)-pentyl]-dodecanamide
N-Benzyl-N-[5-(11β-fluoro-3,17β-dihydroxy-estra-1,3,5(10)-trien-7α-yl)-pentyl]-dodecanamide
N-[5-(11β-Fluoro-3,17β-dihydroxy-estra-1,3,5(10)-trien-7α-yl)-pentyl]-N-(2-methoxy-ethyl)-dodecanamide
N-[5-(11β-Fluoro-3,17β-dihydroxy-estra-1,3,5(10)-trien-7α-yl)-pentyl]-N-(3-phenyl-propyl)-dodecanamide

N-[5-(11β-Fluoro-3,17β-dihydroxy-estra-1,3,5(10)-trien-7α-yl)-pentyl]-N-hexyl-dodecanamide
N-[5-(11β-Fluoro-3,17β-dihydroxy-estra-1,3,5(10)-trien-7α-yl)-pentyl]-N-octyl-dodecanamide
N-Cyclohexylmethyl-N-[5-(11β-fluoro-3,17β-dihydroxy-extra-1,3,5(10)-trien-7α-yl)-pentyl]-naphth-2-yl-acetamide
N-Benzyl-N-[5-(11β-fluoro-3,17β-dihydroxy-estra-1,3,5(10)-trien-7α-yl)-pentyl]-naphth-2-yl-acetamide
N-[5-(11β-Fluoro-3,17β-dihydroxy-estra-1,3,5(10)-trien-7α-yl)-pentyl]-N-(2-methoxy-ethyl)-naphth-2-yl-acetamide
N-[5-(11β-Fluoro-3,17β-dihydroxy-estra-1,3,5(10)-trien-7α-yl)-pentyl]-naphth-2-yl-N-(3-phenyl-propyl)-acetamide
N-[5-(11β-Fluoro-3,17β-dihydroxy-estra-1,3,5(10)-trien-7α-yl)-pentyl]-N-hexyl-naphth-2-yl-acetamide
N-[5-(11β-Fluoro-3,17β-dihydroxy-estra-1,3,5(10)-trien-7α-yl)-pentyl]-naphth-2-yl-N-octyl-acetamide
N-Cyclohexylmethyl-N- [5-(11β-fluoro-3,17β-dihydroxy-estra-1,3,5(10)-trien-7α-yl)-pentyl]-4-phenyl-butyramide
N-Benzyl-N-[5-(11β-fluoro-3,17β-dihydroxy-estra-1,3,5(10)-trien-7α-yl)-pentyl]-4-phenyl-butyramide
N-[5-(11β-Fluoro-3,17β-dihydroxy-estra-1,3,5(10)-trien-7α-yl)-pentyl]-N-(2-methoxy-ethyl)-4-phenylbutyramide
N-[5-(11β-Fluoro-3,17β-dihydroxy-estra-1,3,5(10)-trien-7α-yl)-pentyl]-4-phenyl-N-(3-phenyl-propyl)-butyramide
N-[5-(11β-Fluoro-3,17β-dihydroxy-estra-1,3,5(10)-trien-7α-yl)-pentyl]-N-hexyl-4-phenyl-butyramide
N-[5-(11β-Fluoro-3,17β-dihydroxy-estra-1,3,5(10)-trien-7α-yl)-pentyl]-N-octyl-4-phenyl-butyramide
N-Cyclohexylmethyl-N-[5-(11β-fluoro-3,17β-dihydroxy-estra-1,3,5(10)-trien-7α-yl)-pentyl]-propionamide
N-Benzyl-N-[5-(11β-fluoro-3,17β-dihydroxy-estra-1,3,5(10)-trien-7α-yl)-pentyl]-propionamide
N-[5-(11β-Fluoro-3,17β-dihydroxy-estra-1,3,5(10)-trien-7α-yl)-pentyl]-N-(2-methoxy-ethyl)-propionamide
N-[5-(11β-Fluoro-3,17β-dihydroxy-estra-1,3,5(10)-trien-7α-yl)-pentyl]-N-(3-phenyl-propyl)-propionamide
N-[5-(11β-Fluoro-3,17β-dihydroxy-estra-1,3,5(10)-trien-7α-yl)-pentyl]-N-hexyl-propionamide
N-[5-(11β-Fluoro-3,17β-dihydroxy-estra-1,3,5(10)-trien-7α-yl)-pentyl]-N-octyl-propionamide
4-Biphenyl-N-cyclohexylmethyl-N-[5-(11β-fluoro-3,17β-dihydroxy-estra-1,3,5(10)-trien-7α-yl)-pentyl]-acetamide
4-Biphenyl-N-benzyl-N-[5-(11β-fluoro-3,17β-dihydroxy-estra-1,3,5(10)-trien-7α-yl)-pentyl]-acetamide
4-Biphenyl-N-[5-(11β-fluoro-3,17β-dihydroxy-estra-1,3,5(10)-trien-7α-yl)-pentyl]-N-(2-methoxy-ethyl)-acetamide
4-Biphenyl-N-[5-(11β-fluoro-3,17β-dihydroxy-estra-1,3,5(10)-trien-7α-yl)-pentyl]-N-(3-phenyl-propyl)-acetamide
4-Biphenyl-N-[5-(11β-fluoro-3,17β-dihydroxy-estra-1,3,5(10)-trien-7α-yl)-pentyl]-N-hexyl-acetamide
4-Biphenyl-N-[5-(11β-fluoro-3,17β-dihydroxy-estra-1,3,5(10)-trien-7α-yl)-pentyl]-N-octyl-acetamide
4-Acetylamino-N-cyclohexylmethyl-N-[6-(11β-fluoro-3,17β-dihydroxy-estra-1,3,5(10)-trien-7α-yl)-hexyl]-benzamide
4-Acetylamino-N-benzyl-N-[6-(11β-fluoro-3,17β-dihydroxy-estra-1,3,5(10)-trien-7α-yl)-hexyl]-benzamide
4-Acetylamino-N-[6-(11β-fluoro-3,17β-dihydroxyestra-1,3,5(10)-trien-7α-yl)-hexyl]-N-(2-methoxyethyl)-benzamide
4-Acetylamino-N-[6-(11β-fluoro-3,17β-dihydroxyestra-1,3,5(10)-trien-7α-yl)-hexyl]-N-(3-phenylpropyl)-benzamide
4-Acetylamino-N-[6-(11β-fluoro-3,17β-dihydroxyestra-1,3,5(10)-trien-7α-yl)-hexyl]-N-hexyl-benzamide
4-Acetylamino-N-[6-(11β-fluoro-3,17β-dihydroxyestra-1,3,5(10)-trien-7α-yl)-hexyl]-N-octyl-benzamide
4-Cyano-N-cyclohexylmethyl-N-[6-(11β-fluoro-3,17β-dihydroxy-estra-1,3,5(10)-trien-7α-yl)-hexyl]-benzamide
N-Benzyl-4-cyano-N-[6-(11β-fluoro-3,17β-dihydroxyestra-1,3,5(10)-trien-7α-yl)-hexyl]-benzamide
4-Cyano-N-[6-(11β-fluoro-3,17β-dihydroxy-estra-1,3,5(10)-trien-7α-yl)-hexyl]-N-(2-methoxy-ethyl)-benzamide
4-Cyano-N-[6-(11β-fluoro-3,17β-dihydroxy-estra-1,3,5(10)-trien-7α-yl)-hexyl]-N-(3-phenyl-propyl]-benzamide
4-Cyano-N-[6-(11β-fluoro-3,17β-dihydroxy-estra-1,3,5(10)-trien-7α-yl)-hexyl]-N-hexyl-benzamide
4-Cyano-N-[6-(11β-fluoro-3,17β-dihydroxy-estra-1,3,5(10)-trien-7α-yl)-hexyl]-N-octyl-benzamide
3-Cyclohexyl-N-cyclohexylmethyl-N-[6-(11β-fluoro-3,17β-dihydroxy-estra-1,3,5(10)-trien-7α-yl)-hexyl]-propionamide
N-Benzyl-3-cyclohexyl-N-[6-(11β-fluoro-3,17β-dihydroxy-estra-1,3,5(10)-trien-7α-yl)-hexyl]-propionamide
3-Cyclohexyl-N-[6-(11β-fluoro-3,17β-dihydroxyestra-1,3,5(10)-trien-7α-yl)-hexyl]-N-(2-methoxyethyl)-propionamide

3-Cyclohexyl-N-[6-(11β-fluoro-3,17β-dihydroxyestra-1,3,5(10)-trien-7α-yl)-hexyl]-N-(3-phenylpropyl)-propionamide
3-Cyclohexyl-N-[6-(11β-fluoro-3,17β-dihydroxyestra-1,3,5(10)-trien-7α-yl)-hexyl]-N-hexylpropionamide
3-Cyclohexyl-N-[6-(11β-fluoro-3,17β-dihydroxyestra-1,3,5(10)-trien-7α-yl)-hexyl]-N-octylpropionamide
N-Cyclohexylmethyl-N-[6-(11β-fluoro-3,17β-dihydroxy-estra-1,3,5(10)-trien-7α-yl)-hexyl]-(3,4-dimethoxyphenyl)-acetamide
N-Benzyl-N-[6-(11β-fluoro-3,17β-dihydroxy-estra-1,3,5(10)-trien-7α-yl)-hexyl]-(3,4-dimethoxyphenyl)-acetamide
N-[6-(11β-Fluoro-3,17β-dihydroxy-estra-1,3,5(10)-trien-7α-yl)-hexyl]-N-(2-methoxy-ethyl)-(3,4-dimethoxyphenyl)-acetamide
N-[6-(11β-Fluoro-3,17β-dihydroxy-estra-1,3,5(10)-trien-7α-yl)-hexyl]-(3,4-dimethoxyphenyl)-N-(3-phenylpropyl)-acetamide
N-[6-(11β-Fluoro-3,17β-dihydroxy-estra-1,3,5(10)-trien-7α-yl)-hexyl]-N-hexyl-(3,4-dimethoxyphenyl)-acetamide
N-[6-(11β-Fluoro-3,17β-dihydroxy-estra-1,3,5(10)-trien-7α-yl)-hexyl]-(3,4-dimethoxyphenyl)-N-octylacetamide
N-Cyclohexylmethyl-4-ethoxy-N-[6-(11β-fluoro-3,17β-dihydroxy-estra-1,3,5(10)-trien-7α-yl)-hexyl]-benzamide
N-Benzyl-4-ethoxy-N-[6-(11β-fluoro-3,17β-dihydroxy-estra-1,3,5(10)-trien-7α-yl)-hexyl]-benzamide
4-Ethoxy-N-[6-(11β-fluoro-3,17β-dihydroxy-estra-1,3,5(10)-trien-7α-yl)-hexyl]-N-(2-methoxy-ethyl)-benzamide
4-Ethoxy-N-[6-(11β-fluoro-3,17β-dihydroxy-estra-1,3,5(10)-trien-7α-yl)-hexyl]-N-(3-phenyl-propyl)-benzamide
4-Ethoxy-N-[6-(11β-fluoro-3,17β-dihydroxy-estra-1,3,5(10)-trien-7α-yl)-hexyl]-N-hexyl-benzamide
4-Ethoxy-N-[6-(11β-fluoro-3,17β-dihydroxy-estra-1,3,5(10)-trien-7α-yl)-hexyl]-N-octyl-benzamide
N-Cyclohexylmethyl-N-[6-(11β-fluoro-3,17β-dihydroxy-estra-1,3,5(10)-trien-7α-yl)-hexyl]-dodecanamide
N-Benzyl-N-[6-(11β-fluoro-3,17β-dihydroxy-estra-1,3,5(10)-trien-7α-yl)-hexyl]-dodecanamide
N-[6-(11β-Fluoro-3,17β-dihydroxy-estra-1,3,5(10)-trien-7α-yl)-hexyl]-N-(2-methoxy-ethyl)-dodecanamide
N-[6-(11β-Fluoro-3,17β-dihydroxy-estra-1,3,5(10)-trien-7α-yl)-hexyl]-N-(3-phenyl-propyl)-dodecanamide
N-[6-(11β-Fluoro-3,17β-dihydroxy-estra-1,3,5(10)-trien-7α-yl)-hexyl]-N-hexyl-dodecanamide
N-[6-(11β-Fluoro-3,17β-dihydroxy-estra-1,3,5(10)-trien-7α-yl)-hexyl]-N-octyl-dodecanamide
N-Cyclohexylmethyl-N-[6-(11β-fluoro-3,17β-dihydroxy-estra-1,3,5(10)-trien-7α-yl)-hexyl]-naphth-2-yl-acetamide
N-Benzyl-N-[6-(11β-fluoro-3,17β-dihydroxy-estra-1,3,5(10)-trien-7α-yl)-hexyl]-naphth-2-yl-acetamide
N-[6-(11β-Fluoro-3,17β-dihydroxy-estra-1,3,5(10)-trien-7α-yl)-hexyl]-N-(2-methoxy-ethyl)-naphth-2-yl-acetamide
N-[6-(11β-Fluoro-3,17β-dihydroxy-estra-1,3,5(10)-trien-7α-yl)-hexyl]-naphth-2-yl-N-(3-phenyl-propyl)-acetamide
N-[6-(11β-Fluoro-3,17β-dihydroxy-estra-1,3,5(10)-trien-7α-yl)-hexyl]-N-hexyl-naphth-2-yl-acetamide
N-[6-(11β-Fluoro-3,17β-dihydroxy-estra-1,3,5(10)-trien-7α-yl)-hexyl]-naphth-2-yl-N-octyl-acetamide
N-Cyclohexylmethyl-N-[6-(11β-fluoro-3,17β-dihydroxy-estra-1,3,5(10)-trien-7α-yl)-hexyl]-4-phenylbutyramide
N-Benzyl-N-[6-(11β-fluoro-3,17β-dihydroxy-estra-1,3,5(10)-trien-7α-yl)-hexyl]-4-phenyl-butyramide
N-[6-(11β-Fluoro-3,17β-dihydroxy-estra-1,3,5(10)-trien-7α-yl)-hexyl]-N-(2-methoxy-ethyl)-4-phenylbutyramide
N-[6-(11β-Fluoro-3,17β-dihydroxy-estra-1,3,5(10)-trien-7α-yl)-hexyl]-4-phenyl-N-(3-phenyl-propyl)-butyramide
N-[6-(11β-Fluoro-3,17β-dihydroxy-estra-1,3,5(10)-trien-7α-yl)-hexyl]-N-hexyl-4-phenyl-butyramide
N-[6-(11β-Fluoro-3,17β-dihydroxy-estra-1,3,5(10)-trien-7α-yl)-hexyl]-N-octyl-4-phenyl-butyramide
9-N-Cyclohexylmethyl-N-[6-(11β-fluoro-3,17β-dihydroxy-estra-1,3,5(10)-trien-7α-yl)-hexyl]-propionamide
N-Benzyl-N-[6-(11β-fluoro-3,17β-dihydroxy-estra-1,3,5(10)-trien-7α-yl)-hexyl]-propionamide
N-[6-(11β-Fluoro-3,17β-dihydroxy-estra-1,3,5(10)-trien-7α-yl)-hexyl]-N-(2-methoxy-ethyl)-propionamide
N-[6-(11β-Fluoro-3,17β-dihydroxy-estra-1,3,5(10)-trien-7α-yl)-hexyl]-N-(3-phenyl-propyl)-propionamide
N-[6-(11β-Fluoro-3,17β-dihydroxy-estra-1,3,5(10)-trien-7α-yl)-hexyl] -N-hexyl-propionamide
N-[6-(11β-Fluoro-3,17β-dihydroxy-estra-1,3,5(10)-trien-7α-yl)-hexyl]-N-octyl-propionamide
4-Biphenyl-N-cyclohexylmethyl-N-[6-(11β-fluoro-3,17β-dihydroxy-estra-1,3,5(10)-trien-7α-yl)-hexyl]-acetamide
4-Biphenyl-N-benzyl-N-[6-(11β-fluoro-3,17β-dihydroxy-estra-1,3,5(10)-trien-7α-yl)-hexyl]-acetamide

4-Biphenyl-N-[6-(11β-fluoro-3,17β-dihydroxy-estra-1,3,5(10)-trien-7α-yl)-hexyl]-N-(2-methoxy-ethyl)-acetamide
4-Biphenyl-N-[6-(11β-fluoro-3,17β-dihydroxy-estra-1,3,5(10)-trien-7α-yl)-hexyl]-N-(3-phenyl-propyl)-acetamide
4-Biphenyl-N-[6-(11β-fluoro-3,17β-dihydroxy-estra-1,3,5(10)-trien-7α-yl)-hexyl]-N-hexyl-acetamide
4-Biphenyl-N-[6-(11β-fluoro-3,17β-dihydroxy-estra-1,3,5(10)-trien-7α-yl)-hexyl]-N-octyl-acetamide
11β-Fluoro-7α-{5-[methyl-(2-p-tolyl-ethyl}-amino]-pentyl}-estra-1,3,5(10)-triene-3,17β-diol
7α-(5-{[2-(4-Ethoxy-phenyl)-ethyl]-methyl-amino}-pentyl)-11β-fluoro-estra-1,3,5(10)-triene-3,17β-diol
11β-Fluoro-7α-{5-[methyl-(3-phenyl-propyl)-amino]-pentyl}-estra-1,3,5(10)-triene-3,17β-diol
11β-Fluoro-7α-{5-[methyl-(3-pyridin-3-yl-propyl)-amino]-pentyl}-estra-1,3,5(10)-triene-3,17β-diol
11β-Fluoro-7α-{5-[methyl-(3-p-tolyl-propyl)-amino]-pentyl}-estra-1,3,5(10)-triene-3,17β-diol
7α-(5-{[3-(4-Chloro-phenyl)-propyl]-methyl-amino}-pentyl)-11β-fluoro-estra-1,3,5(10)-triene-3,17β-diol
7α-(5-{[3-(4-Ethoxy-phenyl)-propyl]-methyl-amino}-pentyl)-11β-fluoro-estra-1,3,5(10)-triene-3,17β-diol
11β-Fluoro-7α-{5-[methyl-(4-methyl-pentyl)-amino]-pentyl}-estra-1,3,5(10)-triene-3,17β-diol.

**12.** Use of the compounds of general formula I according to Claim 1 for the production of pharmaceuticals.

**13.** Pharmaceutical preparations that contain at least one compound of general formula I according to Claim 1 and a pharmaceutically compatible vehicle.